# EUROPEAN PATENT APPLICATION

(11) **EP 4 643 878 A2**
(43) Date of publication of application: **05.11.2025**
(21) Application number: 23910855.8
(22) Date of filing: 28.12.2023
(51) Int. Cl.: A61K 39/395, A61K 9/08, C07K 16/18, A61P 37/00, A61P 9/00

(54) **PHARMACEUTICAL PREPARATION COMPRISING THERAPEUTIC ANTIBODY AND USE THEREOF**

(30) Priority: 29.12.2022 CN 202211710550
(71) Applicant: Suzhou Transcenta Therapeutics Co., Ltd., Suzhou, Jiangsu 215123 (CN)
(72) Inventor: ZHANG, Fan, Suzhou, Jiangsu 215123 (CN); HU, Yudi, Suzhou, Jiangsu 215123 (CN); SHAO, Qi, Suzhou, Jiangsu 215123 (CN); QIAN, Xueming, Suzhou, Jiangsu 215123 (CN); LI, Hongjun, Suzhou, Jiangsu 215123 (CN); GU, Yi, Suzhou, Jiangsu 215123 (CN); TENG, Fei, Suzhou, Jiangsu 215123 (CN); SUN, Di, Suzhou, Jiangsu 215123 (CN)
(74) Representative: Bianchetti & Minoja with Trevisan & Cuonzo IPS SRL
(86) International application number: PCT/CN2023/142858
(87) International publication number: WO 2024/140939

(57) **Abstract**

The present invention provides stable pharmaceutical formualtions comprising a monoclonal antibody that specifically binds to human MASP-2, which is present in high and low concentrations, a kit comprising the formulation, a pharmaceutical composition comprising the formulation and a unit dosage form comprising the formulation, a treatment method for inhibiting MASP-2 dependent complement activation, a treatment method for treating related diseases with the formulation, the kit, the pharmaceutical composition and the unit dosage form, and as well as a use in manufacturing a corresponding medicaent. The present invention also provides a novel monoclonal antibody that specifically binds to human MASP-2.

## Description

### TECHNICAL FIELD

The present invention relates to the field of pharmaceutical formulations, and particularly to the field of therapeutic antibody formulations. More specifically, the present invention relates to a pharmaceutical formulation comprising a monoclonal antibody (mAb) that specifically binds to human MASP-2, and the use thereof.

### BACKGROUND

MASP-2 (MBL-associated serine protease 2) is involved in the complement system, which shows a striking homology with the MASP-1 and the two C1q-associated serine proteases C1r and C1s. Once the lectin recognizes and binds to the pathogen, the protozyme form of MASP-2 cleaves between CCP2 and SP domains (cleaved between the conserved R444 and I445), and turns into the active form consisting of two polypeptide chains (heavy chain /A chain and light chain /B chain) linked by disulfide bond (C434-C552) (A.B.W.Boldt et al., Human Immunology, 72(9): 753-760 (2011)). When MBL binds to a pathogen, MASP-2 is activated to cleave complement components C4 and C2 into C4a, C4b, C2a, and C2b, generating C3 convertase C4bC2b, subsequently C3 being converted to C3b by C4bC2b and finally form membrane attack complex (MAC) after C5 being converted to C5b by C3b. Activation of C3 finally leads to the formation of MAC, and then initiates a series of cascade activation processes of downstream complement system to stimulate innate immune response. Indeed C4b can activate the generation of C4d. C4d deposition could be a marker of complement activation. It has been shown that C4d-positive staining is an independent risk factor for the development of ESRD in IgAN (see, Clin J Am Soc Nephrol 9: 897-904, 2014) and kidney with C4d staining has significant short survival time than those without C4d staining in IgAN patients. C4D staining has also been detected in both kidneys of lupus nephritis and membrane glomerulonephritis patients. MASP-2 has been identified as a promising target for the treatment of autoimmune diseases.

In addition to its essential role in immune defense, the complement system contributes to tissue damage in many clinical conditions. Need remains for therapeutically effective complement inhibitors, such as novel MASP-2 antibodies, especially those with favorable high binding affinity and specificity, to prevent the adverse effects.

Therapeutic antibodies must be formulated in a manner that not only makes the formulations suitable for administration to patients, but also maintains their stability during storage and subsequent use. For example, if the formulation solution is improperly formulated, the degradation, aggregation and/or undesirable or even harmful chemical modifications may be more prone to occur to the therapeutic antibodies presented therein. The stability of an antibody in liquid formulation may vary depending on the kinds of excipients used in the formulation, but also on the amounts and proportions of the excipients relative to one another. Furthermore, other considerations aside from stability, such as the concentration of antibody that can be accommodated by a given formulation, and the visual quality or appearance of the formulation, must be taken into account when preparing a liquid formulation of antibody. Thus, when preparing a formulation of therapeutic antibody, great care must be taken to arrive at a formulation that remains stable, contains an adequate concentration of antibody, and possesses a suitable viscosity as well as other properties which enable the formulation to be conveniently administered to patients.

For an anti-MASP-2 antibody, for example, Narsoplimab (OMS721) developed by Omeros has entered Phase III clinical trials, and a formulation containing such an MASP-2 antibody which comprises 185 mg/mL protein, 20 mM L-histidine, 200 mM L-arginine HCl, 0.01% (w/v) of polysorbate 80, with a pH of 5.5-6.5 has been described in Chinese Patent Application No. CN201780051640.7. However, the above-noted formulation cannot provide the desired stability for the anti-MASP-2 monoclonal antibody of the present invention. Thus, for the novel anti-MASP-2 monoclonal antibody provided in the present invention, there is still a need to develop a stable liquid formulation.

### BRIEF SUMMARY OF THE INVENTION

MASP-2 is an important and advantageous therapeutic target. The present invention provides formulae of the formulations containing the anti-MASP-2 antibody of the present invention, which can maintain good stability of the anti-MASP-2 mAb of the present invention in a liquid state either at a higher concentration or at a lower concentration, satisfying the requirements of the production process and the clinical administration, and supporting the development of the anti-MASP-2 monoclonal antibody of the present invention into an intravenous injection form and a subcutaneous injection form. The present invention also provides an antibody against MASP-2.

### I. The antibody formulation of the present invention

In an aspect, the present invention provides a stable liquid pharmaceutical formulation, characterized in that, the drug substance (DS) is a monoclonal antibody or an antigen-binding fragment thereof that specifically binds to human MASP-2, and the formulation further comprises a buffer, a surfactant, and an auxiliary material.

In some embodiments of the present invention, the buffer in the stable liquid pharmaceutical formulation comprises a histidine and/or acetate buffer system; the surfactant comprises a polysorbate; the auxiliary material comprises sucrose, trehalose and/or proline; and the stable liquid pharmaceutical formulation has a pH of 4.7-6.1.

Thus, in some embodiments of the present invention, a stable liquid pharmaceutical formulation is provided, which comprises: (a) a monoclonal antibody that specifically binds to human MASP-2 or an antigen-binding fragment thereof; (b) a buffer comprising a histidine and/or acetate buffer system; (c) a surfactant comprising a polysorbate; and (d) an auxiliary material comprising sucrose, trehalose and/or proline; wherein the formulation has a pH of 4.7-6.1.

In some embodiments, the concentration of the antibody ranges from 10 mg/ml ± 1 mg/ml to 200 mg/ml ± 20 mg/ml. In some embodiments, the concentration of the antibody is 20 mg/ml ± 2 mg/ml. In some embodiments, the concentration of the antibody is 60 mg/ml ± 6 mg/ml. In some embodiments, the concentration of the antibody is 100 mg/ml ± 10 mg/ml. In some embodiments, the concentration of the antibody is 150 mg/ml ± 15 mg/ml. In some embodiments, the concentration of the antibody ranges from about 20 mg/ml to about 100 mg/ml.

In some embodiments, the buffer comprises histidine, and the concentration of histidine is 5 mM ± 1 mM to 20 mM ± 4 mM. In some instances, the concentration of the histidine buffer is 10 mM ± 2 mM. In some embodiments, the histidine buffer comprises L-histidine and L-histidine hydrochloride monohydrate. In some instances, the buffer comprises 0.175 mg/ml of L-histidine and 1.86 mg/ml of L-histidine hydrochloride monohydrate.

In some embodiments, the buffer comprises acetate, and the concentration of acetate is 5 mM ± 1 mM to 20 mM ± 4 mM. In some instances, the concentration of the acetate buffer is 10 mM ± 1 mM. In some embodiments, the acetate buffer comprises acetic acid and sodium acetate trihydrate.

In some embodiments, the concentration of the polysorbate is 0.025% ± 0.01% to 0.1% ± 0.01% (w/v). In some instances, the concentration of the polysorbate is 0.05% ± 0.01% (w/v). In some embodiments, the polysorbate is polysorbate 20. In some embodiments, the polysorbate is polysorbate 80.

In some embodiments, the auxiliary material is sucrose. In some embodiments, the concentration of sucrose is 5.5% ± 0.5% to 9% ± 0.5% (w/v). In certain embodiments, the concentration of sucrose is 5.8% ± 0.5% to 8.6% ± 0.5% (w/v). In some embodiments, the concentration of sucrose is 5.8% ± 0.5% (w/v). In some embodiments, the concentration of sucrose is 6.0% ± 0.5% (w/v). In some embodiments, the concentration of sucrose is 6.5% ± 0.5% (w/v). In some embodiments, the concentration of sucrose is 7.0% ± 0.5% (w/v). In some embodiments, the concentration of sucrose is 8.6% ± 0.5% (w/v).

In some embodiments, the auxiliary material is trehalose. In some embodiments, the concentration of trehalose ranges from 5.5% ± 0.5% to 9% ± 0.5% (w/v). In certain embodiments, the concentration of trehalose ranges from 7.0% ± 0.5% to 9.0% ± 0.5% (w/v). In some embodiments, the concentration of trehalose is 7.0% ± 0.5% (w/v). In some embodiments, the concentration of trehalose is 8.0% ± 0.5% (w/v). In some embodiments, the concentration of trehalose is 9.0% ± 0.5% (w/v).

In some embodiments, the auxiliary material is proline. In some embodiments, the concentration of proline is 200mM to 300mM. In certain embodiments, the concentration of proline is 200mM to 250mM. In some embodiments, the concentration of proline is 230mM.

In some embodiments, the buffer used in the formulation of the present invention can control the pH value of the formulation of the present invention in the range of about 4.7-6.1, preferably about 5.0-6.0, more preferably about 5.0-5.5, most preferably 5.3± 0.1. In some specific embodiments, the antibody formulation of the present invention has a pH of about 4.7, 5.0, 5.2, 5.3, 5.4, 5.5, 6.0, 6.1, 6.2, or 6.5.

In a preferred embodiment of the present invention, the surfactant in the liquid formulation of the present invention is a polysorbate, such as polysorbate-20, polysorbate-80, polysorbate-60, or polysorbate-40; Pluronic, etc. In a preferred embodiment, the liquid formulation of the present invention comprises polysorbate-80 as the surfactant. In another preferred embodiments, the liquid formulation of the present invention comprises polysorbate-20 as the surfactant.

In one embodiment, the liquid formulation is a formulation for parenteral administration, preferably an injection product, more preferably a subcutaneous injection product or an intravenous injection product. In one embodiment, the liquid formulation is an intravenous infusion product.

In one embodiment, the liquid antibody formulation of the present application comprises:
(a) 20 mg/ml ± 2 mg/ml to 200 mg/ml ± 20 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose or 200mM to 300mM of proline,
with a pH of 4.7 to 6.0.

In one embodiment, the liquid antibody formulation of the present application comprises:
(a) 20 mg/ml ± 2 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose,
with a pH of 5.3 ± 0.5.

In one embodiment, the liquid antibody formulation of the present application comprises:
(a) 60 mg/ml ± 6 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% + 0.5% to 8.6% ± 0.5% (w/v) of sucrose or trehalose,
with a pH of 5.3 ± 0.5.

In one embodiment, the liquid antibody formulation of the present application comprises:
(a) 100 mg/ml ± 10 mg/ml of an antibody,
(b) a buffer comprising 5 mM ± 1 mM to 20 mM ± 4 mM of histidine or acetate,
(c) 0.025% + 0.01% to 0.1% ± 0.01% (w/v) of a polysorbate, and
(d) 6.5% ± 0.5% to 8.6% ± 0.5% (w/v) of sucrose or trehalose,
with a pH of 5.3 ± 0.5.

In one embodiment, the liquid antibody formulation of the present application comprises:
(a) 150 mg/ml ± 15 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose,
with a pH of 5.3 ± 0.5.

The liquid formulation of the present invention is is stable after storage for a long time, for example at least 24 months or longer. In one embodiment, the liquid formulation of the present invention can be stored under a condition of about -80°C to about 45°C, for example -80°C, about -30°C, about -20°C, about 0°C, about 5°C, about 25°C, about 35°C, about 38°C, about 40°C, about 42°C or about 45°C, for at least 10 days, at least 20 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer, being stable.

**In** some embodiments, the liquid formulation of the present invention has a mean diameter of 2-10 µm, as measured by Microflow-imaging system (MFI).

In some embodiments, the liquid formulation of the present invention has an osmolatity of 250 to 350 mOsm/kg H₂O.

In some embodiments, the liquid formulation of the present invention has a viscosity of about 1.0 centipoise to 30 centipoise, for example a viscosity of about 1.0 centipoise to 10 centipoise. In one embodiment, the liquid formulation has a viscosity of about 1.0 centipoise to 20 centipoise at 25°C. In one embodiment, the liquid formulation has a viscosity of about 1.0 centipoise to 10 centipoise at 25°C. In one embodiment, the liquid formulation has a viscosity of about 1.0, 2.0, 3.0, 4.0, 5.0, 6.0, 7.0, 8.0, 9.0 or 10.0 centipoise at 25°C. In one embodiment, the liquid formulation has a viscosity of about 5.0 centipoise to 7.0 centipoise at 4-40°C. In one embodiment, the liquid formulation has a viscosity of about 6.8 centipoise at 4-40°C. In one embodiment, the liquid formulation has a viscosity of about 6.8 centipoise at 25°C.

In one embodiment, the liquid formulation of the present invention is stable after storage for at least 24 months. In yet another embodiment, the liquid formulation of the present invention is stable under any temperature between -80°C to 45°C. In yet another embodiment, the liquid formulation of the present invention is stable under about 2°C-8°C for at least 3 months, preferably at least 12 months, more preferably at least 24 months, most preferably at least 36 months. In one embodiment, the liquid formulation of the present invention is stable under room temperature or under, e.g., about 25°C for at least 2 months, preferably at least 3 months, more preferably at least 6 months, most preferably at least 12 months. In yet another embodiment, the liquid formulation of the present invention is stable under about 40°C for at least 2 weeks, preferably at least 1 months, more preferably at least 6 weeks, most preferably at least 8 weeks. The stability is manifested as, for example, a monomer purity of the antibody in a dissolved state of more than 95%, preferably more than 98%, and more preferably more than 99%.

In one embodiment, the stability of the formulation after storage can be indicated by detections of the changes in the appearance, visible foreign matter, protein content, monomer purity, and/or charge variants of the formulation. In one embodiment, the stability of the liquid formulaiton of the present invention is detected in an experiment under a forced high temperature, such as after storage at 40°C ± 2°C for at least 1 week, 2 weeks or preferably 1 month, or in an accelerated experiment, such as after storage at 25°C ± 2°C for at least 1 month or 2 months, or in a long-term experiment, such as after storage at 5°C ± 3°C for at least 6 months or 12 months. The stability is manifested as, for example, a monomer purity of the antibody in a dissolved state of more than 95%, preferably more than 98%, and more preferably more than 99%.

In one embodiment, the stability of the liquid formulation of the present invention after storage is detected by visual inspection, wherein the appearance of the liquid formulation of the present invention remains in clear to slightly opalescent, and colorless to pale yellow liquid, and free of foreign matter. In one embodiment, the visual inspection under clarity detector shows the absence of visible foreign matter in the formulation. In one embodiment, the solution turbidity is measured by detecting the absorbance at about 405 nm or about 350 nm of the formulation. In one embodiment, the stability of the liquid formulation of the present invention is detected by measuring the transition temperature Tₒₙₛₑₜ at which the diameter of the proteins changes due to the occurrence of configuration change or aggregation during heating process, wherein preferably said Tₒₙₛₑₜ is higher than 60°C, 63°C, 65°C, 68°C or 70°C. In one embodiment, the stability of the liquid formulation of the present invention is detected by measuring the change in protein content after storage, wherein the change in the protein content is no more than 10%, preferably no more than 5%, ralative to the initial value at Day 0 of storage. In one embodiment, the stability of the liquid formulation of the present invention is detected by measuring the change in the monomer purity of the antibody in the liquid formulation of the present invention after storage via size-exclusion high-performance liquid chromatography (SEC-HPLC), the change in the monomer purity no more than 10%, for example no more than 5%, 4%, 3%, for example the change is no more than 1-2%, preferably no more than 1%, ralative to the initial value at Day 0 of storage. In another embodiment, after storage, the change in the monomer purity detected by non-reduced and/or reduced sodium dodecyl sulfate capillary electrophoresis (CE-SDS) decreases no more than 10%, for example no more than 5%, 4%, 3%. In one embodiment, after storage, the stability of the liquid formulation of the present invention is detected by whole column imaged capillary isoelectric focusing electrophoresis (icIEF) or cation exchange high performance liquid chromatography (CEX-HPLC), wherein the sum of the change of the charge variants (main component, acidic component and basic component) of the antibody is no more than 50%, for example no more than 40%, 30%, 20%, 10%, 5%, ralative to the initial value at Day 0 of storage.

In some embodiments, the formulation is stable after storage, for example under 2-8°C for at least 24 months, or under room temperature for at least 3 months, or under 40°C+2°C for 1 months, preferably having one or more of the following characteristics:
(i) the monomer purity of the antobody in the formulation is greater than 90%, preferably greater than 95%, 96%, 97%, 98%, 99%, as measured by SEC-HPLC;
(ii) the monomer purity of the antobody in the formulation is greater than 90%, preferably greater than 92%, 94%, 96%, 98%, as measured by reduced or non-reduced CE-SDS (e.g., non-reduced CE-SDS);
(iii) the sum of the change of each components (main component, acidic component and basic component) of the antibody protein in the formulation is no more than 50%, for example no more than 40%, 30%, 20%, 10%, 5%, ralative to the initial value at Day 0 of storage, as measured by iCIEF or CEX.

In some embodiments, the formulation is stable after shaking (e.g., shaking for 14 days), stirring (e.g., stirring for 6 hours), exposuring to light (e.g., exposuring to light for 3days), and/or freezing and thawing (e.g., freezing and thawing for 5 cycles), preferably having one or more of the following characteristics:
(i) the monomer purity of the antobody in the formulation is greater than 90%, preferably greater than 95%, 96%, 97%, 98%, or 99%, as measured via SEC-HPLC;
(ii) the monomer purity of the antobody in the formulation is greater than 90%, preferably greater than 92%, 94%, 96%, or 98%, as measured by reduced or non-reduced CE-SDS (e.g., non-reduced CE-SDS);
(iii) total changes of each components (main component, acidic component and basic component) of the antibody protein in the formulation is no more than 50%, for example no more than 40%, 30%, 20%, 10%, 5 %, ralative to the initial value at Day 0 of storage, as measured by iCIEF or CEX.

In a preferred embodiment of the present invention, the formulation of the present invention has a high concentration of antibody with a low viscosity at the same time suitable for administration, especially subcutaneous administration, and has high physical and chemical stabilities, being not susceptible to the formation of aggregates and particles or changes in charge heterogeneity during storage. These advantages are very beneficial for the production of complete, effective, and highly consistent medicaments for clinical use.

In an aspect, the present invention provides a delivery device comprising the liquid antibody formulation of the present application. In one embodiment, the delivery device of the present invention is provided in a pre-filled syringe comprising the liquid antibody formulation or solid antibody formulation of the present application, for example, for intravenous, subcutaneous, intradermal or intramuscular injection, intravenous infusion.

In another aspect, the present invention provides a method for administering an antibody to a subject, for example a mammal, for example a human, comprising a step of administering the liquid antibody formulation or solid antibody formulation of the present application to the subject, for example, the delivery is carried out using a pre-filled syringe as the delivery device.

In another aspect, the present invention provides a use of the liquid antibody formulation or the solid antibody formulationof the present application in manufacturing a delivery device (e.g., a pre-filled syringe) or a medicament for treating, preventing or delaying in a subject a condition associated with.

In some embodiments, the pharmaceutical formulation comprises: (a) 20 mg/ml ± 2 mg/ml of an antibody, (b) a buffer comprising 5 mM ± 1 mM to 20 mM ± 4 mM of histidine or acetate, (c) 0.025% ± 0.01% to 0.1% ± 0.01% (w/v) of a polysorbate, and (d) 6.5% ± 0.5% to 8.6% ± 0.5% (w/v) of sucrose or trehalose, with a pH of 5.3 ± 0.5.

In some embodiments, the pharmaceutical formulation comprises: (a) 20 mg/ml ± 2 mg/ml of an antibody, (b) a buffer comprising 10 mM ± 2 mM of histidine, (c) 0.05% ± 0.01% (w/v) of a polysorbate, and (d) 8.6% ± 0.5% (w/v) of sucrose, with a pH of 5.3 ± 0.1.

In some embodiments, the pharmaceutical formulation comprises: (a) 20 mg/ml ± 2 mg/ml of an antibody, (b) 0.175 mg/ml of L-histidine, (c) 1.86 mg/ml of L-histidine monohydrochloride monohydrate, (d) 0.05% ± 0.01% (w/v) of a polysorbate, and(e) 8.6% ± 0.5% (w/v) of sucrose, with a pH of 5.3 ± 0.1.

In some embodiments, the pharmaceutical formulation comprises: (a) 60 mg/ml ± 6 mg/ml of an antibody, (b) a buffer comprising 5 mM ± 1 mM to 20 mM ± 4 mM of histidine or acetate, (c) 0.025% ± 0.01% to 0.1% ± 0.01% (w/v) of a polysorbate, and (d) 6.5% ± 0.5% to 8.6% ± 0.5% (w/v) of sucrose or trehalose, with a pH of 5.3 ± 0.5.

In some embodiments, the pharmaceutical formulation comprises: (a) 60 mg/ml ± 6 mg/ml of an antibody, (b) a buffer comprising 10 mM ± 2 mM of histidine buffer system, (c) 0.05% ± 0.01% (w/v) of a polysorbate, and(d) 8.6% ± 0.5% (w/v) of sucrose, with a pH of 5.3 ± 0.1.

In some embodiments, the pharmaceutical formulation comprises: (a) 60 mg/ml ± 6 mg/ml of an antibody, (b) 0.175 mg/ml of L-histidine, (c) 1.86 mg/ml of L-histidine monohydrochloride monohydrate, (d) 0.05% ± 0.01% (w/v) of a polysorbate, and (e) 8.6% ± 0.5% (w/v) of sucrose, with a pH of 5.3 ± 0.1.

In some embodiments, the pharmaceutical formulation comprises: (a) 100 mg/ml ± 10 mg/ml of an antibody, (b) a buffer comprising 5 mM ± 1 mM to 20 mM ± 4 mM of histidine or acetate, (c) 0.025% ± 0.01% to 0.1% ± 0.01% (w/v) of a polysorbate, and (d) 6.5% ± 0.5% to 8.6% ± 0.5% (w/v) of sucrose or trehalose, with a pH of 5.3 ± 0.5.

In some embodiments, the pharmaceutical formulation comprises: (a) 100 mg/ml ± 10 mg/ml of an antibody, (b) a buffer comprising 10 mM ± 1 mM of histidine buffer system, (c) 0.05% ± 0.01% (w/v) of a polysorbate, and (d) 7.0% ± 0.5% (w/v) of sucrose, with a pH of 5.3 ± 0.1.

In some embodiments, the pharmaceutical formulation comprises: (a) 100 mg/ml ± 10 mg/ml of an antibody, (b) 0.175 mg/ml of L-histidine, (c) 1.86 mg/ml of L-histidine monohydrochloride monohydrate, (d) 0.05% ± 0.01% (w/v) of a polysorbate, and (e) 7.0% ± 0.5% (w/v) of sucrose, with a pH of 5.3 ± 0.1.

In some embodiments, the pharmaceutical formulation comprises: (a) 150 mg/ml ± 15 mg/ml of an antibody, (b) a buffer comprising 5 mM + 1 mM to 20 mM + 4 mM of histidine or acetate, (c) 0.025% ± 0.01% to 0.1% + 0.01% (w/v) of a polysorbate, and (d) 6.5% + 0.5% to 8.6% + 0.5% (w/v) of sucrose or trehalose, with a pH of 5.3 ± 0.5.

In some embodiments, the pharmaceutical formulation comprises: (a) 150 mg/ml ± 15 mg/ml of an antibody, (b) a buffer comprising 10 mM + 1 mM of histidine buffer system, (c) 0.05% + 0.01% (w/v) of a polysorbate, and (d) 7.0% + 0.5% (w/v) of sucrose, with a pH of 5.3 ± 0.1.

In some embodiments, the pharmaceutical formulation comprises: (a) 150 mg/ml ± 15mg/ml of an antibody, (b) 0.175 mg/ml of L-histidine, (c) 1.86 mg/ml of L-histidine monohydrochloride monohydrate, (d) 0.05% ± 0.01% (w/v) of a polysorbate, and (e) 7.0% ± 0.5% (w/v) of sucrose, with a pH of 5.3 ± 0.1.

In any of these examples, the polysorbates may be polysorbate 80 or polysorbate 20, preferably polysorbate 80.

In an aspect, the present invention provides a pharmaceutical composition, wherein the composition comprises the pharmaceutical formulation as described above or herein, and the composition is contained in a container. In some embodiments, the container is a vial. In some instances, the vial is a 2ml, 5ml, 10ml or 20ml type 1 transparent glass vial. In some embodiments, the container is a syringe. In some instances, a syringe is made of low-tungsten glass. In some embodiments, the container is a pre-filled syringe. In some embodiments, the composition is contained in an automatic syringe.

In an aspect, the present invention provides a kit comprising (i) a container containing the composition which comprises the pharmaceutical formulation as described above or herein, and an instruction for using the composition. In some embodiments, the container is a glass vial. In some embodiments, the container is a pre-filled syringe. In some embodiments, the container is an automatic syringe.

In an aspect, the present invention provides a unit dosage form comprising the pharmaceutical formulation as described above or herein, wherein the amount of the antibody is present at an amount of 0.1 mg to 500 mg. In some instances, the amount of the antibody is 200-400mg. In some embodiments, the unit dosage form is a glass vial, for example, a "penicillin vial" (injection vial). In some embodiments, the unit dosage form is a pre-filled syringe. In some embodiments, the unit dosage form is an automatic syringe.

In any of the pharmaceutical composition, kit or unit dosage form as described above or herein, the container containing the pharmaceutical formulation may comprises a headspace, the headspace comprises a gas, wherein the gas comprises less than 5 vol% of oxygen. In some instances, the gas comprises less than 1 vol% of oxygen. In some instances, the gas comprises no more than 0.1 vol% of oxygen.

In an aspect, the present invention provides a container containing the pharmaceutical composition, wherein the composition comprises the pharmaceutical formulation as described above or herein. In some instances, the container is a syringe. In some instances, the container is a pre-filled syringe. In some instances, the container is an automatic syringe. In some instances, the container is a glass vial.

In various embodiments, any features or components in the examples as described above or herein can be combined, and such combinations are encompassed within the scope of the present invention. Any specific value as described above or herein can be combined with another related value as described above or herein to describe a range, wherein the value represents the upper and lower ends of the range, and such ranges and all values within such ranges are encompassed within the scope of the present invention. Each of the values described above or herein may vary from the recited value by 1%, 5%, 10% or 20%. For example, a 10mM concentration may be expressed as 10mM + 0.1mM (1% variation), 10mM + 0.5mM (5% variation), 10mM + 1mM (10% variation) or 10mM ± 2mM (20% variation).

### II. The anti-MASP-2 antibody of the present invention

In an aspect, the present invention provides anti-MASP-2 antibodies or a fragment (preferably an antigen binding fragment) thereof that specifically bind to MASP-2 (preferably human MASP-2) or its fragments.

In some embodiments, the present invention provides novel monoclonal anti-MASP-2 antibodies or a fragment thereof.

In one aspect, the present disclosure provides isolated antibodies or an antigen binding fragment thereof that specifically bind to MASP-2, wherein the antibody or antigen binding fragment thereof exhibits one or more of the following characteristics: a) having no cross-reactivity with mouse or rat; b) having longer serum half-life in monkey as compared with OMS721; c) having no cross-reactivity with C1s, C1r, MASP1 or MASP3; d) capable of selectively blocking the MBL pathway complement activation; e) capable of specifically binding to human MASP-2 at a K_{D} value of no more than 27.8 nM (or no more than 25 nM, 20 nM, 15 nM, 10 nM, 9 nM, 8 nM, 7 nM, 6 nM, 5 nM, 4 nM, 3 nM, 2 nM, 1 nM, 0.9 nM, 0.8 nM, 0.7 nM, 0.6 nM, 0.5 nM, 0.4 nM, 0.3 nM, 0.2 nM, 0.1 nM, 0.09 nM, 0.08 nM, 0.07 nM, 0.06 nM, 0.05 nM, 0.04 nM, 0.03 nM, 0.02 nM, 0.01 nM, 0.009 nM, 0.008 nM, 0.007 nM, 0.006 nM, 0.005 nM, 0.004 nM, 0.003 nM, 0.002 nM, or 0.001 nM) as measured by Bio-Layer Interferometry; f) capable of blocking complement C3 activation at an IC50 of no more than 0.08 µg/mL (or no more than 0.07 µg/mL, 0.06 µg/mL, 0.05 µg/mL, 0.04 µg/mL, 0.03 µg/mL, 0.02 µg/mL, or 0.01 µg/mL) in 1% human serum as measured by ELISA assay, or at an IC50 of no more than 0.20 µg/mL (or no more than 0.15 µg/mL, 0.10 µg/mL, 0.09 µg/mL, 0.08 µg/mL, 0.07 µg/mL, 0.06 µg/mL, 0.05 µg/mL, 0.04 µg/mL, 0.03 µg/mL, 0.02 µg/mL, or 0.01 µg/mL) in 10% human serum as measured by ELISA assay; g) capable of blocking complement C3 activation in 50% human serum; h) capable of blocking complement C4 activation at an IC50 value of no more than 0.11 µg/mL(or no more than 0.10 µg/mL, 0.09 µg/mL, 0.08 µg/mL, 0.07 µg/mL, 0.06 µg/mL, 0.05 µg/mL, 0.04 µg/mL, 0.03 µg/mL, 0.02 µg/mL, or 0.01 µg/mL) as measured in 2% human serum by ELISA assay, or at an IC50 value of no more than 0.69 µg/mL(or no more than 0.65 µg/mL, 0.6 µg/mL, 0.55 µg/mL, 0.5 µg/mL, 0.45 µg/mL, 0.4 µg/mL, 0.35 µg/mL, 0.3 µg/mL, 0.25 µg/mL, or 0.2 µg/mL, 0.15 µg/mL, 0.1 µg/mL, 0.09 µg/mL, 0.08 µg/mL, 0.07 µg/mL, 0.06 µg/mL, 0.05 µg/mL, 0.04 µg/mL, 0.03 µg/mL, 0.02 µg/mL, or 0.01 µg/mL) as measured in 10% human serum by ELISA assay; i) capable of blocking MAC formation at an IC50 value of no more than 0.27 µg/mL(or no more than 0.25 µg/mL, or 0.2 µg/mL, 0.15 µg/mL, 0.1 µg/mL, 0.09 µg/mL, 0.08 µg/mL, 0.07 µg/mL, 0.06 µg/mL, 0.05 µg/mL, 0.04 µg/mL, 0.03 µg/mL, 0.02 µg/mL, or 0.01 µg/mL) as measured in 2% human serum by ELISA assay.

In one aspect, the present disclosure provides isolated antibodies or antigen binding fragments thereof, comprising
a heavy chain CDR1 comprising the amino acid sequence of DYYIN (SEQ ID NO: 1),
a heavy chain CDR2 comprising the amino acid sequence of **WIFPGSX₁SX₂YX₃X₄X₅X₆FX₇X₈** (SEQ ID NO: 2), and
a heavy chain CDR3 comprising the amino acid sequence of **GDRSGPFX₉Y** (SEQ ID NO: 3); and/or
a light chain CDR1 comprising the amino acid sequence of **KSSQSLLYSNGKTYLN** (SEQ ID NO: 4),
a light chain CDR2 comprising the amino acid sequence of **LVSKLDS** (SEQ ID NO: 5), and a light chain CDR3 comprising the amino acid sequence of **VQX₁₀THFPFT** (SEQ ID NO: 6);
wherein X₁ is E, D or G, X₂ is A or P, X₃ is H or Y, X₄ is S or N, X₅ is E or Q, X₆ is K or N, X₇ is K or Q, X₈ is A or G, X₉ is A or P, and X₁₀ is V or G.

In certain embodiments, the antibodies or antigen binding fragments thereof comprise:
a heavy chain CDR1 comprising the amino acid sequence of SEQ ID NO: 1, and/or
a heavy chain CDR2 comprising the amino acid sequence selected from the group consisting of: SEQ ID NO: 7, SEQ ID NO: 8, SEQ ID NO: 9 and SEQ ID NO: 10, and/or
a heavy chain CDR3 comprising the amino acid sequence selected from the group consisting of: SEQ ID NO: 11 and SEQ ID NO: 12; and/or
a light chain CDR1 comprising the amino acid sequence of SEQ ID NO: 4, and/or
a light chain CDR2 comprising the amino acid sequence selected from the group consisting of: SEQ ID NO: 5, and/or
a light chain CDR3 comprising the amino acid sequence of SEQ ID NO: 13 and SEQ ID NO: 14.

In one aspect, the present disclosure provides isolated antibodies or antigen binding fragments thereof, comprising
a heavy chain CDR1 comprising the amino acid sequence of SEQ ID NO: 1, a heavy chain CDR2 comprising the amino acid sequence of SEQ ID NO: 7, and a heavy chain CDR3 comprising the amino acid sequence of SEQ ID NO: 11; or
a heavy chain CDR1 comprising the amino acid sequence of SEQ ID NO: 1, a heavy chain CDR2 comprising the amino acid sequence of SEQ ID NO: 9, and a heavy chain CDR3 comprising the amino acid sequence of SEQ ID NO: 12; or
a heavy chain CDR1 comprising the amino acid sequence of SEQ ID NO: 1, a heavy chain CDR2 comprising the amino acid sequence of SEQ ID NO: 10, and a heavy chain CDR3 comprising the amino acid sequence of SEQ ID NO: 11; or
a heavy chain CDR1 comprising the amino acid sequence of SEQ ID NO: 1, a heavy chain CDR2 comprising the amino acid sequence of SEQ ID NO: 8, and a heavy chain CDR3 comprising the amino acid sequence of SEQ ID NO: 11.

In certain embodiments, the antibodies or antigen binding fragments thereof further comprise:
a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 13; or
a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 14.

In certain embodiments, the antibodies or antigen binding fragments thereof comprise:
a heavy chain CDR1 comprising the amino acid sequence of SEQ ID NO: 1, a heavy chain CDR2 comprising the amino acid sequence of SEQ ID NO: 7, and a heavy chain CDR3 comprising the amino acid sequence of SEQ ID NO: 11, a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 13; or
a heavy chain CDR1 comprising the amino acid sequence of SEQ ID NO: 1, a heavy chain CDR2 comprising the amino acid sequence of SEQ ID NO: 9, and a heavy chain CDR3 comprising the amino acid sequence of SEQ ID NO: 12, a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 13; or
a heavy chain CDR1 comprising the amino acid sequence of SEQ ID NO: 1, a heavy chain CDR2 comprising the amino acid sequence of SEQ ID NO: 10, and a heavy chain CDR3 comprising the amino acid sequence of SEQ ID NO: 11, a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 14; or
a heavy chain CDR1 comprising the amino acid sequence of SEQ ID NO: 1, a heavy chain CDR2 comprising the amino acid sequence of SEQ ID NO: 8, and a heavy chain CDR3 comprising the amino acid sequence of SEQ ID NO: 11, a light chain CDR1 comprising the amino acid sequence of SEQ ID NO: 4, a light chain CDR2 comprising the amino acid sequence of SEQ ID NO: 5, and a light chain CDR3 comprising the amino acid sequence of SEQ ID NO: 13.

In certain embodiments, the antibodies or antigen binding fragments thereof comprise:
a heavy chain variable region comprising the amino acid sequence of SEQ ID NO: 15, or a sequence having at least 80% sequence identity thereof;
a heavy chain variable region comprising the amino acid sequence of SEQ ID NO: 17, or a sequence having at least 80% sequence identity thereof;
a heavy chain variable region comprising the amino acid sequence of SEQ ID NO: 18, or a sequence having at least 80% sequence identity thereof;
a heavy chain variable region comprising the amino acid sequence of SEQ ID NO: 20, or a sequence having at least 80% sequence identity thereof;
a heavy chain variable region comprising the amino acid sequence of SEQ ID NO: 22, or a sequence having at least 80% sequence identity thereof;
a heavy chain variable region comprising the amino acid sequence of SEQ ID NO: 24, or a sequence having at least 80% sequence identity thereof; or
a heavy chain variable region comprising the amino acid sequence of SEQ ID NO: 26, or a sequence having at least 80% sequence identity thereof.

In certain embodiments, the antibodies or antigen binding fragments thereof comprise:
a light chain variable region comprising the amino acid sequence of SEQ ID NO: 16, or a sequence having at least 80% sequence identity thereof;
a light chain variable region comprising the amino acid sequence of SEQ ID NO: 19, or a sequence having at least 80% sequence identity thereof;
a light chain variable region comprising the amino acid sequence of SEQ ID NO: 28, or a sequence having at least 80% sequence identity thereof; or
a light chain variable region comprising the amino acid sequence of SEQ ID NO: 30, or a sequence having at least 80% sequence identity thereof.

In certain embodiments, the antibodies or antigen binding fragments thereof comprise the heavy chain variable region comprising an amino acid sequence having at least 80% sequence identity to SEQ ID NO: 15, SEQ ID NO: 17, SEQ ID NO: 18, SEQ ID NO: 20, SEQ ID NO: 22, SEQ ID NO: 24, or SEQ ID NO: 26, and/or the light chain variable region comprises an amino acid sequence having at least 80% sequence identity to SEQ ID NO: 16, SEQ ID NO: 19, SEQ ID NO: 28, or SEQ ID NO: 30.

In certain embodiments, the antibodies or antigen binding fragments thereof comprise:
a heavy chain variable region comprising the amino acid sequence of SEQ ID NO: 15, and a light chain variable region comprising the amino acid sequence of SEQ ID NO: 16;
a heavy chain variable region comprising the amino acid sequence of SEQ ID NO: 17, and a light chain variable region comprising the amino acid sequence of SEQ ID NO: 16;
a heavy chain variable region comprising the amino acid sequence of SEQ ID NO: 18, and a light chain variable region comprising the amino acid sequence of SEQ ID NO: 19;
a heavy chain variable region comprising the amino acid sequence of SEQ ID NO: 20, and a light chain variable region comprising the amino acid sequence of SEQ ID NO: 28;
a heavy chain variable region comprising the amino acid sequence of SEQ ID NO: 20, and a light chain variable region comprising the amino acid sequence of SEQ ID NO: 30;
a heavy chain variable region comprising the amino acid sequence of SEQ ID NO: 22, and a light chain variable region comprising the amino acid sequence of SEQ ID NO: 28;
a heavy chain variable region comprising the amino acid sequence of SEQ ID NO: 22, and a light chain variable region comprising the amino acid sequence of SEQ ID NO: 30;
a heavy chain variable region comprising the amino acid sequence of SEQ ID NO: 24, and a light chain variable region comprising the amino acid sequence of SEQ ID NO: 28;
a heavy chain variable region comprising the amino acid sequence of SEQ ID NO: 24, and a light chain variable region comprising the amino acid sequence of SEQ ID NO: 30;
a heavy chain variable region comprising the amino acid sequence of SEQ ID NO: 26, and a light chain variable region comprising the amino acid sequence of SEQ ID NO: 28; or
a heavy chain variable region comprising the amino acid sequence of SEQ ID NO: 26, and a light chain variable region comprising the amino acid sequence of SEQ ID NO: 30.

In certain embodiments, the antibodies or antigen binding fragments thereof further comprise one or more amino acid residue mutations yet retain specific binding affinity to MASP-2. In certain embodiments, at least one of the mutations is conservative substitution, or all of the mutations are conservative substitutions. In certain embodiments, at least one of the mutations is in one or more of the CDR sequences, and/or in one or more of the non-CDR sequences of the heavy chain variable region or light chain variable region.

In certain embodiments, the antibodies or antigen binding fragments thereof further comprise an immunoglobulin constant region, optionally comprise a heavy chain constant region of IgG, and/or a light chain constant region. In certain embodiments, the constant region comprises a mouse constant region, a rabbit constant region, or a human constant region, optionally the constant region comprises a constant region of human IgG1, IgG2, IgG3, or IgG4. In certain embodiments, the heavy chain constant region comprises one or more amino acid substitutions relative to a wild-type human IgG constant region at amino acid residue 252, 254 or 256, optionally the amino acid substitution at amino acid residue 252 is a substitution with tyrosine, the amino acid substitution at amino acid residue 254 is a substitution with threonine, an amino acid substitution at amino acid residue 256 is a substitution with glutamic acid. In certain embodiments, the heavy chain constant region comprises a sequence having at least 80% sequence identity thereof, provided that the amino acid residue 252 is substituted with tyrosine, the amino acid residue 254 is substituted with threonine, and the amino acid residue 256 is substituted with glutamic acid.

In certain embodiments, the antibodies or antigen binding fragments thereof are a monoclonal antibody, a bispecific antibody, a multi-specific antibody, a recombinant antibody, a chimeric antibody, a humanized antibody, a labeled antibody, a bivalent antibody, an anti-idiotypic antibody, a fusion protein, a dimerized or polymerized antibody, or a modified antibody (e.g. glycosylated antibody).

In certain embodiments, the antibodies or antigen binding fragments thereof are a diabody, a Fab, a Fab', a F(ab')₂, a Fd, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), an scFv dimer (bivalent diabody), a multispecific antibody, a camelized single domain antibody, a nanobody, a domain antibody, or a bivalent domain antibody.

In certain embodiments, the antibodies or antigen binding fragments thereof specifically bind to MASP-2 and have no cross-reactivity with C1s, C1r, MASP1 or MASP3.

In some preferable embodiments of the present invention, the anti-MASP-2 antibodies or antigen binding fragments comprise sequences as shown in the tables below.

**Table 1 CDR sequences of mouse anti-MASP-2 antibodies and humanized 129C10 antibodies**

| | | **CDR1** | **CDR2** | **CDR3** |
|---|---|---|---|---|
| **129C10 mouse/chimeric antibody, and 129C10 HaLa/HaLb/ HbLa/HbLb** | **VH** | SEQ ID NO: 1 | SEQ ID NO: 7 WIFPGSESAYHSEKF KA | SEQ ID NO: 11 |
| | | DYYIN | | GDRSGPFAY |
| **129C10 mouse/chimeric antibody, and 129C10 HaLa/HaLb/ HbLa/HbLb** | **VL** | SEQ ID NO: 4 KSSQSLLYSNGKT YLN | SEQ ID NO: 5 | SEQ ID NO: 13 |
| | | | LVSKLDS | VQVTHFPFT |
| **160D10 mouse/chimeric antibody** | **VH** | SEQ ID NO: 1 | SEQ ID NO: 9 WIFPGSDSAYYNEKF KG | SEQ ID NO: 12 |
| | | DYYIN | | GDRSGPFPY |
| **160D10 mouse/chimeric antibody** | **VL** | SEQ ID NO: 4 KSSQSLLYSNGKT YLN | SEQ ID NO: 5 | SEQ ID NO: 13 |
| | | | LVSKLDS | VQVTHFPFT |
| **125D5 mouse/chimeric antibody** | **VH** | SEQ ID NO: 1 | SEQ ID NO: 10 WIFPGSGSPYYNENF KG | SEQ ID NO: 11 |
| | | DYYIN | | GDRSGPFAY |
| **125D5 mouse/chimeric antibody** | **VL** | SEQ ID NO: 4 KSSQSLLYSNGKT YLN | SEQ ID NO: 5 | SEQ ID NO: 14 |
| | | | LVSKLDS | VQGTHFPFT |
| **129C10 HcLa, HcLb, HdLa, HdLb antibody** | **VH** | SEQ ID NO: 1 | SEQ ID NO: 8 WIFPGSESAYHSQKF QG | SEQ ID NO: 11 |
| | | DYYIN | | GDRSGPFAY |
| **129C10 HcLa, HcLb, HdLa, HdLb antibody** | **VL** | SEQ ID NO: 4 KSSQSLLYSNGKT YLN | SEQ ID NO: 5 | SEQ ID NO: 13 |
| | | | LVSKLDS | VQVTHFPFT |

**Table 2 Sequences of anti-MASP-2 antibodies of the present invention**

| **2-1 Variable region sequences of murine or chimeric antibodies** | | |
|---|---|---|
| **129C10** | **HC** | **Amino acid sequence (SEQ ID NO: 15):** |
| **mouse/chime ric antibody** | | QVQLQQSGPELEKPGTSVKISCEASGYTFTDYYINWVQQR PGQGLEWIGWIFPGSESAYHSEKFKAKATLTVDTSSTTAY MLLTSLTSEDSAVYFCTRGDRSGPFAYWGQGTLVTVSA |
| **129C10** | **LC** | **Amino acid sequence (SEQ ID NO: 16):** |
| **mouse/chime ric antibody** | | DVVMTQTPLTLSVTIGQPASISCKSSQSLLYSNGKTYLNWL LQRPGQSPKRLIYLVSKLDSGVPDRFTGSGSGADFTLKISR VEAEDLGVYYCVQVTHFPFTFGTGTKLEIK |
| **160D10** | **HC** | **Amino acid sequence (SEQ ID NO: 17):** |
| **mouse/chime ric antibody** | | QVQLQQSGPELVKPGASVKISCKASGYTFTDYYINWVKQ RPGQGLEWVGWIFPGSDSAYYNEKFKGKATLTVDTSSST AYMLLSSLTSEDSAVYFCARGDRSGPFPYWGQGTLVTVS A |
| **160D10** | **LC** | **Amino acid sequence (SEQ ID NO: 16):** |
| **mouse/chime ric antibody** | | DVVMTQTPLTLSVTIGQPASISCKSSQSLLYSNGKTYLNWL LQRPGQSPKRLIYLVSKLDSGVPDRFTGSGSGADFTLKISR VEAEDLGVYYCVQVTHFPFTFGTGTKLEIK |
| **125D5** | **HC** | **Amino acid sequence (SEQ ID NO: 18):** |
| **mouse/chime ric antibody** | | QVQLQQSGPDLVKPGTSVKISCTASGYTFTDYYINWVKQR PGQGLEWIGWIFPGSGSPYYNENFKGRAMFTVDYSSSSAY MLLSSLTSEDSAVYFCTRGDRSGPFAYWGQGTLVTVSA |
| **125D5** | **LC** | **Amino acid sequence (SEQ ID NO: 19):** |
| **mouse/chime ric antibody** | | DVVMTQTPLTLSVTIGQPASISCKSSQSLLYSNGKTYLNWL LQRPGQSPKRLIYLVSKLDSGVPDRFTGSGSGADFTLKISR VEAEDLGVYYCVQGTHFPFTFGTGTKLEIK |

| **2-2 Variable region sequences of humanized 129C10** | | |
|---|---|---|
| **Heavy chain variable region of 129C10** | **Ha** | **Amino acid sequence (SEQ ID NO: 20):** |
| | | QVQLVQSGAEVKKPGASVKVSCKASGYTFTDYYINWVRQ APGQGLEWMGWIFPGSESAYHSEKFKARVTMTVDTSISTA YMELSRLRSDDTAVYYCTRGDRSGPFAYWGQGTLVTVSS |
| **Heavy chain variable region of humanized 129C10** | **Hb** | **Amino acid sequence (SEQ ID NO: 22):** |
| | | QVQLVQSGAEVKKPGASVKVSCKASGYTFTDYYINWVRQ APGQGLEWMGWIFPGSESAYHSEKFKARATLTVDTSISTA YMELSRLRSDDTAVYYCTRGDRSGPFAYWGQGTLVTVSS |
| **Heavy chain variable region of humanized 129C10** | **Hc** | **Amino acid sequence (SEQ ID NO: 24):** |
| | | QVQLVQSGAEVKKPGASVKVSCKASGYTFTDYYINWVRQ APGQGLEWMGWIFPGSESAYHSQKFQGRVTMTVDTSIST AYMELSRLRSDDTAVYYCTRGDRSGPFAYWGQGTLVTVS S |
| **Heavy chain variable region of humanized 129C10** | **Hd** | **Amino acid sequence (SEQ ID NO: 26):** |
| | | QVQLVQSGAEVKKPGASVKVSCKASGYTFTDYYINWVRQ APGQGLEWMGWIFPGSESAYHSQKFQGRATLTVDTSISTA YMELSRLRSDDTAVYYCTRGDRSGPFAYWGQGTLVTVSS |
| **Light chain variable region of humanized 129C10** | **La** | **Amino acid sequence (SEQ ID NO: 28):** |
| | | DVVMTQSPLSLPVTLGQPASISCKSSQSLLYSNGKTYLNW LQQRPGQSPRRLIYLVSKLDSGVPDRFSGSGSGTDFTLKIS RVEAEDVGVYYCVQVTHFPFTFGQGTKLEIK |
| **Light chain variable region of humanized 129C10** | **Lb** | **Amino acid sequence (SEQ ID NO: 30):** |
| | | DVVMTQSPLSLPVTLGQPASISCKSSQSLLYSNGKTYLNW LQQRPGQSPRRLIYLVSKLDSGVPDRFSGSGSGADFTLKIS RVEAEDVGVYYCVQVTHFPFTFGQGTKLEIK |
| **2-3 Sequences of 129C10-hu-YTE antibody** | | |
| **Heavy chain constant region of IgG4 YTE** | | **Amino acid sequence (SEQ ID NO: 35):** |
| | | ASTKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVFL FPPKPKDTLYITREPEVTCVVVDVSQEDPEVQFNWYVDGV EVHNAKTKPREEQFNSTYRVVSVLTVLHQDWLNGKEYKC KVSNKGLPSSIEKTISKAKGQPREPQVYTLPPSQEEMTKNQ VSLTCLVKGFYPSDIAVEWESNGQPENNYKTTPPVLDSDG SFFLYSRLTVDKSRWQEGNVFSCSVMHEALHNHYTQKSL SLSLGK |
| **Light chain constant region of IgG4** | | **Amino acid sequence (SEQ ID NO: 34):** |
| | | |
| **Heavy chain of Hu129C10 (hIgG4_YT E kappa) antibody** | **HC** | **Amino acid sequence (SEQ ID NO: 36):** |
| | | QVQLVQSGAEVKKPGASVKVSCKASGYTFTDYYINWVRQ APGQGLEWMGWIFPGSESAYHSEKFKARVTMTVDTSISTA YMELSRLRSDDTAVYYCTRGDRSGPFAYWGQGTLVTVSS ASTKGPSVFPLAPCSRSTSESTAALGCLVKDYFPEPVTVSW NSGALTSGVHTFPAVLQSSGLYSLSSVVTVPSSSLGTKTYT CNVDHKPSNTKVDKRVESKYGPPCPPCPAPEFLGGPSVFL FPPKPKDTLYITREPEVTCVVVDVSQEDPEVQFNWYVDGV EVHNAKTKPREEQFNSTYRVVSVLTVLHQDWLNGKEYKC KVSNKGLPSSIEKTISKAKGQPREPQVYTLPPSQEEMTKNQ VSLTCLVKGFYPSDIAVEWESNGQPENNYKTTPPVLDSDG SFFLYSRLTVDKSRWQEGNVFSCSVMHEALHNHYTQKSL SLSLGK |
| **Light chain of Hu129C10 (hIgG4_YT E kappa) antibody** | **LC** | **Amino acid sequence (SEQ ID NO: 29):** |
| | | DVVMTQSPLSLPVTLGQPASISCKSSQSLLYSNGKTYLNW LQQRPGQSPRRLIYLVSKLDSGVPDRFSGSGSGTDFTLKIS RVEAEDVGVYYCVQVTHFPFTFGQGTKLEIKRTVAAPSVF IFPPSDEQLKSGTASVVCLLNNFYPREAKVQWKVDNALQS GNSQESVTEQDSKDSTYSLSSTLTLSKADYEKHKVYACEV THQGLSSPVTKSFNRGEC |

In certain embodiments, the antibodies and antigen-binding fragments thereof provided herein are humanized. A humanized antibody or antigen-binding fragment is desirable in its reduced immunogenicity in human. A humanized antibody is chimeric in its variable regions, as non-human CDR sequences are grafted to human or substantially human FR sequences. Humanization of an antibody or antigen-binding fragment can be essentially performed by substituting the non-human (such as murine) CDR genes for the corresponding human CDR genes in a human immunoglobulin gene (see, for example, Jones et al. (1986) Nature 321:522-525; Riechmann et al. (1988) Nature 332:323-327; Verhoeyen et al. (1988) Science 239:1534-1536). In certain embodiments, the humanized antibodies or antigen-binding fragments provided herein are composed of substantially all human sequences except for the CDR sequences which are non-human. In some embodiments, the variable region FRs, and constant regions if present, are entirely or substantially from human immunoglobulin sequences. The human FR sequences and human constant region sequences may be derived different human immunoglobulin genes, for example, FR sequences derived from one human antibody and constant region from another human antibody. In some embodiments, the humanized antibody or antigen-binding fragment comprise human heavy/light chain FR1-4.

In certain embodiments, the humanized antibodies and antigen-binding fragment thereof provided herein comprise one or more heavy chain FR sequences of human germline framework sequence VH/1-2, and/or one or more light chain FR sequences of human germline framework sequence VK/2-30 without or without back mutations. Back mutations can be introducted in to the human germline framework sequence, if needed. In certain embodiments, the humanized antibody 129C10 may contain one or more mutations selected from the group consisting of: R71V, A93T, V67A, M69L, all based on Kabat numbering, in heavy chain framework sequence VH/1-2, and/or A65G, K64Q, E61Q, all based on Kabat numbering, in heavy chain CDR2. The humanized antibody 129C10 may contain one or more back mutations selected from the group consisting of: F36L and T69A, all based on Kabat numbering, in light chain framework sequence VK/2-30.

In certain embodiments, the humanized heavy and light chains of the antibodies and antigen-binding fragments thereof provided herein are substantially non-immunogenic in humans and retain substantially the same affinity as or even higher affinity than the parent antibody to MASP-2.

In some embodiments, the anti-MASP-2 antibody described herein is a human antibody. Various kinds of well-known techniques in the art could be used to prepare human antibodies. Human antibodies are described in van Dijk and van de Winkel, Curr.Opin.Pharmacol 5:368-74(2001), and Lonberg, Curr.Opin.Immunol 20:450-459(2008).

In some embodiments, the anti-MASP-2 antibody described herein is a chimeric antibody.

### III. Methods for treatment and Use of the antibody or formulation of the present invention

The present disclosure also provides methods of treatment comprising: administering a therapeutically effective amount of the antibody or antigen-binding fragment, the antibody pharmaceutical formulations, the pharmaceutical compositions, the kit, or the unit dosage form as provided herein to a subject in need thereof, thereby treating or preventing a MASP-2 dependent complement activation related disease or condition.

The present disclosure provides methods of inhibiting MASP-2 dependent complement activation in a subject in need thereof, or methods of treating or preventing a disease or a disorder associated with MASP-2 dependent complement activation, the methods comprising administering a therapeutically effective amount of the antibody or antigen-binding fragment, the antibody pharmaceutical formulations, the pharmaceutical compositions, the kit, or the unit dosage form as provided herein to the subject.

In another aspect, methods are provided to treat a condition in a subject that would benefit from inhibition of MASP-2 dependent complement activation, comprising administering a therapeutically effective amount of the antibody or antigen-binding fragment, the antibody pharmaceutical formulations, the pharmaceutical compositions, the kit, or the unit dosage form as provided herein to a subject in need thereof.

In another aspect, the present disclosure further provides a method of reducing level of serum C4 level in a subject, comprising administering to the subject a therapeutically effective amount of the antibody or antigen binding fragment thereof, the antibody pharmaceutical formulations, the pharmaceutical compositions, the kit, or the unit dosage form provided herein, thereby reducing the level of serum C4 in the subject.

In another aspect, the present disclosure further provides a method of treating a condition in a subject that would benefit from reduction of serum C4 level in a subject, or treating or preventing a disease or a disorder associated with abnormal (e.g. elevated) serum C4 level, comprising administering to the subject a therapeutically effective amount of the antibody or antigen binding fragment thereof, the antibody pharmaceutical formulations, the pharmaceutical compositions, the kit, or the unit dosage form provided herein, thereby treating the condition.

In certain embodiments, the method further comprises administration of a second therapeutic agent.

In certain embodiments, the subject is human. In certain embodiments, the administration is via oral, nasal, intravenous, subcutaneous, sublingual, or intramuscular administration.

In one aspect, the present disclosure further provides use of the antibodies or antigen binding fragments thereof, the antibody pharmaceutical formulations, the pharmaceutical compositions, the kit, or the unit dosage form provided herein in the manufacture of a medicament, wherein the medicament is used for one or more of the follows:
(1) for inhibiting MASP-2 dependent complement activation;
(2) for treating or preventing a disease or a disorder associated with MASP-2 dependent complement activation;
(3) for treating a condition in a subject that would benefit from inhibition of MASP-2 dependent complement activation;
(4) for reducing level of serum C4 level in a subject;
(5) for treating a condition in a subject that would benefit from reduction of serum C4 level in a subject;
(6) for treating or preventing a disease or a disorder associated with abnormal (e.g. elevated) serum C4 level.

In certain embodiments, said disease or condition is an autoimmune disease, a vascular condition, an ischemia-reperfusion injury, atherosclerosis, an inflammation, a pulmonary condition, extracorporeal reperfusion procedure, a musculoskeletal condition, a renal condition, a skin condition, an organ or tissue transplant procedure, a nervous system disorder or injury, a blood disorder, urogenital condition, a non-obese diabetes or a complication associated with Type 1 or Type 2 diabetes, cancer, endocrine disorder, an ophthalmologic condition, or COVID-19.

In some particular embodiments, said autoimmune disease comprises thrombotic microangiopathies (TMAs), atypical hemolytic uremic syndrome (aHUS), hematopoietic transplant-associated thrombotic microangiopathy (TA-TMA), lupus nephritis, systemic lupus erythematosus (SLE) and IgA nephropathy.

In some particular embodiments, the vascular condition comprises a cardiovascular condition, a cerebrovascular condition, a peripheral (e.g., musculoskeletal) vascular condition, a renovascular condition, a mesenteric/enteric vascular condition, revascularization to transplants and/or replants, vasculitis, Henoch-Schonlein purpura nephritis, systemic lupus erythematosus-associated vasculitis, vasculitis associated with rheumatoid arthritis, immune complex vasculitis, Takayasu's disease, dilated cardiomyopathy, diabetic angiopathy, Kawasaki's disease (arteritis), venous gas embolus (VGE), and restenosis following stent placement, rotational atherectomy and percutaneous transluminal coronary angioplasty (PTCA).

In some particular embodiments, the ischemia-reperfusion injury comprises an ischemia-reperfusion injury associated with aortic aneurysm repair, cardiopulmonary bypass, vascular reanastomosis in connection with organ transplants and/or extremity/digit replantation, stroke, myocardial infarction, and hemodynamic resuscitation following shock and/or surgical procedures.

In some particular embodiments, the inflammation comprises inflammatory gastrointestinal disorder comprising pancreatitis, Crohn's disease, ulcerative colitis, irritable bowel syndrome and diverticulitis.

In some particular embodiments, the pulmonary condition comprises acute respiratory distress syndrome, transfosion-related acute lung injury, ischemia/reperfosion acute lung injury, chronic obstructive pulmonary disease, asthma, Wegener's granulomatosis, antiglomerular basement membrane disease (Goodpasture's disease), meconium aspiration syndrome, bronchiolitis obliterans syndrome, idiopathic pulmonary fibrosis, acute lung injury secondary to burn, non- cardiogenic pulmonary edema, transfosion-related respiratory depression, emphysema, cystic fibrosis, SARS-CoV, MERS-CoV and SARS-CoV-2(Covid-19) related pathogenesis.

In some particular embodiments, the extracorporeal reperfusion procedure comprises hemodialysis, plasmapheresis, leukopheresis, exfracorporeal membrane oxygenator (ECMO), heparin-induced extracorporeal membrane oxygenation LDL precipitation (HELP) and cardiopulmonary bypass (CPB).

In some particular embodiments, the musculoskeletal condition comprises osteoarthritis, rheumatoid arthritis, juvenile rheumatoid arthritis, gout, neuropathic arthropathy, psoriatic arthritis, spondyloarthropathy, crystalline arthropathy and systemic lupus erythematosus (SLE).

In some particular embodiments, the renal condition comprises mesangioproliferative glomerulonephritis, membranous glomerulonephritis, membranoproliferative glomerulonephritis (mesangiocapillary glomerulonephritis), acute postinfectious glomerulonephritis (poststreptococcal glomerulonephritis), cryoglobulinemic glomerulonephritis, lupus nephritis, Henoch- Schonlein purpura nephritis and IgA nephropathy.

In some particular embodiments, the skin condition comprises psoriasis, autoimmune bullous dermatoses, eosinophilic spongiosis, bullous pemphigoid, Epidermolysis bullosa acquisita (EBA), herpes gestationis, thermal burn injury and chemical burn injury.

In some particular embodiments, the organ or tissue transplant procedure comprises organ allotransplantation, organ xenotransplantation organ and tissue graft.

In some particular embodiments, the nervous system disorder or injury comprises multiple sclerosis, myasthenia gravis, Huntington's disease, amyotrophic lateral sclerosis, Guillain Barre syndrome, reperfusion following stroke, degenerative discs, cerebral trauma, Parkinson's disease, Alzheimer's disease, Miller-Fisher syndrome, cerebral frauma and/or hemorrhage, demyellination and meningitis.

In some particular embodiments, the blood disorder comprises sepsis, severe sepsis, septic shock, acute respiratory distress syndrome resulting from sepsis, systemic inflammatory response syndrome, hemorrhagic shock, hemolytic anemia, autoimmune thrombotic thrombocytopenic purpura and hemolytic uremic syndrome.

In some particular embodiments, the urogenital condition comprises painful bladder disease, sensory bladder disease, chronic abacterial cystitis, interstitial cystitis, infertility, placental dysfunction and miscarriage and pre-eclampsia.

In some particular embodiments, the endocrine disorder comprises Hashimoto's thyroiditis, stress, anxiety and hormonal disorders involving regulated release of prolactin, growth or other insulin-like growth factor and adrenocorticotropin from the pituitary.

In some particular embodiments, the ophthalmologic condition comprises age-related macular degeneration.

The methods for treatment of the present invention comprise administrating to a subject any formulations, pharmaceutical compositions and unit dosage forms comprising the monoclonal anti-antibodies or an antigen binding fragment thereof of the present invention that specifically bind to human MASP-2. The subject that get administrated could be any human or non-human animals in need of such treatment.

The following examples are provided to better illustrate the claimed invention and are not to be interpreted as limiting the scope of the invention. All specific compositions, materials, and methods described below, in whole or in part, fall within the scope of the present invention. These specific compositions, materials, and methods are not intended to limit the invention, but merely to illustrate specific embodiments falling within the scope of the invention. One skilled in the art may develop equivalent compositions, materials, and methods without the exercise of inventive capacity and without departing from the scope of the invention. It will be understood that many variations can be made in the procedures herein described while still remaining within the bounds of the present invention. It is the intention of the inventors that such variations are included within the scope of the invention.

### IV. Others

In an aspect, the present invention provides nucleic acids encoding any of the above anti-MASP-2 antibody or a fragment thereof. In one embodiment, a vector comprising the nucleic acids is provided. In one embodiment, the vector is an expression vector. In one embodiment, a host cell comprising the vector is provided. In one embodiment, the host cell is eukaryotic. In another embodiment, the host cell is selected from a yeast cell, a mammalian cell, or other cells suitable for the preparation of the antibody or an antigen-binding fragment thereof. In another embodiment, the host cell is prokaryotic.

In one embodiment, the present invention provides a method for the preparation of an anti-MASP-2 antibody or a fragment thereof (preferably an antigen-binding fragment thereof), wherein the method comprises culturing the host cells under a condition suitable for expressing the nucleic acids encoding the antibody or the fragment thereof (preferably, the antigen-binding fragment thereof), and optionally isolating the antibody or the fragment thereof (preferably, the antigen-binding fragment thereof). In certain embodiments, the method further comprises recovering the anti-MASP-2 antibody or the fragment thereof (preferably the antigen-binding fragment thereof) from the host cell.

In an aspect, the present invention relates to a method for detecting the MASP-2 protein in a sample, the method comprises (a) contacting the sample with any of the anti-MASP-2 antibody formulation of the present invention or the antibody of the present invention; and (b) detecting the formation of a complex between the anti-MASP-2 antibody or the fragment thereof and the MASP-2 protein. In one embodiment, the anti-MASP-2 antibody is labeled with a detectable label.

The present invention also encompasses any combination of any of the embodiments described herein. Any of the embodiments described herein or any combination thereof is applicable to any and all of the formulations or the anti-MASP-2 antibodies or the fragments thereof, the methods and the uses of described in the present invention.

### BRIEF DESCRIPTION OF FIGURES

Figure 1 shows that MASP-2 antibodies block activation of the complement C3.
Figure 2 shows that MASP-2 antibodies block activation of the complement C4.
Figure 3 shows that MASP-2 antibodies block formation of the MAC.
Figure 4 shows that chimeric and humanized MASP-2 antibodies block activation of the complement C3.
Figure 5 shows that MASP-2 antibodies 129C10-hu and OMS721-analog block complement C4 activation in 2% human serum.
Figure 6 shows that MASP-2 antibodies 129C10-hu and OMS721-analog blocks MAC formation in 2% human serum.
Figure 7 shows that MASP-2 antibody blocks C3 activation in 1% human serum.
Figure 8 shows that MASP-2 antibody blocks C3 activation in 10% human serum.
Figure 9 shows that MASP-2 antibody blocks C3 activation in 50% human serum.
Figure 10 shows that MASP-2 antibody blocks C4 activation in 10% human serum.
Figure 11 shows affinity kinetics of MASP-2 antibody 129C10-hu to hMASP-2.
Figures 12A-12E show ELISA binding of MASP-2 antibody 129C10-Hu to human C1s (12A), C1r (12B), MASP1(12C), MASP3 (12D) and MASP2 (12E).
Figures 13A-13B show ELISA binding of MASP-2 antibodies 129C10-hu (13A) and OMS721-analog (13B) to human, cynomolgus, rat and mouse MASP2.
Figure 14 shows cross reactivity of MASP-2 antibody 129C10-hu to cynomolgus MASP-2.
Figure 15 shows selectivity of MASP-2 antibody 129C10-hu in neutralizing three complement activation pathways.
Figures 16A-16B show binding kinetic curves of MASP-2 antibodies 129C10-hu-WT (16A) and 129C10-hu-YTE (16B) to human FcRn.
Figure 17 shows Pharmacokinetic (PK) results of MASP-2 antibodies 129C10-hu-WT and 129C10-hu-YTE in cynomolgus serum.
Figures 18A-18C show PK and Pharmacodynamic (PD) results of MASP-2 antibodies 129C10-hu-WT and 129C10-hu-YTE in cynomolgus serum.
Figure 19 shows reduction effect of 129C10-hu on serum C4c in monkeys following the first dose.
Figure 20 shows reduction effect of 129C10-hu on serum C4c in monkeys following the fourth dose.
Figure 21 to Figure 23 relate to the experimental results of F12-F20 formulae (which are used for screening the optimal pH value of the formulation) after being placed at a high temperature of 40°C for 14 days. Figure 21 shows the results of HMW% of said nine formulae detected by SEC. Figure 22 shows the results of LMW% of said nine formulae detected by NR CE-SDS. Figure 23 shows the results of the percentage of main peak, acidic peak and basic peak of said nine formulae detected by CEX.
Figure 24 to Figure 29 relate to the experimental results of F1-F5 formulae which are used for screening the optimal auxiliary materials. Figure 24 ro Figure 26 are respectively SEC, NR CE-SDS and CEX results of the five formulae after being placed at a high temperature of 40°C for 14 days. Figure 27 shows CEX results of each formula under shaking for 14 days. Figure 28-29 respectively show CEX and NR CE-SDS results of each formula under stirring for 6 hours.
Figure 30 to Figure 32 relate to the experimental results of F6-F11 formulae which are used for screening the optimal surfactant. Figure 30 shows SEC results of six formulae after being placed at high temperature of 40°C for 14 days. Figure 31-32 respectively show the stability results of each formula under stirring at 100 rpm for 6 hours, deteced by NR CE-SDS and CEX.

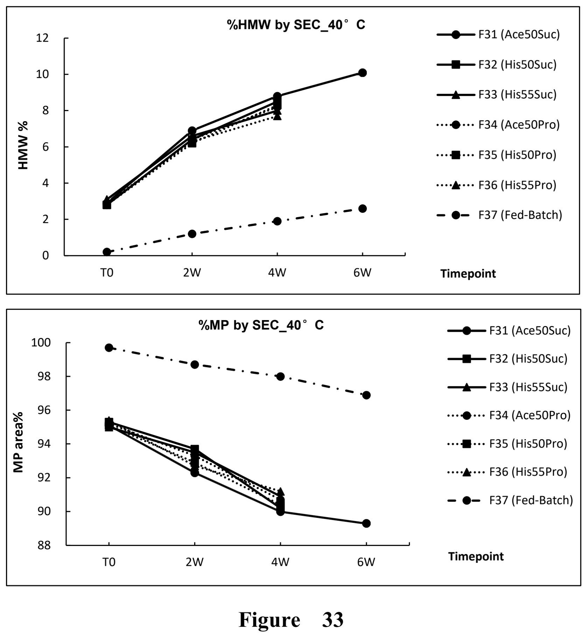

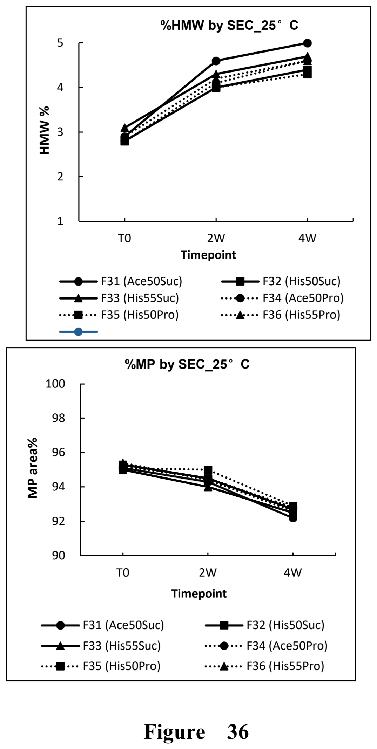

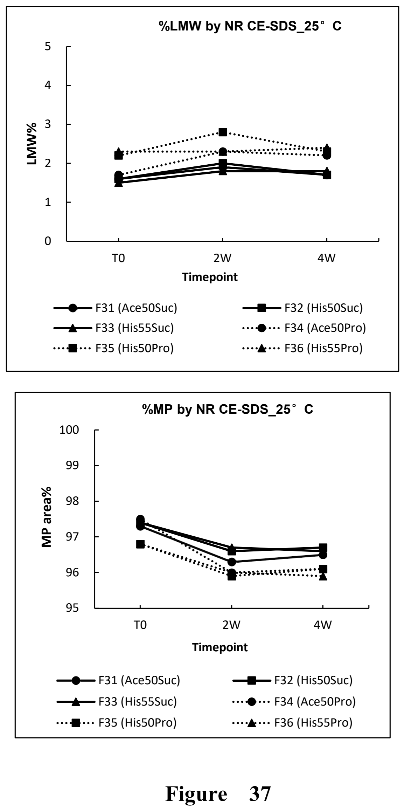

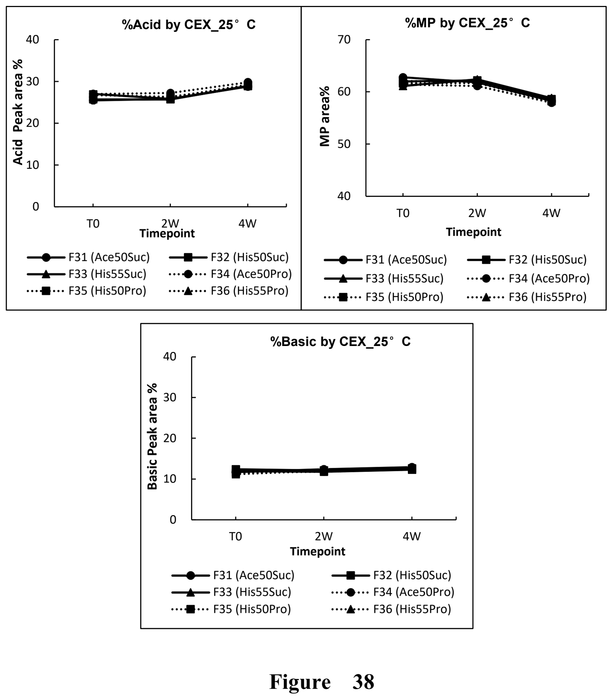

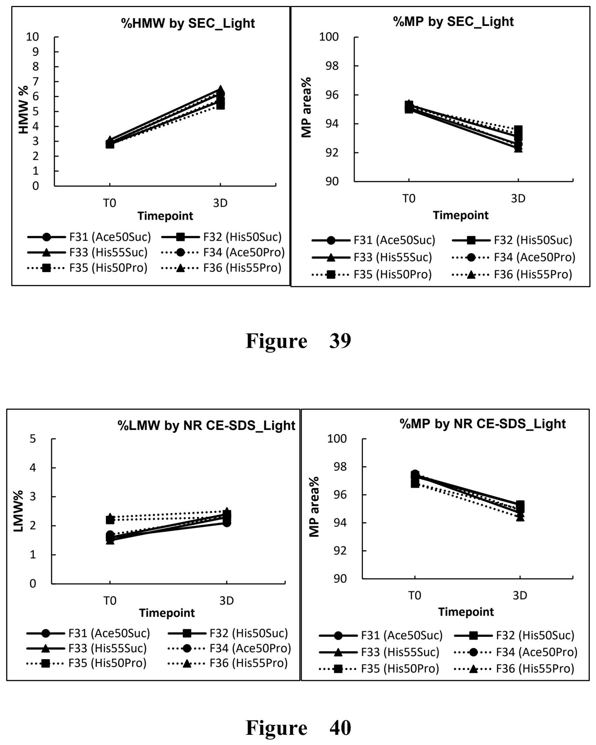

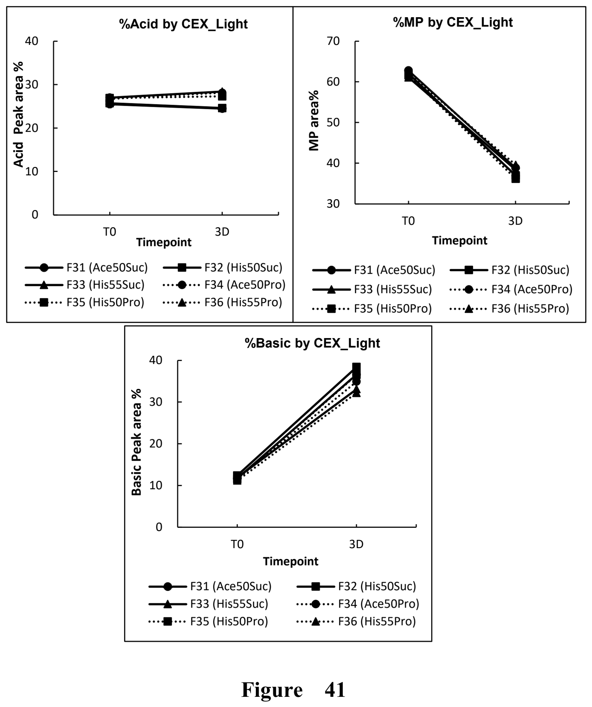

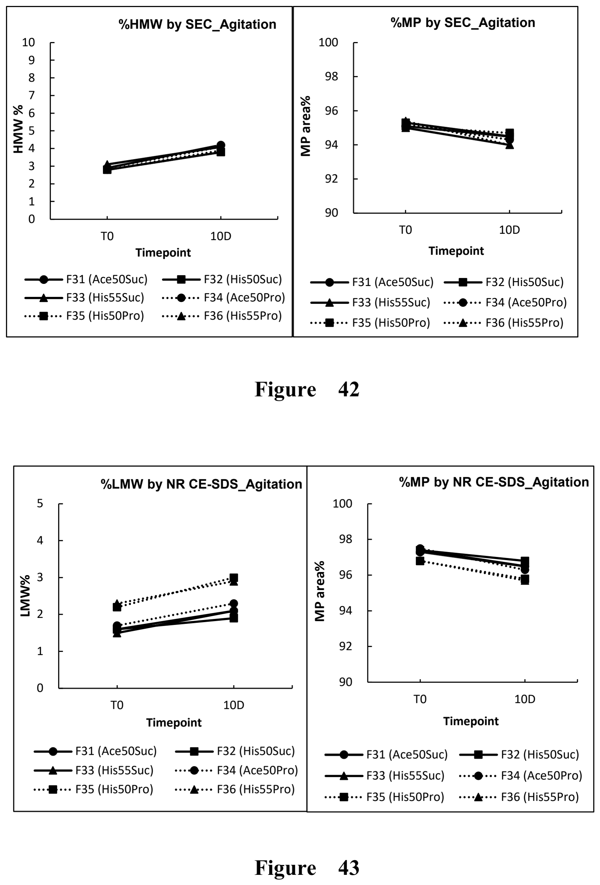

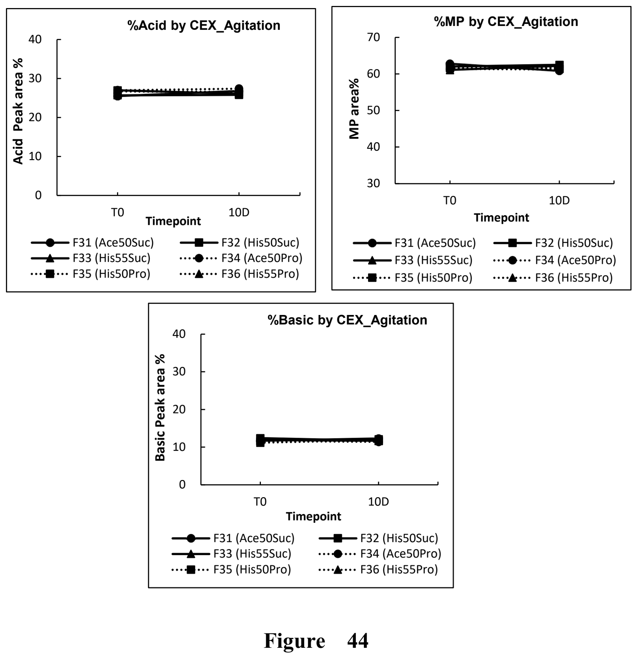

Figure 33 to Figure 44 relate to to the experimental results of F31-F37 formulae which are used for screening the optimal formula(e). Figures 33 to 35 are respectively SEC, NR CE-SDS and CEX results of seven formlaes after being placed at high temperature of 40°C for 4-6 weeks. Figures 36 to 38 are respectively SEC, NR CE-SDS and CEX results of seven formlaes after being placed at 25°C for 4-6 weeks. Figures 39 to 41 are respectively SEC, NR CE-SDS and CEX results of seven formlaes after exposuring to light for 3 days. Figures 42 to 44 are respectively SEC, NR CE-SDS and CEX results of seven formlaes after shaking at 200 rpm for 10 days.

### DETAILED DESCRIPTION

### Definitions

Unless speficied otherwise, all technical and scientific terms used herein have the same meaning as commonly understood by one of ordinary skill in the art to which this invention belongs. As used herein, the term "about", when used in reference to a particular recited numerical value or range of values, means that the value may vary from the recited value by +10%. For example, as used herein, the expression "about 100" includes 90 and 110 and all values in between (for example 90.5, 95, 101, 105, 109.95......, etc). For a ratio, the term "about" is used to define each number of the given ratio. For example, a ratio of about 1:1 means a ratio of (0.9 to 1.1):(0.9 to 1.1).

Although any methods and materials similar or equivalent to those described herein can be used in the practice or testing of the present invention, exemplary methods and materials are now described. All patents, applications, and non-patent publicationss mentioned in the present description are incorporated herein by reference in their entirety

The term "patient" as used herein refers to a mammal and a non-mammalian individual who can benefit from the poorly soluble anti-tumor active agent of the present invention. The mammal refers to any member of the mammal class, includes but is not limited to human; non-human primates, such as chimpanzee and other ape and monkey species; farm animals, such as cattle, horse, sheep, goat and pig; domestic animals, such as rabbit, dog and cat; laboratory animals, including rodents, such as rat, mouse and guinea pig; etc. Examples of non-mammals include but are not limited to birds, etc. The term "patient" does not limit a specific age or gender. In some embodiments, the "patient" is a human.

Unless otherwise specified, "administering" and "administration" are used interchangeably herein.

As used herein, the term "weight percent by volume" or "% w/v" means the percentage weight (in grams) of a single component relative to the total volume of a mixture containing the component. For example, 500 mg of a component in a total volume of 8 ml is 6.25% w/v, and 500 mg of a component in a total volume of 5 ml is 10% w/v.

The term "antibody" as used herein includes any immunoglobulin, monoclonal antibody, polyclonal antibody, multivalent antibody, bivalent antibody, monovalent antibody, multispecific antibody, or bispecific antibody that binds to a specific antigen. A native intact antibody comprises two heavy (H) chains and two light (L) chains. Mammalian heavy chains are classified as alpha, delta, epsilon, gamma, and mu, each heavy chain consists of a variable region (V_{H}) and a first, second, and third constant region (C_{H1}, C_{H2}, C_{H3}, respectively); mammalian light chains are classified as λ or κ, while each light chain consists of a variable region (V_{L} for λ light chain or Vₖ for κ light chain, respectively) and a constant region(C_{L} for λ light chain or C_{K} for κ light chain, respectively). The antibody has a "Y" shape, with the stem of the Y consisting of the second and third constant regions of two heavy chains bound together via disulfide bonding. Each arm of the Y includes the variable region and first constant region of a single heavy chain bound to the variable and constant regions of a single light chain. The variable regions of the light and heavy chains are responsible for antigen binding. The variable regions in both chains generally contain three highly variable loops called the complementarity determining regions (CDRs) (light chain CDRs including LCDR1, LCDR2, and LCDR3, heavy chain CDRs including HCDR1, HCDR2, HCDR3). CDR boundaries for the antibodies and antigen-binding fragments disclosed herein may be defined or identified by the conventions of Kabat, IMGT, Chothia, or Al-Lazikani (Al-Lazikani, B., Chothia, C., Lesk, A. M., J. Mol. Biol., 273(4), 927 (1997); Chothia, C. et al., J Mol Biol. Dec 5;186(3):651-63 (1985); Chothia, C. and Lesk, A.M., J.Mol.Biol., 196,901 (1987); Chothia, C. et al., Nature. Dec 21-28;342(6252):877-83 (1989); Kabat E.A. *et al.*, National Institutes of Health, Bethesda, Md. (1991)). The three CDRs are interposed between flanking stretches known as framework regions (FRs), which are more highly conserved than the CDRs and form a scaffold to support the hypervariable loops. The constant regions of the heavy and light chains are not involved in antigen-binding, but exhibit various effector functions. Antibodies are assigned to classes based on the amino acid sequence of the constant region of their heavy chain. The five major classes or isotypes of antibodies are IgA, IgD, IgE, IgG, and IgM, which are characterized by the presence of α, delta, epsilon, gamma, and µ heavy chains, respectively. Several of the major antibody classes are divided into subclasses such as IgG1 (gamma1 heavy chain), IgG2 (gamma2 heavy chain), IgG3 (gamma3 heavy chain), IgG4 (gamma4 heavy chain), IgA1 (α1 heavy chain), or IgA2 (α2 heavy chain).

The term "bivalent" as used herein refers to an antibody or an antigen-binding fragment having two antigen-binding sites; the term "monovalent" refers to an antibody or an antigen-binding fragment having only one single antigen-binding site; and the term "multivalent" refers to an antibody or an antigen-binding fragment having multiple antigen-binding sites. In some embodiments, the antibody or antigen-binding fragment thereof is bivalent.

As used herein, a "bispecific" antibody refers to an artificial antibody which has fragments derived from two different monoclonal antibodies and is capable of binding to two different epitopes. The two epitopes may present on the same antigen, or they may present on two different antigens.

The term "antigen-binding fragment" as used herein refers to an antibody fragment formed from a portion of an antibody comprising 1, 2, or 3 CDRs, or any other antibody fragment that binds to an antigen but does not comprise an intact native antibody structure. Examples of antigen-binding fragment include, without limitation, a diabody, a Fab, a Fab', a F(ab')₂, an Fv fragment, a disulfide stabilized Fv fragment (dsFv), a (dsFv)₂, a bispecific dsFv (dsFv-dsFv'), a disulfide stabilized diabody (ds diabody), a single-chain antibody molecule (scFv), an scFv dimer (bivalent diabody), a bispecific antibody, a multispecific antibody, a camelized single domain antibody, a nanobody, a domain antibody, and a bivalent domain antibody. An antigen-binding fragment is capable of binding to the same antigen to which the parent antibody binds.

"Fab" with regard to an antibody refers to that portion of the antibody consisting of a single light chain (both variable and constant regions) bound to the variable region and first constant region of a single heavy chain by a disulfide bond.

"Fab'" refers to a Fab fragment that includes a portion of the hinge region.

"F(ab')₂"refers to a dimer of Fab'. "Fv" with regard to an antibody refers to the smallest fragment of the antibody to bear the complete antigen-binding site. An Fv fragment consists of the variable region of a single light chain bound to the variable region of a single heavy chain.

A "dsFv" refers to a disulfide-stabilized Fv fragment that the linkage between the variable region of a single light chain and the variable region of a single heavy chain is a disulfide bond. In some embodiments, a "(dsFv)₂" or "(dsFv-dsFv')" comprises three peptide chains: two V_{H} moieties linked by a peptide linker (e.g., a long flexible linker) and bound to two V_{L} moieties, respectively, via disulfide bridges. In some embodiments, dsFv-dsFv' is bispecific in which each disulfide paired heavy and light chain has a different antigen specificity.

"Single-chain Fv antibody" or "scFv" refers to an engineered antibody consisting of a light chain variable region and a heavy chain variable region connected to one another directly or via a peptide linker sequence (Huston JS et al. Proc Natl Acad Sci USA, 85:5879(1988)).

"Fc" with regard to an antibody refers to that portion of the antibody consisting of the second and third constant regions of a first heavy chain bound to the second and third constant regions of a second heavy chain via disulfide bonding. The Fc portion of the antibody is responsible for various effector functions such as antibody-dependent cell-mediated cytotoxicity (ADCC), and complement dependent cytotoxicity (CDC), but does not function in antigen binding.

"Single-chain Fv-Fc antibody" or "scFv-Fc" refers to an engineered antibody consisting of a scFv connected to the Fc region of an antibody.

"Camelized single domain antibody", "heavy chain antibody" , or "HCAb" refers to an antibody that contains two V_{H} domains and no light chains (Riechmann L. and Muyldermans S., J Immunol Methods. Dec 10;231(1-2):25-38 (1999); Muyldermans S., J Biotechnol. Jun;74(4):277-302 (2001); WO94/04678; WO94/25591; U.S. Patent No. 6,005,079). Heavy chain antibodies were originally derived from *Camelidae* (camels, dromedaries, and llamas). Although devoid of light chains, camelized antibodies have an authentic antigen-binding repertoire (Hamers-Casterman C. et al., Nature. Jun 3;363(6428):446-8 (1993); Nguyen VK. et al. "Heavy-chain antibodies in Camelidae; a case of evolutionary innovation," Immunogenetics. Apr;54(1):39-47 (2002); Nguyen VK. et al.Immunology. May;109(1):93-101 (2003)). The variable domain of a heavy chain antibody (VHH domain) represents the smallest known antigen-binding unit generated by adaptive immune responses (Koch-Nolte F. et al., FASEB J. Nov;21(13):3490-8. Epub 2007 Jun 15 (2007) ).

A "nanobody" refers to an antibody fragment that consists of a VHH domain from a heavy chain antibody and two constant domains, CH2 and CH3.

"Diabodies" or "dAbs" include small antibody fragments with two antigen-binding sites, wherein the fragments comprise a V_{H} domain connected to a V_{L} domain in the same polypeptide chain (V_{H}-V_{L} or V_{L}-V_{H}) (see, e.g., Holliger P. et al., Proc Natl Acad Sci U S A. Jul 15;90(14):6444-8 (1993); EP404097; WO93/11161). By using a linker that is too short to allow pairing between the two domains on the same chain, the domains are forced to pair with the complementary domains of another chain, thereby creating two antigen-binding sites. The antigen-binding sites may target the same or different antigens (or epitopes). In certain embodiments, a "bispecific ds diabody" is a diabody target two different antigens (or epitopes).In certain embodiments, an "scFv dimer" is a bivalent diabody or bivalent ScFv (BsFv) comprising V_{H}-V_{L} (linked by a peptide linker) dimerized with another V_{H}-V_{L} moiety such that V_{H}'s of one moiety coordinate with the V_{L}'s of the other moiety and form two binding sites which can target the same antigens (or epitopes) or different antigens (or epitopes). In other embodiments, an "scFv dimer" is a bispecific diabody comprising V_{H1}-V_{L2} (linked by a peptide linker) associated with V_{L1}-V_{H2} (also linked by a peptide linker) such that V_{H1} and V_{L1} coordinate and V_{H2} and V_{L2} coordinate and each coordinated pair has a different antigen specificity.

A "domain antibody" refers to an antibody fragment containing only the variable region of a heavy chain or the variable region of a light chain. In certain instances, two or more V_{H} domains are covalently joined with a peptide linker to create a bivalent or multivalent domain antibody. The two V_{H} domains of a bivalent domain antibody may target the same or different antigens.

The term "chimeric" as used herein, means an antibody or antigen-binding fragment, having a portion of heavy and/or light chain derived from one species, and the rest of the heavy and/or light chain derived from a different species. In an illustrative example, a chimeric antibody may comprise a constant region derived from human and a variable region from a non-human animal, such as from mouse or rat. In some embodiments, the non-human animal is a mammal, for example, a mouse, a rat, a rabbit, a goat, a sheep, a guinea pig, or a hamster.

The term "humanized" as used herein means that the antibody or antigen-binding fragment comprises CDRs derived from non-human animals, FR regions derived from human, and when applicable, constant regions derived from human.

As used herein, the term "MASP-2" refers to the mannan-binding lectin associated serine protease 2, which is a crucial member of the MBL pathway of the complement system. The human, mouse and cynomolgus MASP-2 amino acid and nucleic acid sequences can be found in a public database, such as GenBank, UniProt and Swiss-Prot. As used herein, the term MASP-2 includes proteins comprising mutations, e.g., point mutations, fragments, insertions, deletions and splice variants of full length wild-type MASP-2. In certain embodiments, the human MASP-2 protein comprises an amino acid sequence of SEQ ID NO: 39. In certain embodiments, the mouse MASP-2 protein comprises an amino acid sequence of SEQ ID NO: 40. In certain embodiments, the cynomolgus MASP-2 protein comprises an amino acid sequence of SEQ ID NO: 43. In certain embodiments, a chimeric MASP-2 protein comprising mouse MASP-2 CUB1-EGF-CUB2 domain (residues 20-297) and human MASP-2 CCP1-CCP2-SP domain (residues 298-686) were synthesized and has an amino acid sequence of SEQ ID NO: 42.

The term "specific binding" or "specifically binds" as used herein refers to a non-random binding reaction between two molecules, such as for example between an antibody and an antigen. In certain embodiments, the antibodies or antigen-binding fragments provided herein specifically bind to human MASP-2 with a binding affinity (K_{D}) of ≤10⁻⁶ M (e.g., ≤5×10⁻⁸ M, ≤2×10⁻⁸ M, ≤10⁻⁸ M, ≤5×10⁻⁹ M, ≤2×10⁻⁹ M, ≤10⁻⁹ M, ≤5×10⁻¹⁰ M, ≤2×10⁻¹⁰ M, ≤10⁻¹⁰ M, ≤5×10⁻¹¹ M, ≤2×10⁻¹¹ M, ≤10⁻¹¹ M, ≤5×10⁻¹² M, ≤4×10⁻¹² M, ≤3×10⁻¹² M,≤2×10⁻¹² M, or ≤10⁻¹² M. K_{D} used herein refers to the ratio of the dissociation rate to the association rate (k_{off}/kₒₙ), which may be determined by using any conventional method known in the art, including but are not limited to surface plasmon resonance method, microscale thermophoresis method, HPLC-MS method, Bio-Layer Interferometry and flow cytometry (such as FACS) method. In certain embodiments, the K_{D} value can be appropriately determined by using ForteBio method.

The ability to "block binding" or "compete for the same epitope" as used herein refers to the ability of an antibody or antigen-binding fragment to inhibit the binding interaction between two molecules (e.g., MASP-2 protein and an anti-MASP-2 antibody) to any detectable degree. In certain embodiments, an antibody or antigen-binding fragment that blocks binding between two molecules inhibits the binding interaction between the two molecules by at least 50%, at least 60%, at least 70%, at least 75%, at least 80%, at least 85%, or at least 90%. In certain embodiments, this inhibition may be greater than 60%, greater than 70%, greater than 75%, greater than 80%, greater than 85%, or greater than 90%.

The term "epitope" as used herein refers to the specific group of atoms or amino acids on an antigen to which an antibody binds. Two antibodies may bind the same or a closely related epitope within an antigen if they exhibit competitive binding for the antigen. For example, if an antibody or antigen-binding fragment blocks binding of a reference antibody to the antigen (e.g., human/monkey MASP-2) by at least 50%, at least 60%, at least 70%, at least 75%, at least 80%, at least 85%, or at least 90%, then the antibody or antigen-binding fragment may be considered to bind the same/closely related epitope as the reference antibody.

Those skilled in the art will recognize that it is possible to determine, without undue experimentation, if a human monoclonal antibody binds to the same epitope as the antibody of present disclosure by ascertaining whether the former prevents the latter from binding to a MASP-2 antigen polypeptide. If the test antibody competes with the antibody of present disclosure, as shown by a decrease in binding by the antibody of present disclosure to the MASP-2 antigen polypeptide, then the two antibodies bind to the same, or a closely related, epitope. Or if the binding of a test antibody to the MASP-2 antigen polypeptide was inhibited by the antibody of present disclosure, then the two antibodies bind to the same, or a closely related, epitope.

The term "OMS721" used herein refers to the monoclonal antibody Narsoplimab (OMS721) developed by Omeros Corporation, the full structure of which is described in U.S. Patent No. US9011860B2, and the OMS721 antibody formualton is disclosed in Chinese Patent Application No. CN201780051640.7, which are incorporated herein by reference in their entirety. OMS721 comprises a constant region of human IgG4. The heavy chain of OMS721 consists of a sequence set forth in SEQ ID NO: 32, and the light chain consists of a sequence set forth in SEQ ID NO: 33.

The heavy chain of OMS721 (hIgG4 kappa) (SEQ ID NO: 32):

QVTLKESGPVLVKPTETLTLTCTVSGFSLSRGKMGVSWIRQPPGKALEWLAHIFSSDEKS
YRTSLKSRLTISKDTSKNQVVLTMTNMDPVDTATYYCARIRRGGIDYWGQGTLVTVSSASTK
GPSVFPLAPCSRSTSESTAALGCLVKDYFPEPVTVSWNSGALTSGVHTFPAVLQSSGLYSLSSV
VTVPSSSLGTKTYTCNVDHKPSNTKVDKRVESKYGPPCPPCPAPEFLGGPSVFLFPPKPKDTL
MISRTPEVTCVVVDVSQEDPEVQFNWYVDGVEVHNAKTKPREEQFNSTYRVVSVLTVLHQD
WLNGKEYKCKVSNKGLPSSIEKTISKAKGQPREPQVYTLPPSQEEMTKNQVSLTCLVKGFYPS
DIAVEWESNGQPENNYKTTPPVLDSDGSFFLYSRLTVDKSRWQEGNVFSCSVMHEALHNHYT
QKSLSLSLGK

The light chain of OMS721 (hIgG4 kappa) (SEQ ID NO: 33):

QPVLTQPPSLSVSPGQTASITCSGEKLGDKYAYWYQQKPGQSPVLVMYQDKQRPSGIPE
RFSGSNSGNTATLTISGTQAMDEADYYCQAWDSSTAVFGGGTKLTVLRTVAAPSVFIFPPSDE
QLKSGTASVVCLLNNFYPREAKVQWKVDNALQSGNSQESVTEQDSKDSTYSLSSTLTLSKAD
YEKHKVYACEVTHQGLSSPVTKSFNRGEC

A "conservative substitution" with reference to amino acid sequence refers to replacing an amino acid residue with a different amino acid residue having a side chain with similar physiochemical properties. For example, conservative substitutions can be made among amino acid residues with hydrophobic side chains (e.g., Met, Ala, Val, Leu, and Ile), among residues with neutral hydrophilic side chains (e.g., Cys, Ser, Thr, Asn and Gln), among residues with acidic side chains (e.g., Asp, Glu), among amino acids with basic side chains (e.g., His, Lys, and Arg), or among residues with aromatic side chains (e.g., Trp, Tyr, and Phe). As known in the art, conservative substitution usually does not cause significant change in the protein conformational structure, and therefore could retain the biological activity of a protein.

The term "homologue" and "homologous" as used herein are interchangeable and refer to nucleic acid sequences (or its complementary strand) or amino acid sequences that have sequence identity of at least 80% (e.g., at least 85%, 88%, 90%, 91%, 92%, 93%, 94%, 95%, 96%, 97%, 98%, 99%) to another sequence when optimally aligned.

"Percent (%) sequence identity" with respect to amino acid sequence (or nucleic acid sequence) is defined as the percentage of amino acid (or nucleic acid) residues in a candidate sequence that are identical to the amino acid (or nucleic acid) residues in a reference sequence, after aligning the sequences and, if necessary, introducing gaps, to achieve the maximum number of identical amino acids (or nucleic acids). Conservative substitution of the amino acid residues may or may not be considered as identical residues. Alignment for purposes of determining percent amino acid (or nucleic acid) sequence identity can be achieved, for example, using publicly available tools such as BLASTN, BLASTp (available on the website of U.S. National Center for Biotechnology Information (NCBI), see also, Altschul S.F. et al, J. Mol. Biol., 215:403-410 (1990); Stephen F. et al, Nucleic Acids Res., 25:3389-3402 (1997)), ClustalW2 (available on the website of European Bioinformatics Institute, see also, Higgins D.G. et al, Methods in Enzymology, 266:383-402 (1996); Larkin M.A. et al, Bioinformatics (Oxford, England), 23(21): 2947-8 (2007)), and ALIGN or Megalign (DNASTAR) software. Those skilled in the art may use the default parameters provided by the tool, or may customize the parameters as appropriate for the alignment, such as for example, by selecting a suitable algorithm.

"Treating" or "treatment" of a condition as used herein includes preventing or alleviating a condition, slowing the onset or rate of development of a condition, reducing the risk of developing a condition, preventing or delaying the development of symptoms associated with a condition, reducing or ending symptoms associated with a condition, generating a complete or partial regression of a condition, curing a condition, or some combination thereof.

An "isolated" substance has been altered by the hand of man from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide is "isolated" if it has been sufficiently separated from the coexisting materials of its natural state so as to exist in a substantially pure state. An isolated "nucleic acid" or "polynucleotide" are used interchangeably and refer to the sequence of an isolated nucleic acid molecule. In certain embodiments, an "isolated antibody or antigen-binding fragment thereof" refers to the antibody or antigen-binding fragments having a purity of at least 60%, 70%, 75%, 80%, 81%, 82%, 83%, 84%, 85%, 86%, 87%, 88%, 89%, 90%, 91%, 92%, 93%, 94%, 95%, 96%, 97%, 98%, 99% as determined by electrophoretic methods (such as SDS-PAGE, isoelectric focusing, capillary electrophoresis), or chromatographic methods (such as ion exchange chromatography or reverse phase HPLC).

The term "vector" as used herein refers to a vehicle into which a polynucleotide encoding a protein may be operably inserted so as to bring about the expression of that protein. A vector may be used to transform, transduce, or transfect a host cell so as to bring about expression of the genetic element it carries within the host cell. Examples of vectors include plasmids, phagemids, cosmids, artificial chromosomes such as yeast artificial chromosome (YAC), bacterial artificial chromosome (BAC), or P1-derived artificial chromosome (PAC), bacteriophages such as lambda phage or M13 phage, and animal viruses. Categories of animal viruses used as vectors include retrovirus (including lentivirus), adenovirus, adeno-associated virus, herpesvirus (e.g., herpes simplex virus), poxvirus, baculovirus, papillomavirus, and papovavirus (e.g., SV40). A vector may contain a variety of elements for controlling expression, including promoter sequences, transcription initiation sequences, enhancer sequences, selectable elements, and reporter genes. In addition, the vector may contain an origin of replication. A vector may also include materials to aid in its entry into the cell, including but not limited to a viral particle, a liposome, or a protein coating. A vector can be an expression vector or a cloning vector. The present disclosure provides vectors (e.g., expression vectors) containing the nucleic acid sequence provided herein encoding the antibody or antigen-binding fragment thereof, at least one promoter (e.g., SV40, CMV, EF-1α) operably linked to the nucleic acid sequence, and at least one selection marker. Examples of vectors include, but are not limited to, retrovirus (including lentivirus), adenovirus, adeno-associated virus, herpesvirus (e.g., herpes simplex virus), poxvirus, baculovirus, papillomavirus, papovavirus (e.g., SV40), lambda phage, and M13 phage, plasmid pcDNA3.3, pMD18-T, pOptivec, pCMV, pEGFP, pIRES, pQD-Hyg-GSeu, pALTER, pBAD, pcDNA, pCal, pL, pET, pGEMEX, pGEX, pCI, pEGFT, pSV2, pFUSE, pVITRO, pVIVO, pMAL, pMONO, pSELECT, pUNO, pDUO, Psg5L, pBABE, pWPXL, pBI, p15TV-L, pPro18, pTD, pRS10, pLexA, pACT2.2, pCMV-SCRIPT.RTM., pCDM8, pCDNA1.1/amp, pcDNA3.1, pRc/RSV, PCR 2.1, pEF-1, pFB, pSG5, pXT1, pCDEF3, pSVSPORT, pEF-Bos etc.

The phrase "host cell" as used herein refers to a cell into which an exogenous polynucleotide and/or a vector has been introduced.

The complement system has been implicated in the pathogenesis of numerous acute and chronic diseases or conditions, including: myocardial infarction, stroke, acute respiratory distress syndrome (ARDS), reperfusion injury, septic shock, capillary leakage following thermal burns, post cardiopulmonary bypass inflammation, transplant rejection, rheumatoid arthritis, multiple sclerosis, myasthenia gravis, and Alzheimer's disease. In almost all of these diseases or conditions, complement is not the cause but is one of several factors involved in pathogenesis. Complement activation may be a major pathological mechanism and represents an effective point for clinical control in many of these disease states. Participating the MBL pathway, one of the three major complement activation pathways, MASP-2 may also be involved in the pathogenesis of many diseases or conditions. In some embodiments, the disease or condition that is related to MASP-2 dependent complement activation is an autoimmune disease, a vascular condition, an ischemia-reperfosion injury, atherosclerosis, an inflammation, a pulmonary condition, exfracorporeal reperfosion procedure, a musculoskeletal condition, a renal condition, a skin condition, an organ or tissue transplant procedure, a nervous system disorder or injury, a blood disorder, urogenital condition, a nonobese diabetes or a complication associated with Type 1 or Type 2 diabetes, cancer, endocrine disorder, or an ophthalmologic condition.

The term "mannan-binding lectin" ("MBL") is equivalent to mannan-binding protein ("MBP"). The term "membrane attack complex" ("MAC", also referred to as C5b-9) refers to a complex of the terminal five complement components (C5-C9) that inserts into and disrupts cell membranes.

An "autoimmune disease" as used herein refers to a pathophysiological state wherein immune responses are directed against, and damage, the body's own tissues (autoimmunity). Examples of autoimmune disease include but are not limited to thrombotic microangiopathies (TMAs), atypical hemolytic uremic syndrome (aHUS), hematopoietic transplant-associated thrombotic microangiopathy (TA-TMA), lupus nephritis, systemic lupus erythematosus (SLE) and IgA nephropathy.

The term "tumor", "cancer" or "carcinoma" as used herein refers to a neoplasm formed by the proliferation (e.g., uncontrollable) of local tissue cells under the influence of various tumorigenic factors.

The term "pharmaceutically acceptable" indicates that the designated carrier, vehicle, diluent, excipient(s), and/or salt is generally chemically and/or physically compatible with the other ingredients comprising the formulation, and physiologically compatible with the recipient thereof.

### Pharmaceutical formulation

As used herein, the term "pharmaceutical formulation" refers to a combination of at least one active ingredient (e.g., a monoclonal antibody or an antigen-binding fragment thereof that specifically binds to human MASP-2) and at least one inactive ingredient. When combined with the active ingredient and/or one or more additional inactive ingredients, the inactive ingredient is suitable for therapeutic administration to a human or non-human animal. Unless specifically indicated otherwise, the term "formulation", as used herein, refers to "pharmaceutical formulation". The present invention provides a pharmaceutical formulation comprising at least one therapeutic antibody. According to certain embodiments of the present invention, the therapeutic antibody is a monoclonal antibody or an antigen-binding fragment thereof that specifically binds to human MASP-2. More specifically, the present invention includes a pharmaceutical formulation comprising: (i) a monoclonal antibody or an antigen-binding fragment thereof that specifically binds to human MASP-2; (ii) a buffer comprising a histidine and/or acetate buffer system; (iii) a surfactant comprising polysorbates; and (iv) an auxiliary material comprising sucrose, trehalose and/or proline. If additional components do not significantly interfere with the stability of the formulation, such components may be included in the formulation of the present invention. Specific exemplary components and formulations included within the present invention are described in detail below.

In certain embodiments, the pharmaceutical formulation of the present invention is a liquid formulation. As used herein, the expression "liquid formulation" refers to a mixture of at least two components that primarily exists in a liquid state at about 2° C to about 40° C. The liquid formulation may have a low viscosity, a medium viscosity, or a high viscosity, depending on the specific ingredients therein.

As used herein, the term "buffer" refers to a mixture of a weak acid and its conjugate base or a weak base and its conjugate acid. For example, "acetate buffer" as used herein refers to a mixture comprising sodium acetate and its conjugate acid, namely acetate. A chemical equilibrium is established between a weak acid and its conjugate base, thus a solution containing a buffer resists sudden changes in pH when a small amount of acid or base is added to the solution.

As used herein, the term "osmolatity" refers to a measure of the osmotic pressure of dissolved solute particles in an aqueous solution. The solute particles include two ions as well as non-ionized molecules. The osmotic pressure is expressed as the concentration (i.e.,) of osmotically active particles dissolved in 1 kg of solvent (i.e., water). Herein, the osmolatity is expressed in milliosmoles/kilogram of water (mOsm/kg).

The term "auxiliary material" refers to any non-therapeutic agent added to the formulation to provide a desired consistency, viscosity and/or stabilization effect. The preferred auxiliary material of the present invention includes, but is not limited to, trehalose, sucrose, and proline.

"Diameter" and "Mean Diameter" refer to the intensity-based volume diameter of the corresponding microparticle formulation, which can be measured using a dynamic light scattering instrument such as a laser diameter analyzer.

### Antibodies of the present invention

The amount of antibody, or antigen-binding fragment thereof, contained within the pharmaceutical formulations of the present invention may vary depending on the desired specific properties of the formulations, as well as the particular circumstances and purposes for which the formulations are intended to be used. In certain embodiments,, the pharmaceutical formulation may contain about 0.1 mg/mL to about 500 mg/mL of an antibody; about 0.5 mg/mL to about 400 mg/mL of an antibody; about 1 mg/mL to about 200 mg/mL of an antibody; about 2 mg/mL to about 100 mg/mL; about 1 mg/mL to about 5 mg/mL of an antibody; about 10 mg/mL to about 30 mg/mL of an antibody; about 75 mg/mL to about 125 mg/mL; about 5 mg/mL to about 50 mg/mL; or about 2 mg/mL to about 150 mg/mL of an antibody. For example, the pharmaceutical formulation is a liquid formulation that may contain about 0.5 mg/mL; about 1 mg/mL; about 2 mg/mL; about 3 mg/mL; about 4 mg/mL; about 5 mg/mL; about 6 mg/mL; about 7 mg/mL; about 8 mg/mL; about 9 mg/mL; about 10 mg/mL; about 11 mg/mL; about 12 mg/mL; about 13 mg/mL; about 14 mg/mL; about 15 mg/mL; about 16 mg/mL; about 17 mg/mL; about 18 mg/mL; about 19 mg/mL; about 20 mg/mL; about 21 mg/mL; about 22 mg/mL; about 23 mg/mL; about 24 mg/mL; about 25 mg/mL; about 26 mg/mL; about 27 mg/mL; about 28 mg/mL; about 29 mg/mL; about 30 mg/mL; about 35 mg/mL; about 40 mg/mL; about 45 mg/mL; about 50 mg/mL; about 55 mg/mL; about 60 mg/mL; about 65 mg/mL; about 70 mg/mL; about 75 mg/mL; about 80 mg/mL; about 85 mg/mL; about 90 mg/mL; about 95 mg/mL; about 96 mg/mL; about 97 mg/mL; about 98 mg/mL; about 99 mg/mL; about 100 mg/mL; about 101 mg/mL; about 102 mg/mL; about 103 mg/mL; about 104 mg/mL; about 105 mg/mL; about 110 mg/mL; about 115 mg/mL; about 120 mg/mL; about 125 mg/mL; about 130 mg/mL; about 135 mg/mL; about 140 mg/mL; about 145 mg/mL; about 150 mg/mL; about 155 mg/mL; about 160 mg/mL; about 165 mg/mL; about 170 mg/mL; about 175 mg/mL; about 180 mg/mL; about 185 mg/mL; about 190 mg/mL; about 195 mg/mL; or about 200 mg/mL of a monoclonal antibody or an antigen-binding fragment thereof that specifically binds to human MASP-2. In certain embodiments, the pharmaceutical formulation is a liquid formulation that may contain 1 ± 0.1 mg/mL to 200 ± 20 mg/mL of an antibody; 2 ± 0.2 mg/mL to 100 ± 10 mg/mL of an antibody; 1 ± 0.5 mg/mL to 30 ± 5 mg/mL of an antibody; 10 ± 1 mg/mL to 30 ± 3 mg/mL of an antibody; 1 ± 0.1 mg/mL to 3 ± 0.3 mg/mL of an antibody; 15 ± 1.5 mg/mL to 25 ± 2.5 mg/mL of an antibody; 90 ± 5 mg/mL to 110 ± 5 mg/mL of an antibody; or 150 ± 7.5 mg/mL to 170 ± 7.5 mg/mL of an antibody. In some embodiments, the pharmaceutical formulation contains 20 ± 2 mg/mL of an antibody. In some embodiments, the pharmaceutical formulation contains 60 ± 6 mg/mL of an antibody. In some embodiments, the pharmaceutical formulation contains 100 ± 10 mg/mL of an antibody. In some embodiments, the pharmaceutical formulation contains 150 ± 15 mg/mL of an antibody.

### Bioequivalent

The present invention encompasses antibodies or antigen-binding fragments thereof having amino acid sequences that differ from those of the exemplary molecules disclosed herein but that retain the ability to bind human MASP-2 specifically. Such variants may contain one or more additions, deletions or replacements of amino acids when compared to the parent sequence, but still exhibit biological activity that is substantially equivalent to that of the antibodies described herein.

The present invention includes antigen-binding molecules that are bioequivalent to any of the exemplary antibodies set forth herein. For example, if two antibodies are pharmaceutical equivalents or pharmaceutical alternatives whose rate and extent of absorption do not show a significant difference when administered at the same dose (either single does or multiple dose) under similar experimental conditions, they are considered bioequivalent. Some antibodies will be considered equivalents or pharmaceutical alternatives if they are equivalent in the extent of their absorption but not in their rate of absorption and yet may be considered bioequivalent, since such differences in the rate of absorption are intentional and are reflected in the labeling, are not essential to the attainment of effective body drug concentrations on, e.g., chronic use, and are considered medically insignificant for the particular drug product studied.

In one embodiment, two antibodies are bioequivalent if there are no clinically meaningful differences in their safety, purity, and efficacy.

In one embodiment, two antibodies are bioequivalent if a patient can be switched one or more times between the reference product and the biological product without an expected increase in the risk of adverse effects, including a clinically significant change in immunogenicity, or diminished effectiveness, as compared to continued therapy without such switching.

Bioequivalence may be demonstrated by in vivo and in vitro methods. Bioequivalence measurements include, e.g., (a) an in vivo test in humans or other mammals, in which the concentration of the antibody or the metabolites thereof is measured in blood, plasma, serum, or other biological fluid as a function of time; (b) an in vitro test that has been correlated with and is reasonably predictive of human in vivo bioavailability data; (c) an in vivo test in humans or other mammals in which the appropriate acute pharmacological effect of the antibody (or its target) is measured as a function of time; and (d) a well-controlled clinical trial that establishes safety, efficacy, or bioavailability or bioequivalence of an antigen-binding protein.

### Auxiliary materials and pH of the formulation

The pharmaceutical formulation of the present invention comprises one or more auxiliary materials. As used herein, the term "auxiliary material" means any non-therapeutic agent added to a formulation to provide the desired consistency, viscosity and/or stabilizing effect.

In certain embodiments, the pharmaceutical formulation of the present invention has a viscosity of less than 30cP, which is conducive to delivering the composition from a pre-filled syringe or an automatic syringe. In some embodiments, the stable liquid formulation of the present invention has a viscosity of about 1.0 cP-30cP. In some instances, when measured using a dynamic light scattering instrument (DLS), the pharmaceutical formulation at 25°C has a viscosity of less than 28 cP, less than 25 cP, less than 23 cP, less than 20 cP, less than 18 cP, less than 15 cP, less than 13 cP, less than 10 cP, less than 7 cP or less than 5 cP, for example about 1.0 cP-10cP. In one embodiment, the liquid formulation at 25°C has a viscosity of about 1.0 cP- 20cP. In some embodiments, when measured using DLS, the pharmaceutical formulation with an antibody concentration of up to 150mg/ml at 25°C has a viscosity of less than 15 cP. In one embodiment, the liquid formulation at 25°C has a viscosity of about 1.0 cP- 10cP. In one embodiment, the liquid formulation at 25°C has a viscosity of about 1.0, 2.0, 3.0, 4.0, 5.0, 6.0, 7.0, 8.0, 9.0 or 10.0cP. In some embodiments, when measured using DLS, the pharmaceutical formulation with an antibody concentration of up to 150mg/ml at 25°C has a viscosity of less than 7 cP. In one embodiment, the liquid formulation at 4-40°C has a viscosity of about 5.0 cP-7.0cP. In one embodiment, the liquid formulation at 4-40°C has a viscosity of about 6.8cP. In one embodiment, the liquid formulation at 25°C has a viscosity of about 6.8cP.

The amount of the auxiliary material contained in the pharmaceutical formulation of the present invention varies depending on the specific circumstances and intended purpose of the formulation. In certain embodiments, the formulation may contain about 0.1% to about 20% of an auxiliary material; about 0.5% to about 20% of an auxiliary material; about 1% to about 20% of an auxiliary material; about 2% to about 15% of an auxiliary material; about 5% to about 15% of an auxiliary material; about 7.5% to about 12.5% of an auxiliary material; or about 9% to about 11% of an auxiliary material. For example, the pharmaceutical formulationof the present invention may comprise about 0.5%, about 1.0%, about 1.5%, about 2.0%, about 2.5%, about 3.0%, about 3.5%, about 4.0%, about 4.5%, about 5.0%, about 5.5%, about 6.0%, about 6.5%, about 7.0%, about 7.5%, about 8.0%, about 8.5%, about 9.0%, about 9.5%, about 10.0%, about 10.5%, about 11.0%, about 11.5%, about 12.0%, about 12.5%, about 13.0%, about 13.5%, about 14.0%, about 14.5%, about 15% or about 20% of an auxiliary material (e.g., sucrose) . In some embodiments, the formulation contains about 5.8% ± 0.5% of an auxiliary material (e.g., sucrose). In some embodiments, the formulation contains about 6.0% ± 0.5% of an auxiliary material (e.g., sucrose). In some embodiments, the formulation contains about 6.5% ± 0.5% of an auxiliary material (e.g., sucrose). In some embodiments, the formulation contains about 7.0% ± 0.5% of an auxiliary material (e.g., sucrose). In some embodiments, the formulation contains about 8.6% ± 0.5% of an auxiliary material (e.g., sucrose). Each of the above percentages corresponds to a weight/volume percentage (w/v). In some instances, the formulation contains 5.5% ± 0.5% to 9% ± 0.5% (w/v) of sucrose. In some instances, the formulation contains 5.5% ± 0.5% to 9% ± 0.5% (w/v) of trehalose. In some instances, the formulation contains 200mM to 300mM of proline.

### Surfactants in the formulation

The pharmaceutical formulation of the present invention may also comprise one or more surfactants in a type and in an amount that stabilizes the monoclonal antibody or the antigen-binding fragment thereof that specifically binds to human MASP-2 under conditions of rough handling or agitation, such as, e.g., orbital shaking. As used herein, the term "surfactant" refers to a substance which reduces the surface tension of a fluid in which it is dissolved and/or reduces the interfacial tension between oil and water. The surfactant may be ionic or nonionic. Exemplary non-ionic surfactants that may be included in the formulations of the present invention include, e.g., alkyl poly(ethylene oxide), alkyl polyglucosides (e.g., octyl glucoside and decyl maltoside), fatty alcohols such as cetyl alcohol and oleyl alcohol, cocamide MEA, cocamide DEA, and cocamide TEA. Specific non-ionic surfactants that may be included in the formulations of the present invention include, for example, polysorbates such as polysorbate 20, polysorbate 28, polysorbate 40, polysorbate 60, polysorbate 65, polysorbate 80, polysorbate 81, and polysorbate 85; poloxamers such as poloxamer 188 (also known as Pluronic F68), poloxamer 407; polyethylene-polypropylene glycol; or polyethylene glycol (PEG). Polysorbate 20 is also known as TWEEN 20, sorbitan monolaurate and polyoxyethylenesorbitan monolaurate. In some embodiments, the surfactant is polysorbate 80 or polysorbate 20, preferably polysorbate 80.

The amount of the surfactant contained within the pharmaceutical formulations of the present invention may vary depending on the specific properties desired of the formulations, as well as the particular circumstances and purposes for which the formulations are intended to be used. In certain embodiments, the formulation may contain about 0.01% to about 1% of a surfactant; about 0.01% to about 0.5% of a surfactant; about 0.05% to about 0.15%; about 0.08% to about 0.12%; or about 0.09% to about 0.11% of a surfactant. For example, the formulation of the present invention may comprise about 0.01%; about 0.02%; about 0.03%; about 0.04%; about 0.05%; about 0.06%; about 0.07%; about 0.08%; about 0.09%; about 0.10%; about 0.11%; about 0.12%; about 0.13%; about 0.14%; about 0.15%; about 0.16%; about 0.17%; about 0.18%; about 0.19%; about 0.20%; about 0.21%; about 0.22%; about 0.23%; about 0.24%; about 0.25%; about 0.26%; about 0.27%; about 0.28%; about 0.29%; or about 0.30% of a surfactant (e.g., polysorbate 80) . In some embodiments, the formulation contains about 0.1% of a surfactant (e.g., polysorbate80). Each of the percentages noted above corresponds to a percent weight/volume (w/v). In some instances, the formulation contains 0.025% ± 0.01% to 0.1% ± 0.01% w/v of polysorbate 80. In some instances, the formulation contains 0.05% ± 0.01% w/v of polysorbate 80.

### Buffer systems and pH of the formulation

The pharmaceutical formulations of the present invention may also comprise a buffer or a buffer system, which serves to maintain a stable pH and to help stabilize the monoclonal antibody or the antigen-binding fragment thereof that specifically binds to human MASP-2. In some embodiments, the buffer or the buffer system comprises at least one buffer that has a buffering range that overlaps fully or in part the range of pH 4.7 to 6.1.

In certain embodiments, the buffer comprises a histidine buffer and/or acetate buffer. In certain embodiments, the buffer (e.g., histidine) is present at a concentration of about 1 mM to about 40 mM, about 1 mM to about 30 mM, about 1 mM to about 20 mM; about 3 mM to about 18 mM, about 5 mM to about 15 mM; or about 8 mM to about 12 mM. In some embodiments, the buffer (e.g., histidine) is present at a concentration of about 1mM; about 2 mM; about 3 mM; about 4 mM; about 5 mM; about 6 mM; about 7 mM; about 8 mM; about 9 mM; about 10 mM; about 11 mM; about 12 mM; about 13 mM; about 14 mM; about 15 mM; about 16 mM; about 17 mM; about 18 mM; about 19 mM; or about 20 mM. In some instances, the formulation contains a histidine buffer at a concentration of 5 mM ± 1 mM to 20 mM ± 4 mM. In some instances, the formulation contains a histidine buffer at a concentration of 10 mM ± 2 mM. In some embodiments, the histidine buffer comprises L- histidine and L-histidine hydrochloride monohydrate. In some instances, the histidine buffer comprises L-histidine at a concentration of 0.175 mg/mL and L-histidine hydrochloride monohydrate at a concentration of 1.86 mg/ml.

In some embodiments, the formulation contains an acetate buffer. In some embodiments, the buffer (e.g., acetate) is present at a concentration of about 1 mM to about 40 mM, about 1 mM to about 30 mM, about 1 mM to about 20 mM; about 3 mM to about 18 mM, about 5 mM to about 15 mM; or about 8 mM to about 12 mM. In some embodiments, the buffer (e.g., acetate) is present at a concentration of about 1mM; about 2 mM; about 3 mM; about 4 mM; about 5 mM; about 6 mM; about 7 mM; about 8 mM; about 9 mM; about 10 mM; about 11 mM; about 12 mM; about 13 mM; about 14 mM; about 15 mM; about 16 mM; about 17 mM; about 18 mM; about 19 mM; or about 20 mM. In some instances, the formulation contains an acetate buffer at a concentration of 5 mM ± 1 mM to 20 mM ± 4 mM. In some instances, the formulation contains an acetate buffer at a concentration of 10 mM ± 2 mM. In some embodiments, the acetate buffer comprises acetic acid and sodium acetate trihydrate. In some instances, the histidine buffer comprises histidine salts at a concentration of 10 mM.

During the antibody purification process it may be desired or necessary to exchange one buffer for another to achieve appropriate excipient concentrations, antibody concentration, pH, etc. Buffer exchange can be accomplished, e.g., by ultrafiltration/diafiltration (UF/DF) using, e.g., a semipermeable tangential flow filtration membrane. However, use of such techniques has the potential to cause the Gibbs-Donnan effect [Bolton et al., 2011, Biotechnol. Prog. 27(1):140-152]. The accumulation of positive charge on the product side of the membrane during protein concentration is counterbalanced electrically by the preferential movement of positive ions to the opposite side of the membrane. The potential consequence of this phenomenon is that the final concentrations of certain components (e.g., histidine) may be lower than the intended target concentrations of these components due to the electrostatic repulsion of positively charged diafiltration buffer excipients to the positively charged antibody protein during the UF/DF step. Thus, the present invention includes formulations in which the concentration of, for example, histidine/acetate vary from the recited amounts or ranges herein due to the Gibbs-Donnan effect.

Volume exclusion describes the behavior of highly concentrated samples in which a significant portion of the total volume of the solution is taken up by the solute, especially large molecules such as proteins, excluding the solvent from this space. This then decreases the total volume of solvent available for other solutes to be dissolved in, which may result in unequal partition across the ultrafiltration membrane. Thus, the present invention includes formulations in which the concentration of, for example, histidine may vary from the recited amounts or ranges herein due to the volume exclusion effect.

During the manufacture of the formulations of the present invention, variations in the composition of the formulation may occur. These variations may include the concentration of the active ingredient, the concentration of the excipients, and/or the pH of the formulation. The present invention includes a formulation comprising a monoclonal antibody or an antigen-binding fragment thereof that specifically binds to human MASP-2 which is stable and retain potency with up to at least 10% variation in the excipient concentration. For example, a formulation comprising a monoclonal antibody or an antigen-binding fragment thereof that specifically binds to human MASP-2 is included herein, wherein stability and potency of said formulation is unaffected by ±10%, ±20%, variation in the concentration of an antibody, an auxiliary material, a buffer and/or a surfactant.

### Stability of the pharmaceutical formulation

The pharmaceutical formulations of the present invention exhibit high levels of stability. The term "stable", as used herein in reference to the pharmaceutical formulations, refers to that the antibody within the pharmaceutical formulation retains an acceptable degree of chemical structure or biological function after storage under defined conditions. The formulation may be stable even though the antibody contained therein does not maintain 100% of its chemical structure or biological function after storage for a defined amount of time. Under certain circumstances, maintenance of about 90%, about 95%, about 96%, about 97%, about 98% or about 99% of the structure and/or function and/or biological activity of an antibody after storage for a defined amount of time may be regarded as "stable".

In some embodiments, the stable liquid the formulation of the present invention has a mean diameter of 2-10 µm, as measured by Microflow- imaging system (MFI).

In some embodiments, the stable liquid formulation of the present invention has an osmolality of 250 to 350 mOsm/kg H₂O.

In some embodiments, the stability can be measured by, in particular, determining the protein content (concentration) of the antibody in the formulation after storage for a defined amount of time at a given temperature. An "acceptable stability," as that phrase is used herein, refers to that no more than 10% of change in the protein content of the antibody in the formulation is detected after storage for a defined amount of time at a given temperature. In certain embodiments, no more than 10%, 9%, 8%, 7%, 6%, 5%, 4%, 3%, 2%, 1% or less of the change in the protein content of the antibody in the formulation is detected after storage for a defined amount of time at a given temperature. The defined amount of time after which stability is measured may be at least 3 days, at least 5 days, at least 1 weeks, at least 10 days, at least 2 weeks, at least 3 weeks, at least 4 weeks, at least 5 weeks, at least 6 weeks, at least 3 months, at least 4 months, at least 5 months, at least 6 months, at least 7 months, at least 8 months, at least 9 months, at least 10 months, at least 11 months, at least 12 months, at least 24 months, at least 36 months or longer. The temperature at which the pharmaceutical formulation may be stored when assessing stability may be any temperature of about -80°C to about 45°C, for example stored at about - 80°C, about -30°C, about -20°C, about 0°C, about 4°C-8°C, about 5°C, about 25°C, about 35°C, about 37°C, about 40°C or about 45°C. For example, a pharmaceutical formulation may be regarded as stable, if no more than 10%, 9%, 8%, 7%, 6%, 5%, 4%, 3%, 2%, 1% or less of the change in the protein content of the antibody in the formulation is detected after 3 months of storage at 4° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 6 months of storage at 4° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 9 months of storage at 4° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 12 months of storage at 4° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 24 months of storage at 4° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 36 months of storage at 4° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 2 weeks of storage at 25° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 1 months of storage at 25° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 4 weeks of storage at 25° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 6 weeks of storage at 25° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 2 weeks of storage at 40° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 4 weeks of storage at 40° C. A pharmaceutical formulation may also be regarded as stable, if no more than 10%, 9%, 8%, 7%, 6%, 5%, 4%, 3%, 2%, 1% or less of the change in the protein content of the antibody in the formulation is detected after 6 weeks of storage at 40° C.

In some embodiments, the stability can be measured by, in particular, determining the percentage of the monomer purity of the antibody in the formulation after storage for a defined amount of time at a given temperature. The monomer purity of the antibody can be determined by, in particular, size exclusion chromatography (e.g., size exclusion high performance liquid chromatography [SE-HPLC]). An "acceptable stability," as that phrase is used herein, refers to that at least 90% of the monomer purity of the antibody is detected in the formulation after storage for a defined amount of time at a given temperature. In certain embodiments, at least about 90%, 91%, 92%, 93%, 94%, 95%, 96%, 97%, 98%, 99% or 100% of the monomer purity of the antibody is detected in the formulation after storage for a defined amount of time at a given temperature. The defined amount of time after which stability is measured can be at least 3 days, at least 5 days, at least 1 weeks, at least 10 days, at least 2 weeks, at least 3 weeks, at least 4 weeks, at least 5 weeks, at least 6 weeks, at least 3 months, at least 4 months, at least 5 months, at least 6 months, at least 7 months, at least 8 months, at least 9 months, at least 10 months, at least 11 months, at least 12 months, at least 24 months, at least 36 months or longer. The temperature at which the pharmaceutical formulation may be stored when assessing stability can be any temperature of about -80°C to about 45°C, for example, stored at about -80°C, about -30°C, about - 20°C, about 0°C, about 4°C-8°C, about 5°C, about 25°C, about 35°C, about 37°C, about 40°C or about 45°C. For example, a pharmaceutical formulation may be regarded as stable, if greater than about 90%, 95%, 96% or 97% of the monomer purity of the antibody is detected by SEC-HPLC after 3 months of storage at 4° C. A pharmaceutical formulation may also be regarded as stable, if greater than about 90%, 95%, 96% or 97% of the monomer purity of the antibody is detected by SEC-HPLC after 6 months of storage at 4° C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC after 9 months of storage at 4° C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC after 12 months of storage at 4° C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC after 24 months of storage at 4° C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC after 36 months of storage at 4° C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC, after 2 weeks of storage at 25°C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC after 1 months of storage at 25° C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC after 4 weeks of storage at 25° C. A pharmaceutical formulation may also be regarded as stable, if greater than about 90%, 95%, 96%, 96.5%, 97%, 97.5%, 98%, 98.5%, 99% or 99.5% of the monomer purity of the antibody is detected by SEC-HPLC after 6 weeks of storage at 25° C. A pharmaceutical formulation may also be regarded as stable, if greater than about 90%, 91%, 92%, 93%, 94%, 95%, 96%, 97% or 98% of the monomer purity of the antibody is detected by SEC-HPLC after 2 weeks of storage at 40° C. A pharmaceutical formulation may also be regarded as stable, if greater than about 90%, 91%, 92%, 93%, 94%, 95%, 96%, 97% or 98% of the monomer purity of the antibody is detected by SEC-HPLC after 4 weeks of storage at 40° C. A pharmaceutical formulation may also be regarded as stable, if greater than about 90%, 91%, 92%, 93%, 94%, 95%, 96%, 97% or 98% of the monomer purity of the antibody is detected by SEC-HPLC after 6 weeks of storage at 40° C.

In other embodiments, other methods may be used to assess the stability of the formulations of the present invention such as, differential scanning calorimetry (DSC) to determine thermal stability, controlled agitation to determine mechanical stability, and absorbance at about 350 nm or about 405 nm to determine the turbidity of the solution. For example, a formulation of the present invention may be considered as stable, if, the change in OD405 of the formulation is less than about 0.05 (e.g., 0.04, 0.03, 0.02, 0.01, or less) from the OD405 of the formulation at t=0, after 6 weeks or longer of storage at about 4° C to about 25° C.

In some embodiments, the stability of the liquid formulation after storage can be inidcated by measuring the appearance and visible foreign matter of the formulation. In a specific embodiment, the stability of the liquid formulation of the present invention after storage is detected by visual inspection, wherein the appearance of the liquid formulation of the present invention remains in clear to slightly opalescent, and colorless to pale yellow liquid, and free of foreign matter. In one embodiment, the visual inspection under clarity detector shows the absence of visible foreign matter in the formulation.

In other embodiments, the stability may be assessed by measuring the binding affinity of the antibody to its target. For example, a formulation may be regarded as stable, if after storage at e.g., - 80°C, -30°C, -20°C, 5°C, 25°C, 37°C, 45°C, etc. for a defined amount of time (e.g., 7 days to 12 months, 24 months or 36 months), the monoclonal antibody or the antigen-binding fragment thereof that specifically binds to human MASP-2 contained in the formulation of the present invention binds to human MASP-2 with an affinity that is at least 80%, 85%, 90%, 95%, or more of the binding affinity of the antibody prior to said storage. Binding affinity may be determined by any method, such as e.g., ELISA or plasmon resonance. Biological activity may be determined by an MASP-2 activity assay, such as by contacting cells expressing MASP-2 with the formulation comprising the monoclonal antibody or the antigen-binding fragment thereof that specifically binds to human MASP-2. The binding of the antibody to such cells may be measured directly, such as via FACS analysis.

In some embodiments, the stability can be measured, by determining the percentage of the antibody that forms an aggregate in the formulation after storage for a defined amount of time at a defined temperature, wherein stability is inversely proportional to the percent aggregate that is formed. The percentage of aggregated antibody can be determined by, in particular, size exclusion chromatography (e.g., size exclusion high performance liquid chromatography [SE-HPLC]). An "acceptable stability", as that phrase is used herein, refers to that at most 6% of the antibody is detected in an aggregated form in the formulation after storage for a defined amount of time at a given temperature. In certain embodiments, an acceptable stability refers to at most about 6%, 5%, 4%, 3%, 2%, 1%, 0.5% or 0.1% of the antibody is detected in an aggregated form in the formulation after storage for a defined amount of time at a given temperature. The defined amount of time after which stability is measured can be at least 3 days, at least 5 days, at least 1 weeks, at least 10 days, at least 2 weeks, at least 3 weeks, at least 4 weeks, at least 5 weeks, at least 6 weeks or longer. The temperature at which the pharmaceutical formulation may be stored when assessing stability can be any temperature of about -80°C to about 45°C, for example stored at about -80°C, about -30°C, about -20°C, about 0°C, about 4°C-8°C, about 5°C, about 25°C, about 35°C, about 37°C, about 40°C or about 45°C. For example, a pharmaceutical formulation may be regarded as stable, if less than about 10%, 8%, 7%, 6%, 5%, 4%, 3%, 2%, 1.75%, 1.5%, 1.25%, 1%, 0.75%, 0.5%, 0.25% or 0.1% of the antibody is detected in an aggregated form after 6 weeks of storage at 40° C. A pharmaceutical formulation may also be regarded as stable, if less than about 10%, 8%, 7%, 6%, 5%, 4%, 3%, 2%, 1.75%, 1.5%, 1.25%, 1%, 0.75%, 0.5%, 0.25% or 0.1% of the antibody in an aggregated form is detected after 6 weeks of storage at 25° C.

In some embodiments, the stability can be measured, in particular, by determining the percentage of antibody that migrates in a more acidic fraction during ion exchange ("acidic form") than in the main fraction of antibody ("main charge form"), wherein the stability is inversely proportional to the fraction of antibody in the acidic form. The percentage of "acidified" antibody can be determined by ion exchange chromatography (e.g., cation exchange high performance liquid chromatography [CEX-HPLC] or whole column imaged capillary isoelectric focusing electrophoresis [icIEF]). An "acceptable stability", as that phrase is used herein, refers to that up to 52% of the antibody is in a more acidic form detected in the formulation after storage for a defined amount of time at a defined temperature. In certain embodiments, an acceptable stability refers to up to about 52%, 50%, 45%, 40%, 35%, 30%, 29%, 28%, 27%, 26%, 25%, 20%, 15%, 10%, 5%, 4%, 3%, 2%, 1%, 0.5% or 0.1% of the antibody in an acidic form can be detected in the formulation after storage for a defined amount of time at a given temperature. The defined amount of time after which stability is measured can be at least 2 weeks, at least 28 days, at least 1 months, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 30 months, at least 36 months or longer. The temperature at which the pharmaceutical formulation may be stored when assessing stability can be any temperature of about -80°C to about 45°C, for example stored at about -80°C, about -30°C, about -20°C, about 0°C, about 4°C-8°C, about 5°C, about 25°C, about 35°C, about 37°C, about 40°C or about 45°C. For example, A pharmaceutical formulation may be regarded as stable, if less than about 29%, 28%, 27%, 26%, 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% of the antibody in a more acidic form can be detected, after storage for 36 months at -80°C, -30°C or -20°C. A pharmaceutical formulation may also be regarded as stable, if less than about 28%, 27%, 26%, 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% of the antibody in a more acidic form can be detected after 12 months, 24 months, even 36 months of storage at 4° C. A pharmaceutical formulation may also be regarded as stable, if less than about 30%, 29%, 28%, 27%, 26%, 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% of the antibody in a more acidic form can be detected after 4 weeks or 6 weeks of storage at 25° C. A pharmaceutical formulation may also be regarded as stable, if less than about 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% of the antibody in a more acidic form can be detected after 4 weeks or 6 weeks of storage at 37°C. A pharmaceutical formulation may also be regarded as stable, if less than about 52%, 51%, 50%, 49%, 48%, 47%, 46%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 10%, 9%, 8%, 7%, 6%, 5%, 4%, 3%, 2%, 1%, 0.5% or 0.1% of the antibody in a more acidic form can be detected after 4 weeks or 6 weeks of storage at 40° C.

In one embodiment, the stability of the liquid formulation of the present invention is detected by measuring the transition temperature Tₒₙₛₑₜ at which configuration change or aggregation occur to protein during heating process so that the diameter changes. For example, Tₒₙₛₑₜ of the liquid formulation of the present inventionis higher than about 60°C, 63°C, 65°C, 68°C or 70°C.

References to stability of the pharmaceutical formulations "after" a specified period of time are intended to mean that a measurement of a stability parameter is taken at or about the end of the specific time period, and is not intended to mean that the pharmaceutical formulation necessarily maintains the same degree of stability for the measured parameter thereafter. For example, reference to a particular stability after 6 weeks means that the measurement of stability was taken at or about 6 weeks after the start of the study. Additional methods for assessing the stability of an antibody in formulation are demonstrated in the Examples presented below.

In some of the preferred embodiments of the present invention, the formulation is stable after shaking (e.g., after shaking for 14 days), as assessed by the method described above.

In some of the preferred embodiments of the present invention, the formulation is stable after stirring (e.g., after stirring for 6 hours), as assessed by the method described above.

In some of the preferred embodiments of the present invention, the formulation is stable after exposuring to light (e.g., after exposuring to light for 3 days), as assessed by the method described above.

In some of the preferred embodiments of the present invention, the formulation is stable after freezing and thawing (e.g., after freezing and thawing for 5 cycles), as assessed by the method described above.

### Exemplary formulations

In one embodiment, the liquid antibody formulation of the present application comprises:
(a) 20 mg/ml ± 2 mg/ml to 200 mg/ml ± 20 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose, or 200mM to 300mM of proline,
with a pH of 4.7 to 6.0.

In one embodiment, the liquid antibody formulation of the present application comprises:
(a) 20 mg/ml ± 2 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose,
with a pH of 5.3 ± 0.5.

In one embodiment, the liquid antibody formulation of the present application comprises:
(a) 60 mg/ml ± 6 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose,
with a pH of 5.3 ± 0.5.

In one embodiment, the liquid antibody formulation of the present application comprises:
(a) 100 mg/ml ± 10 mg/ml of an antibody,
(b) a buffer comprising 5 mM ± 1 mM to 20 mM ± 4 mM of histidine or acetate,
(c) 0.025% + 0.01% to 0.1% ± 0.01% (w/v) of a polysorbate, and
(d) 6.5% + 0.5% to 8.6% ± 0.5% (w/v) of sucrose or trehalose,
with a pH of 5.3 ± 0.5.

In one embodiment, the liquid antibody formulation of the present application comprises:
(a) 150 mg/ml ± 15 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose,
with a pH of 5.3 ± 0.5.

In some embodiments, the pharmaceutical formulation comprises: (a) 20 mg/ml ± 2 mg/ml of an antibody, (b) a buffer comprising 5 mM ± 1 mM to 20 mM ± 4 mM of histidine or acetate, (c) 0.025% ± 0.01% to 0.1% ± 0.01% (w/v) of a polysorbate, and (d) 6.5% ± 0.5% to 8.6% ± 0.5% (w/v) of sucrose or trehalose, with a pH of 5.3 ± 0.5.

In some embodiments, the pharmaceutical formulation comprises: (a) 20 mg/ml ± 2 mg/ml of an antibody, (b) comprises 10 mM ± 2 mM of histidinea buffer comprising, (c) 0.05% ± 0.01% (w/v) of a polysorbate, and(d) 8.6% ± 0.5% (w/v) of sucrose, with a pH of 5.3 ± 0.1.

In some embodiments, the pharmaceutical formulation comprises: (a) 20 mg/ml ± 2 mg/ml of an antibody, (b) 0.175 mg/ml of L-histidine, (c) 1.86 mg/ml of L-histidine monohydrochloride monohydrate, (d) 0.05% ± 0.01% (w/v) of a polysorbate, and (e) 8.6% ± 0.5% (w/v) of sucrose, with a pH of 5.3 ± 0.1.

In some embodiments, the pharmaceutical formulation comprises: (a) 60 mg/ml ± 6 mg/ml of an antibody, (b) a buffer comprising 5 mM ± 1 mM to 20 mM ± 4 mM of histidine or acetate, (c) 0.025% ± 0.01% to 0.1% ± 0.01% (w/v) of a polysorbate, and (d) 6.5% ± 0.5% to 8.6% ± 0.5% (w/v) of sucrose or trehalose, with a pH of 5.3 ± 0.5.

In some embodiments, the pharmaceutical formulation comprises: (a) 60 mg/ml ± 6 mg/ml of an antibody, (b) a buffer comprising 10 mM ± 2 mM of histidine buffer system, (c) 0.05% ± 0.01% (w/v) of a polysorbate, and (d) 8.6% ± 0.5% (w/v) of sucrose, with a pH of 5.3 ± 0.1.

In some embodiments, the pharmaceutical formulation comprises: (a) 60 mg/ml ± 6 mg/ml of an antibody, (b) 0.175 mg/ml of L-histidine, (c) 1.86 mg/ml of L-histidine monohydrochloride monohydrate, (d) 0.05% ± 0.01% (w/v) of a polysorbate, and (e) 8.6% ± 0.5% (w/v) of sucrose, with a pH of 5.3 ± 0.1.

In some embodiments, the pharmaceutical formulation comprises: (a) 100 mg/ml ± 10 mg/ml of an antibody, (b) a buffer comprising 5 mM ± 1 mM to 20 mM ± 4 mM of histidine or acetate, (c) 0.025% ± 0.01% to 0.1% ± 0.01% (w/v) of a polysorbate, and (d) 6.5% ± 0.5% to 8.6% ± 0.5% (w/v) of sucrose or trehalose, with a pH of 5.3 ± 0.5.

In some embodiments, the pharmaceutical formulation comprises: (a) 100 mg/ml ± 10 mg/ml of an antibody, (b) a buffer comprising 10 mM + 1 mM of histidine buffer system, (c) 0.05% ± 0.01% (w/v) of a polysorbate, and (d) 7.0% ± 0.5% (w/v) of sucrose, with a pH of 5.3 ± 0.1.

In some embodiments, the pharmaceutical formulation comprises: (a) 100 mg/ml ± 10 mg/ml of an antibody, (b) 0.175 mg/ml of L-histidine, (c) 1.86 mg/ml of L-histidine monohydrochloride monohydrate, (d) 0.05% + 0.01% (w/v) of a polysorbate, and (e) 7.0% + 0.5% (w/v) of sucrose, with a pH of 5.3 ± 0.1.

In some embodiments, the pharmaceutical formulation comprises: (a) 150 mg/ml ± 15 mg/ml of an antibody, (b) a buffer comprising 5 mM ± 1 mM to 20 mM ± 4 mM of histidine or acetate, (c) 0.025% ± 0.01% to 0.1% ± 0.01% (w/v) of a polysorbate, and (d) 6.5% ± 0.5% to 8.6% ± 0.5% (w/v) of sucrose or trehalose, with a pH of 5.3 ± 0.5.

In some embodiments, the pharmaceutical formulation comprises: (a) 150 mg/ml ± 15mg/ml of an antibody, (b) a buffer comprising 10 mM ± 1 mM of histidine buffer system, (c) 0.05% ± 0.01% (w/v) of a polysorbate, and (d) 7.0% ± 0.5% (w/v) of sucrose, with a pH of 5.3 ± 0.1.

In some embodiments, the pharmaceutical formulation comprises: (a) 150 mg/ml ± 15 mg/ml of an antibody, (b) 0.175 mg/ml of L-histidine, (c) 1.86 mg/ml of L-histidine monohydrochloride monohydrate, (d) 0.05% ± 0.01% (w/v) of a polysorbate, and (e) 7.0% ± 0.5% (w/v) of sucrose, with a pH of 5.3 ± 0.1.

In any of these examples, the polysorbates may be polysorbate 80 or polysorbate 20, preferably polysorbate 80.

In some embodiments, the pharmaceutical formulation does not comprise other buffer system/buffer.

In some embodiments, the pharmaceutical formulation does not comprise other surfactant.

In some embodiments, the pharmaceutical formulation does not comprise other auxiliary material.

The formulae of the pharmaceutical formulation comprising the anti-MASP-2 antibody or the antigen-binding fragment thereof of the present invention can maintain good stability of the antibody in a liquid state at a higher concentration (e.g., about 100 mg/mL, about 150 mg/mL) and a lower concentration (e.g., about 20 mg/mL, about 50 mg/mL), satisfy the requirements of production process and clinical administration, and support the development of the antibody into an intravenous injection and a subcutaneous injection.

Additional non-limiting examples of the pharmaceutical formulations encompassed by the present invention are set forth elsewhere herein, including the working Examples presented below.

### Containers and Methods of Administration

The pharmaceutical formulations of the present invention may be contained in any container suitable for storage of medicines and other therapeutic compositions. For example, the pharmaceutical formulations may be contained in a sealed and sterilized plastic or glass container having a defined volume such as a vial, penicillin vial (injection vial), ampule, syringe, cartridge, bottle or IV bag. Different types of vials can be used to contain the formulations of the present invention including, e.g., clear and opaque (e.g., amber) glass or plastic vials. Likewise, any type of syringe can be used to contain and/or administer the pharmaceutical formulations of the present invention. In some embodiments, the pharmaceutical formulation is contained in a pre-filled syringe. In some embodiments, the pharmaceutical formulation is contained in a prefilled staked needle syringe.

The pharmaceutical formulations of the present invention may be contained in "normal tungsten" syringes or "low tungsten" syringes. As will be appreciated by an ordinary person skilled in the art, the process of making glass syringes generally involves the use of a hot tungsten rod which functions to pierce the glass thereby creating a hole from which liquids can be drawn and expelled from the syringe. This process results in the deposition of trace amounts of tungsten on the interior surface of the syringe. Subsequent washing and other processing steps can be used to reduce the amount of tungsten in the syringe. As used herein, the term "normal tungsten" refers to that the syringe contains greater than 500 parts per billion (ppb) of tungsten. The term "low tungsten" refers to that the syringe contains less than 500 ppb of tungsten. For example, a low tungsten syringe, according to the present invention, may contain less than about 490, 480, 470, 460, 450, 440, 430, 420, 410, 390, 350, 300, 250, 200, 150, 100, 90, 80, 70, 60, 50, 40, 30, 20, 10 or fewer ppb of tungsten.

The pharmaceutical formulations may be administered to a patient by parenteral routes such as injection (e.g., subcutaneous, intravenous, intramuscular, intraperitoneal, etc.) or percutaneous, mucosal, nasal, pulmonary and/or oral administration. In some embodiments, numerous reusable pen and/or automatic syringe delivery devices can be used to subcutaneously deliver the pharmaceutical formulations of the present invention. In some instances, the pharmaceutical preparation is contained in a syringe that is particularly suitable for use with an automatic syringe.

The use of a microinfusor to deliver the pharmaceutical formulations of the present invention is also contemplated herein. As used herein, the term "microinfusor" refers to a subcutaneous delivery device designed to slowly administer large volumes (e.g., up to about 2.5 mL, about 3.0 mL or more) of a therapeutic formulation over a prolonged period of time (e.g., about 10, 15, 20, 25, 30 or longer).

In certain embodiments, the pharmaceutical the pharmaceutical formulation is administered via an IV drip, such that the formulation is diluted in an IV bag containing a physiologically acceptable solution. In one embodiment, the pharmaceutical composition is a compounded sterile formulation in an intravenous infusion bag, such that a single dose of drug product is diluted into 100 mL, 250 mL (or other like amount suitable for intravenous drip delivery) of a physiological buffer (e.g., 0.9% saline).

The pharmaceutical formulations of the present invention can also be contained in a unit dosage form. As used herein, the term "unit dosage form," refers to a physically discrete unit suitable as a unitary dosage for the patient to be treated, each unit containing a predetermined quantity of active compound calculated to produce the desired therapeutic effect in association with the required pharmaceutical carrier, diluent, or excipient. In various embodiments, the unit dosage form is contained in a container as discussed herein. Actual dosage levels of the active ingredient (e.g., a monoclonal antibody or an antigen-binding fragment thereof that specifically binds to human MASP-2) in the formulation of the present invention may vary so as to obtain an amount of the active ingredient which is effective to achieve the desired therapeutic response for a particular patient, composition, and mode of administration, without adverse effect to the patient. The selected dosage level is depend upon a variety of pharmacokinetic factors including the activity of the particular compositions of the present invention employed, the route of administration, the time of administration, the rate of excretion of the particular compound being employed, the duration of the treatment, other drugs, compounds used in combination with the particular compositions employed, and/or the species, the age, sex, weight, condition, general health and prior medical history of the patient being treated, and like factors well known in the medical arts. The term "diluent" as used herein refers to a solution suitable for altering or achieving an exemplary or appropriate concentration or concentrations as described herein.

In various embodiments, the unit dosage form contains an amount of the active ingredient (e.g., monoclonal antibody or an antigen-binding fragment thereof that specifically binds to human MASP-2) intended for a single use. In various embodiments, the amount of the active ingredient in the unit dosage form is about 0.1 mg to about 5000 mg, about 100 mg to about 1000 mg, and about 100 mg to about 500 mg, about 100 mg to about 400 mg, about 100 mg to about 200 mg, about 200 mg to about 400 mg, about 250 mg to about 350 mg, about 125 mg to about 175 mg, about 275 mg to about 325 mg, about 1 mg to about 250 mg, about 1 mg to about 100 mg, about 1 mg to about 50 mg, about 1 mg to about 25 mg, about 1 mg to about 10 mg, about 1 mg to about 5 mg or ranges or intervals thereof. Ranges intermediate to the above recited amounts, for example, about 2 mg to about 100 mg or 2 mg to 20 mg, are also intended to be part of this invention. For example, ranges of values using a combination of any of the above recited values (or values contained within the above recited ranges) as upper and/or lower limits are intended to be included. In a particular example, the formulation often is supplied as a liquid in unit dosage form. In some embodiments, a unit dosage form contains 2 to 2.5 mg or 10 to 11 mg, 20 to 25 mg, 80 to 90 mg, 100 to 125 mg, 160 to 180 mg, 200 to 225 or 320 to 360 mg, 200 to 400mg. In some embodiments, a unit dosage form according to the present invention (e.g., a unit dosage form containing an antibody at a concentration of about 100 mg/ml or about 150 mg/mL), is suitable for subcutaneous administration to a patient.

In one embodiment, the present invention provides a unit dosage form comprising about 20 mg, about 80 mg, about 160 mg, about 200mg, about 320 mg or about 400mg of an antibody contained in a stable formulation, wherein the formulation comprises 200mg or 400mg of an antibody.

The present invention also comprises a method of preparing a unit dosage form. In an exemplary example, the method for preparing a pharmaceutical unit dosage form includes combining the formulation of any of foregoing examples in a suitable container (e.g., those containers discussed herein).

In various embodiments, the pharmaceutical formulation is contained in a container (e.g., a vial or a pre-filled syringe) that may contain a headspace gas with less than 5% of an oxidizing gas (e.g., oxygen) by volume. In various embodiments, the concentration of oxidizing gas (e.g., oxygen) in the headspace of the container may be less than 4.5%, less than 4%, less than 3.5%, less than 3%, less than 2.5%, less than 2%, or less than 1.5%. In various embodiments, the concentration of the oxidizing gas (e.g., oxygen) in the headspace is less than about 1%. In one embodiment, the concentration of the oxidizing gas (e.g. oxygen) in the headspace is no more than about 0.5%. In one embodiment, the concentration of the oxidizing gas (e.g. oxygen) in the headspace is no more than about 0.1%. In various embodiments, the concentration of the oxidizing gas (e.g., oxygen) in the headspace of the drug product container is less than 0.9%, less than 0.8%, less than 0.7%, less than 0.6%, less than 0.5%, less than 0.4%, less than 0.3%, less than 0.2%, or less than 0.1%. In some instances, the concentration of oxygen in the headspace gas is from about 0.01% to about 1.5%. In some instances, the concentration of oxygen in the headspace gas is from about 0.75% to about 1.25%. In some instances, the concentration of oxygen in the headspace gas is from about 0.05% to about 0.15%. In various embodiments, the oxidizing gas (e.g., oxygen) in the headspace is replaced, or substantially replaced, with an inert gas, such as nitrogen, argon, helium, xenon, neon, krypton or radon. In one embodiment, the non-oxidizing gas is nitrogen. In one embodiment, the non-oxidizing gas is argon.

### Therapeutic use of the formulations

The pharmaceutical formulation of the present invention is particularly useful for the treatment, prevention and/or alleviation of any disease or disorder associated with cells expressing human MASP-2. Non-limiting exemplary diseases and disorders that could be treated by administration of the pharmaceutical formulation of the present invention comprise those diseases and disorders that woud benefit from the inhibition of MASP-2 dependent compliment activation.

In some embodiment, diseases and disorders that woud benefit from the inhibition of MASP-2 dependent compliment activation comprise an autoimmune disease, a vascular condition, an ischemia-reperfusion injury, atherosclerosis, an inflammation, a pulmonary condition, extracorporeal reperfusion procedure, a musculoskeletal condition, a renal condition, a skin condition, an organ or tissue transplant procedure, a nervous system disorder or injury, a blood disorder, urogenital condition, a nonobese diabetes or a complication associated with Type 1 or Type 2 diabetes, cancer, endocrine disorder, an ophthalmologic condition, or COVID-19 (pneumonia caused by SARS-Cov-2).

In some embodiments, the autoimmune disease comprises thrombotic microangiopathies (TMAs), atypical hemolytic uremic syndrome (aHUS), hematopoietic transplant-associated thrombotic microangiopathy (TA-TMA), lupus nephritis, systemic lupus erythematosus (SLE) and IgA nephropathy.

In some embodiments, the vascular condition comprises a cardiovascular condition, a cerebrovascular condition, a peripheral (e.g., musculoskeletal) vascular condition, a renovascular condition, a mesenteric/enteric vascular condition, revascularization to transplants and/or replants, vasculitis, Henoch-Schonlein purpura nephritis, systemic lupus erythematosus-associated vasculitis, vasculitis associated with rheumatoid arthritis, immune complex vasculitis, Takayasu's disease, dilated cardiomyopathy, diabetic angiopathy, Kawasaki's disease (arteritis), venous gas embolus (VGE), and restenosis following stent placement, rotational atherectomy and percutaneous transluminal coronary angioplasty (PTCA).

In some embodiments, the ischemia-reperfusion injury comprises an ischemia-reperfusion injury associated with aortic aneurysm repair, cardiopulmonary bypass, vascular reanastomosis in connection with organ transplants and/or extremity/digit replantation, stroke, myocardial infarction, and hemodynamic resuscitation following shock and/or surgical procedures.

In some embodiments, the inflammation comprises inflammatory gastrointestinal disorder comprising pancreatitis, Crohn's disease, ulcerative colitis, irritable bowel syndrome and diverticulitis.

In some embodiments, the pulmonary condition comprises acute respiratory distress syndrome, transfusion-related acute lung injury, ischemia/reperfusion acute lung injury, chronic obstructive pulmonary disease, asthma, Wegener's granulomatosis, antiglomerular basement membrane disease (Goodpasture's disease), meconium aspiration syndrome, bronchiolitis obliterans syndrome, idiopathic pulmonary fibrosis, acute lung injury secondary to burn, non- cardiogenic pulmonary edema, transfusion-related respiratory depression, emphysema, cystic fibrosis, SARS-CoV, MERS-CoV and SARS-CoV-2(Covid-19) related condition.

In some embodiments, the extracorporeal reperfusion procedure comprises hemodialysis, plasmapheresis, leukopheresis, exfracorporeal membrane oxygenator (ECMO), heparin-induced extracorporeal membrane oxygenation LDL precipitation (HELP) and cardiopulmonary bypass (CPB).

In some embodiments, the musculoskeletal condition comprises osteoarthritis, rheumatoid arthritis, juvenile rheumatoid arthritis, gout, neuropathic arthropathy, psoriatic arthritis, spondyloarthropathy, crystalline arthropathy and systemic lupus erythematosus (SLE). In some embodiments, the renal condition comprises mesangioproliferative glomerulonephritis, membranous glomerulonephritis, membranoproliferative glomerulonephritis (mesangiocapillary glomerulonephritis), acute postinfectious glomerulonephritis (poststreptococcal glomerulonephritis), cryoglobulinemic glomerulonephritis, lupus nephritis, Henoch- Schonlein purpura nephritis and IgA nephropathy.

In some embodiments, the skin condition comprises psoriasis, autoimmune bullous dermatoses, eosinophilic spongiosis, bullous pemphigoid, Epidermolysis bullosa acquisita (EBA), herpes gestationis, thermal burn injury and chemical burn injury.

In some embodiments, the organ or tissue transplant procedure comprises organ allotransplantation, organ xenotransplantation organ and tissue graft. In some embodiments, the nervous system disorder or injury comprises multiple sclerosis, myasthenia gravis, Huntington's disease, amyotrophic lateral sclerosis, Guillain Barre syndrome, reperfusion following stroke, degenerative discs, cerebral trauma, Parkinson's disease, Alzheimer's disease, Miller-Fisher syndrome, cerebral frauma and/or hemorrhage, demyellination and meningitis.

In some embodiments, the blood disorder comprises sepsis, severe sepsis, septic shock, acute respiratory distress syndrome resulting from sepsis, systemic inflammatory response syndrome, hemorrhagic shock, hemolytic anemia, autoimmune thrombotic thrombocytopenic purpura and hemolytic uremic syndrome.

In some embodiments, the urogenital condition comprises painful bladder disease, sensory bladder disease, chronic abacterial cystitis, interstitial cystitis, infertility, placental dysfunction and miscarriage and pre-eclampsia. In some embodiments, the endocrine disorder comprises Hashimoto's thyroiditis, stress, anxiety and hormonal disorders involving regulated release of prolactin, growth or other insulin-like growth factor and adrenocorticotropin from the pituitary.

In some embodiments, the ophthalmologic condition comprises age-related macular degeneration.

In some embodiments of the methods provided herein, the subject is determined to have an elevated C4 serum level, or have C4d deposition or C4d positive staining in a sample of interest. In some embodiments, the condition or disease is IgA nephropathy. C4d-positive staining has been reported as an independent risk factor for the development of ESRD in IgAN. C4d can be detected using any suitable methods known in the art, for example, by using ELISA, or immunofluorescence microscopy.

The therapeutically effective amount of an antibody or antigen-binding fragment as provided herein will depend on various factors known in the art, such as for example body weight, age, past medical history, present medications, state of health of the subject and potential for cross-reaction, allergies, sensitivities and adverse side-effects, as well as the administration route and extent of disease development. Dosages may be proportionally reduced or increased by one of ordinary skill in the art (e.g., physician or veterinarian) as indicated by these and other circumstances or requirements.

In certain embodiments, an anti-MASP-2 antibody or antigen-binding fragment as provided herein may be administered at a therapeutically effective dosage of about 0.01 mg/kg to about 100 mg/kg (e.g., about 0.01 mg/kg, about 0.5 mg/kg, about 1 mg/kg, about 2 mg/kg, about 3 mg/kg, about 5 mg/kg, about 10 mg/kg, about 15 mg/kg, about 20 mg/kg, about 25 mg/kg, about 30 mg/kg, about 35 mg/kg, about 40 mg/kg, about 45 mg/kg, about 50 mg/kg, about 55 mg/kg, about 60 mg/kg, about 65 mg/kg, about 70 mg/kg, about 75 mg/kg, about 80 mg/kg, about 85 mg/kg, about 90 mg/kg, about 95 mg/kg, or about 100 mg/kg). In certain of these embodiments, the antibody or antigen-binding fragment is administered at a dosage of about 50 mg/kg or less, and in certain of these embodiments the dosage is 10 mg/kg or less, 5 mg/kg or less, 3 mg/kg or less, 1 mg/kg or less, 0.5 mg/kg or less, or 0.1 mg/kg or less. In certain embodiments, the administration dosage may change over the course of treatment. For example, in certain embodiments the initial administration dosage may be higher than subsequent administration dosages. In certain embodiments, the administration dosage may vary over the course of treatment depending on the reaction of the subject.

Dosage regimens may be adjusted to provide the optimum desired response (e.g., a therapeutic response). For example, a single dose may be administered, or several divided doses may be administered over time.

The anti-MASP-2 antibodies and antigen-binding fragments disclosed herein may be administered by any route known in the art, such as for example parenteral (e.g., subcutaneous, intraperitoneal, intravenous, including intravenous infusion, intramuscular, or intradermal injection) or non-parenteral (e.g., oral, intranasal, intraocular, sublingual, rectal, or topical) routes.

In some embodiments, the anti-MASP-2 antibodies or antigen-binding fragments disclosed herein may be administered alone or in combination with one or more additional therapeutic means or agents. For example, the antibodies or antigen-binding fragments disclosed herein may be administered in combination with another therapeutic agent, for example, an anti-autoimmune drug.

In certain of these embodiments, an anti-MASP-2 antibody or antigen-binding fragment as disclosed herein that is administered in combination with one or more additional therapeutic agents may be administered simultaneously with the one or more additional therapeutic agents, and in certain of these embodiments the antibody or antigen-binding fragment and the additional therapeutic agent(s) may be administered as part of the same pharmaceutical composition. However, an anti-MASP-2 antibody or antigen-binding fragment administered "in combination" with another therapeutic agent does not have to be administered simultaneously with or in the same composition as the agent. An anti-MASP-2 antibody or antigen-binding fragment administered prior to or after another agent is considered to be administered "in combination" with that agent as the phrase is used herein, even if the antibody or antigen-binding fragment and second agent are administered via different routes. Where possible, additional therapeutic agents administered in combination with the antibodies or antigen-binding fragments disclosed herein are administered according to the schedule listed in the product information sheet of the additional therapeutic agent, or according to the Physicians' Desk Reference 2003 (Physicians' Desk Reference, 57th Ed; Medical Economics Company; ISBN: 1563634457; 57th edition (November 2002)) or protocols well known in the art.

The treatment method of the present invention comprises administering to a subject any formulation comprising the monoclonal antibody or an antigen-binding fragment thereof that specifically binds to human MASP-2 as disclosed herein. The subject to whom the formulation is administered may be, for example, any human or non-human animal in need of such treatment. For example, the subject may be diagnosed with any of the aforementioned diseases or disorders or may be considered at risk of developing any of the aforementioned diseases or disorders. The present invention further includes the use of any of the formulations disclosed herein in the manufacture of a medicament for the treatment of any disease or disorder associated with cells expressing human MASP-2, wherein such diseases or disorders include any of the exemplary diseases, disorders or conditions as mentioned above.

In some embodiments, the present invention provides a kit comprising the formulations described herein (e.g., a container comprising a formulation or a unit dosage form) and packaging or labeling (e.g., a package insert) and instructions for use of the formulation for treating the diseases or disorders as discussed above. In some cases, the instructions provide the use of the unit dosage form discussed herein for treating diseases or conditions.

### SPECIFIC EMBODIMENTS

The following examples are put forth so as to provide those skilled in the art with a complete disclosure and description of how to make and use the methods and compositions of the present invention, and are not intended to limit the scope claimed in the invention. Efforts have been made to ensure accuracy with respect to numbers used (e.g., amounts, temperature, etc.) but some experimental errors and deviations should be accounted for. Unless indicated otherwise, parts are parts by weight, molecular weight is mean molecular weight, temperature is in degrees Centigrade, and pressure is at or near atmospheric.

Notes: in the table of the present invention, NT unless otherwise specified, refers to not tested; ND unless otherwise specified, refers to not detected.

### EXAMPLE 1: Generation of human, mouse, cynomolgus MASP-2 antigens

### 1. Construction of Antigen for expression

To limit antibodies' epitope to the complement binding and activation domains of human MASP-2, a chimeric antigen for animal immunization was designed. The coding sequences expressing mouse MASP-2 CUB1-EGF-CUB2 domain (residues 20-297 of SEQ ID NO: 40) and human MASP-2 CCP1-CCP2-SP domain (residues 298-686 of SEQ ID NO: 39) were synthesized. In addition, an IL-2 secretion signal peptide sequence (SEQ ID NO:41) was added at the N terminal and a FLAG-tag sequence was added at the C terminal. The above elements were combined into one open reading frame (ORF) to make a chimeric antigen expression construct (SEQ ID NO:42). Other constructs expressing the full-length human MASP-2 (SEQ ID NO:39), mouse MASP-2 (SEQ ID NO:40) and cynomolgus MASP-2 (SEQ ID NO: 43), with a HIS-tag at the C terminal, were made by synthesis. Also, another construct expressing the full length human MASP-2 (SEQ ID NO:39) with a FLAG-tag at the C terminal was made by PCR.
Human MASP-2 protein (SEQ ID NO:39) 
MRLLTLLGLLCGSVATPLGPKWPEPVFGRLASPGFPGEYANDQERRWTLTAPPGYRLRLYFT
HFDLELSHLCEYDFVKLSSGAKVLATLCGQESTDTERAPGKDTFYSLGSSLDITFRSDYSNEK
PFTGFEAFYAAEDIDECQVAPGEAPTCDHHCHNHLGGFYCSCRAGYVLHRNKRTCSALCSGQ
VFTQRSGELSSPEYPRPYPKLSSCTYSISLEEGFSVILDFVESFDVETHPETLCPYDFLKIQTDRE
EHGPFCGKTLPHRIETKSNTVTITFVTDESGDHTGWKIHYTSTAQPCPYPMAPPNGHVSPVQA
KYILKDSFSIFCETGYELLQGHLPLKSFTAVCQKDGSWDRPMPACSIVDCGPPDDLPSGRVEYI
TGPGVTTYKAVIQYSCEETFYTMKVNDGKYVCEADGFWTSSKGEKSLPVCEPVCGLSARTT
GGRIYGGQKAKPGDFPWQVLILGGTTAAGALLYDNWVLTAAHAVYEQKHDASALDIRMGT
LKRLSPHYTQAWSEAVFIHEGYTHDAGFDNDIALIKLNNKVVINSNITPICLPRKEAESFMRTD
DIGTASGWGLTQRGFLARNLMYVDIPIVDHQKCTAAYEKPPYPRGSVTANMLCAGLESGGK
DSCRGDSGGALVFLDSETERWFVGGIVSWGSMNCGEAGQYGVYTKVINYIPWIENIISDF
Mouse MASP-2 protein (SEQ ID NO:40) 
MRLLIFLGLLWSLVATLLGSKWPEPVFGRLVSPGFPEKYADHQDRSWTLTAPPGYRLRLYFT
HFDLELSYRCEYDFVKLSSGTKVLATLCGQESTDTEQAPGNDTFYSLGPSLKVTFHSDYSNEK
PFTGFEAFYAAEDVDECRVSLGDSVPCDHYCHNYLGGYYCSCRAGYVLHQNKHTCSALCSG
QVFTGRSGYLSSPEYPQPYPKLSSCTYSIRLEDGFSVILDFVESFDVETHPEAQCPYDSLKIQTD
KGEHGPFCGKTLPPRIETDSHKVTITFATDESGNHTGWKIHYTSTARPCPDPTAPPNGSISPVQ
AIYVLKDRFSVFCKTGFELLQGSVPLKSFTAVCQKDGSWDRPMPECSIIDCGPPDDLPNGHVD
YITGPEVTTYKAVIQYSCEETFYTMSSNGKYVCEADGFWTSSKGEKLPPVCEPVCGLSTHTIG
GRIVGGQPAKPGDFPWQVLLLGQTTAAAGALIHDNWVLTAAHAVYEKRMAASSLNIRMGIL
KRLSPHYTQAWPEEIFIHEGYTHGAGFDNDIALIKLKNKVTINGSIMPVCLPRKEAASLMRTD
FTGTVAGWGLTQKGLLARNLMFVDIPIADHQKCTAVYEKLYPGVRVSANMLCAGLETGGK
DSCRGDSGGALVFLDNETQRWFVGGIVSWGSINCGAADQYGVYTKVINYIPWIENIISNF
IL-2 secretion signal peptide sequence (SEQ ID NO:41)
   MYRMQLLSCI ALSLALVTNS
Chimeric MASP-2 protein (SEQ ID NO: 4) 
MYRMQLLSCIALSLALVTNSSKWPEPVFGRLVSPGFPEKYADHQDRSWTLTAPPGYRLRLYF
THFDLELSYRCEYDFVKLSSGTKVLATLCGQESTDTEQAPGNDTFYSLGPSLKVTFHSDYSNE
KPFTGFEAFYAAEDVDECRVSLGDSVPCDHYCHNYLGGYYCSCRAGYVLHQNKHTCSALCS
GQVFTGRSGYLSSPEYPQPYPKLSSCTYSIRLEDGFSVILDFVESFDVETHPEAQCPYDSLKIQT
DKGEHGPFCGKTLPPRIETDSHKVTITFATDESGNHTGWKIHYTSTAQPCPYPMAPPNGHVSP
VQAKYILKDSFSIFCETGYELLQGHLPLKSFTAVCQKDGSWDRPMPACSIVDCGPPDDLPSGR
VEYITGPGVTTYKAVIQYSCEETFYTMKVNDGKYVCEADGFWTSSKGEKSLPVCEPVCGLSA
RTTGGRIYGGQKAKPGDFPWQVLILGGTTAAGALLYDNWVLTAAHAVYEQKHDASALDIR
MGTLKRLSPHYTQAWSEAVFIHEGYTHDAGFDNDIALIKLNNKVVINSNITPICLPRKEAESF
MRTDDIGTASGWGLTQRGFLARNLMYVDIPIVDHQKCTAAYEKPPYPRGSVTANMLCAGLE
SGGKDSCRGDSGGALVFLDSETERWFVGGIVSWGSMNCGEAGQYGVYTKVINYIPWIENIIS
DFDYKDDDDK
Cynomolgus MASP-2 protein (SEQ ID NO: 43)

MRLLTLLGLLCGSVATPLGPKWPEPVFGRLASPGFPGEYANDQERRWTLTAPPGYRLRLYFT
HFDLELSHLCEYDFVKLSSGAKVLATLCGHESTDTERAPGNDTFYSLGSSLDITFRSDYSNEK
PFTGFEAFYAAEDIDECQVAPGEAPACDHHCHNHLGGFYCSCRVGYILHRNKRTCSALCSGQ
VFTQRSGELSSPEYPQPYPKLSSCTYSIRLEEGFSVILDFVESFDVETHPETLCPYDFLKIQIDSE
EHGPFCGKTLPRRIETKSNTVTITFVTDESGDHTGWKIHYTSTAQPCPYPMAPPNGHLSPVQA
KYILKDSFSIFCEPGYELLQGHLPLKSFAAVCQKDGSWDQPMPSCSIVDCGPPDDLPSGRVEYI
TGPEVTTYKAVIQYSCEETFYTMKVNDGKYVCEADGFWTSSKGERSPPVCEPVCGLSARTTG
GRIYGGQKAKPGDFPWQVLILGGSTAAGALLYDNWVLTAAHAIYEQKHDASSLDIRLGALK
RLSPHYTQAWAEAVFIHEGYTHDAGFDNDIALIKLNNKVVINSNITPICLPRKEAESFMRTDDI
GTASGWGLTQRGLLARNLMYVDIPIVDHQKCTAAYEKPPYSGGSVTANMLCAGLESGGKDS
CRGDSGGALVFLDNETQRWFVGGIVSWGSMNCGEAGQYGVYTKVINYIPWIKNIISNF

### 2. Expression and Purification of MASP-2 Antigens

The MASP-2 expression constructs as described above were separately transfected into ExpiCHO-s cells with an ExpiFectamine CHO transfection kit. ExpiCHO-s cells were cultured in the ExpiCHO Expression Medium without serum. 14 days post transfection, the supernatants were collected. After centrifuge and filtration, supernatants were loaded onto anti-FLAG or anti-HIS column and then purified with a GE AKTA purification system. After washing, MASP-2 proteins were eluted with citric acid (pH3.5) for animal immunization.

### EXAMPLE 2: MASP-2 antibody generation

### 1. Immunization and Hybridoma Fusion

Mice or rats of different lineages were immunized with DNA that expressing the chimeric MASP-2 antigen described above via a Helios gene gun system (Bio-Rad). The immunized animals were boosted with the recombinant MASP-2 protein every two weeks. The animals were sacrificed for hybridoma fusion 4 days after the final boost. Spleen cells were isolated and fused with SP2-0 cells via an electro-fusion method. The resulting hybridoma cells were cultured in DMEM medium containing hypoxanthine-aminopterin-thymidine.

### 2. Hybridoma Screening

After 10-days culture, hybridoma supernatants were collected for antigen binding screening. Full-length human MASP-2 protein was coated at a concentration of 0.5 µg/ml 100 µl per well in ELISA plate. After blocking with PBS containing 1% BSA + 1% normal goat serum + 0.05% Tween20, the hybridoma supernatants were added to the plate for 1hr. The specific binding of the hybridoma antibodies to human MASP-2 was detected with a horseradish peroxidase (HRP)-linked anti-mouse antibody. The ELISA binding positive clones were selected and proceeded to further activity screening.

### 3. Activity Screening

ELISA plates were coated with 10 µg/ml mannan 100 µl per well at 4°C for overnight. After washing 3 times with PBS + 0.1% Tween20, the plates were blocked for 1hr with the blocking buffer (10 mM Tris-HCl + 0.1% human serum albumin + 140 mM NaCl). 50 µl of the hybridoma supernatants were mixed with 50 µl of 1% human serum (Quidel, A113) diluted with the assay buffer (0.1% human serum albumin + 20 mM Tris-HCl + 2 mM CaCl₂ + 140 mM NaCl + 1 mM MgCl₂ + 0.05% Tween20) and then incubated on ice for 45min. Blocking buffer was removed from the mannan coated plates, and the supernatant-serum mixture were added. The plates were incubated at 37°C for 1.5hr. After washing 3 times with the washing buffer, activation of C4 was monitored by measuring C4b deposition. The deposited C4b was detected by an HRP-linked anti-C4c antibody (Quidel-A211). If the activity of MASP-2 is inhibited by antibody, there is less C4b deposited on the bottom of the plate. By using this method, the hybridoma antibodies with MASP-2 inhibition activity were selected.

The positive antibodies were then subjected to subclone and re-screened with both ELISA binding and C4 activation assays.

### EXAMPLE 3: Characterization of inhibition activity of anti-human MASP-2 antibodies.

### 1. Hybridoma Antibodies Purification

After subcloning, the positive hybridoma clones with neutralization activity were expanded in 10cm dish. After centrifuge and filtration, the antibody-containing supernatant was loaded onto protein A column and purified with the GE AKTA purification system. After washing, antibody was eluted with citric acid (pH3.5).

### 2. Activity Evaluation of Purified Antibodies

MASP-2 is a key component of lectin pathway, and it cleaves complement factors C4 and C2, generating C3 convertase C4bC2a. Activation of C3 finally leads to the formation of membrane attack complex (MAC). To test whether antibodies that inhibit MASP-2 could reduce activation of the lectin pathway, activation of C4, C3 and MAC was evaluated in the presence of the purified MASP-2 antibodies.

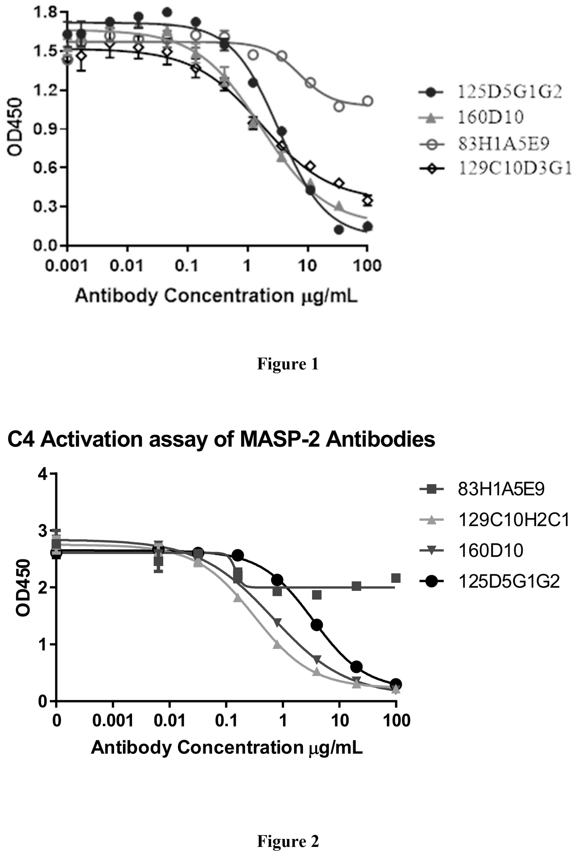

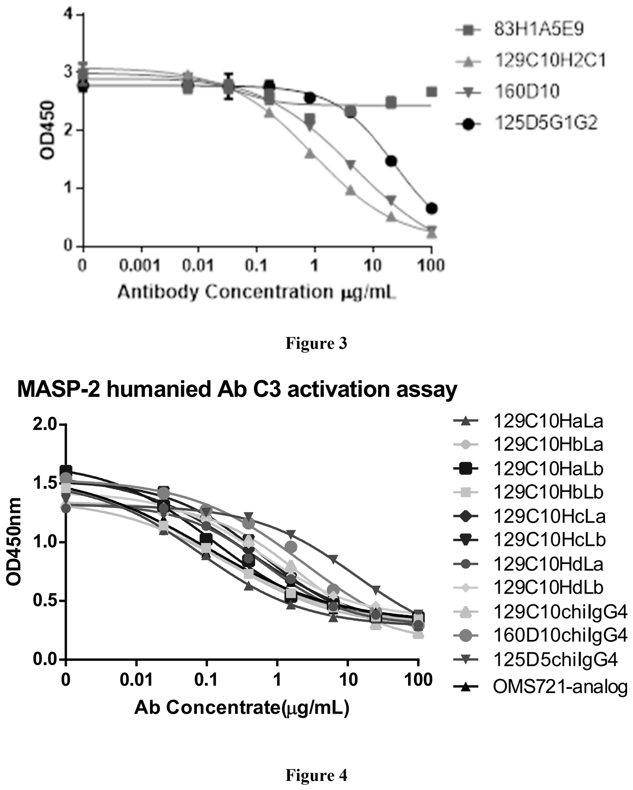

ELISA plates were coated with 10 µg/ml mannan 100 µl per well at 4°C for overnight. After washing 3 times with PBS + 0.1% Tween20, the plates were blocked for 1hr with the blocking buffer (10 mM Tris-HCl + 0.1% human serum albumin + 140 mM NaCl). Antibodies were serially diluted with the assay buffer (0.1% human serum albumin + 20 mM Tris-HCl + 2 mM CaCl₂ + 140mM NaCl + 1 mM MgCl₂ + 0.05% Tween20) containing 1% human serum (Quidel, A113) and incubated on ice for 45min. The blocking buffer was removed from the mannan coated plates and the antibody-serum mixture was added. The plates were incubated at 37°C for 1.5hr. The activated complement components should be deposited on the bottom surface of the plate, while the inactivated components remain soluble in the buffer. After washing for 3 times with the washing buffer, activation of the complement components was monitored by HRP-linked complement antibodies including anti-C3c antibody (Quidel-A205), anti-C4c antibody (Quidel-A211) and anti-MAC (SC5b-9) antibody (Quidel-A239). The detection antibodies were linked with HRP in house. The MASP-2 antibodies purified from hybridoma cells blocked the activation of complement C3 (see Figure 1), complement C4 (see Figure 2) and MAC (see Figure 3) in a dose-dependent manner.

### EXAMPLE 4: V-gene cloning and generation of chimeric antibodies

### 1. V-gene cloning and sequencing of hybridoma antibodies

The lead antibodies with desired profile were selected for V-gene cloning. The sequences of the murine anti-human MASP-2 light chain and heavy chain variable regions were obtained by the polymerase chain reaction (PCR) amplification technique. Total RNA from the positive hybridoma cell was isolated by using the MiniBest Universal RNA Extraction Kit (TaKaRa), and the cDNA was synthesized by using 1st Strand cDNA Synthesis Kit (TaKaRa) with Oligo (dT) primer. The variable regions of mouse IgG gene were amplified by PCR by using primers of different isotypes for heavy chain variable region and Kappa chain primers for light chain variable region. The PCR products were subcloned into a TA cloning vector. For each variable gene construct, more than 10 single colonies were used for DNA sequencing by Synbio Technologies (Suzhou, China). The amino acid sequences of VH and Vκ were derived from the DNA sequencing results.

### 2. Construction of chimeric antibodies

Three antibodies with different sequences, including 129C10, 160D10 and 125D5 were selected as lead antibodies to generate chimeric antibodies, and their SEQ were listed in the Tables 1 and 2. After sequencing analysis and confirmation, cDNAs of the variable regions of heavy chain and light chain were synthesized and fused with the sequences of the constant region of human IgG4 and human kappa. To enhance antibody secretion, the signal peptide sequences were added at N-terminal of heavy chain and light chain, respectively. The resulting chimeric antibody genes were cloned into an expression vector. Large-scale DNA was prepared by using Plasmid Maxi-prep System from Qiagen.

### 3. Expression and purification of chimeric antibodies

Co-transfection of heavy chain and light chain was carried out using the ExpiFectamine^{™} CHO Reagent from Invitrogen according to the manufacturer's protocol. ExpiCHO-S cells in ExpiCHO Expression Medium at 5-6x10⁶ cell / ml were transfected with equal amount of heavy chain vector and light chain vector DNA at a final concentration of 0.8 µg/ml by using ExpiFectamine^{™} CHO Reagent. The plasmid DNA or ExpiFectamine^{™} CHO Reagent was diluted with cold OptiPRO^{™} medium, and then mixed by swirling tube and/or inversion. The ExpiFectamine^{™} CHO/plasmid DNA mixtures were incubated at room temperature for 1-5 minutes, and then slowly transferred into a shaker flask with cells. The transfecting cells were incubated at 37°C with a humidified atmosphere of 5% CO₂ on an orbital shaker (125 rpm shaking speed). 18 to 22 hours after transfection, ExpiCHO^{™} Feed was added, and the conditioned medium was harvested on day 10. The supernatant was centrifuged at 4,000 rpm for 20 minutes and then filtered through 0.22 µm filtration capsule to remove cell debris. The filtered supernatant was loaded onto a pre-equilibrated Protein-A affinity column. Protein-A resin was washed with equilibration buffer (PBS), and 25 mM citrate (pH3.5) was then used to elute the antibody. The purified antibody solution was adjusted to pH 6.0-7.0 by using 1M Tris-base (pH 9.0). The endotoxin was controlled below 1 EU/mg. Finally, the purified antibody was characterized by SDS-PAGE.

### EXAMPLE 5: Generation and Characterization of humanized antibodies

### 1. Generation, expression and purification of humanized antibodies

The sequences of the variable domains of mouse antibody 129C10 were used to identify the germline sequences with the highest homology to their respective murine framework. Computer-modelling was used for designing the humanized variants with CDR grafting and back mutations. **129C10**

Human germline framework sequences VH/1-2 for heavy chain and VK/2-30 for light chain were used for CDR grafting, respectively.

Heavy chain (HC) variants 1, 2, 3 and 4 were obtained by direct grafting the three CDRs to the germline sequence (SEQ ID NO: 37), and in addition the mutations of R71V, A93T for HC variant 1 (SEQ ID NO: 20), R71V, A93T, V67A, M69L for HC variant 2 (SEQ ID NO: 22), R71V, A93T, A65G, K64Q, E61Q for HC variant 3 (SEQ ID NO: 24) and R71V, A93T, V67A, M69L, A65G, K64Q, E61Q for HC variant 4 (SEQ ID NO: 26), respectively. It should be noted that there are 3 mutations (A65G, K64Q, E61Q) of HC variants 3 and 4 introduced in HC CDR2, in order to further improve the antibody's humanness.
Germline sequence for 129C10 HC:
VH/1-2 (129C10-HC germline, SEQ ID NO: 37): 
QVQLVQSGAEVKKPGASVKVSCKASGYTFTGYYMHWVRQAPGQGLEWMGWINPNSGGTN
YAQKFQGRVTMTRDTSISTAYMELSRLRSDDTAVYYCAR
VH/1-2 variant 1 (Hu129C10_Ha, SEQ ID NO: 20): 
QVQLVQSGAEVKKPGASVKVSCKASGYTFTDYYINWVRQAPGQGLEWMGWIFPGSESAYH
SEKFKARVTMTVDTSISTAYMELSRLRSDDTAVYYCTRGDRSGPFAYWGQGTLVTVSS
VH/1-2 variant 2 (Hu129C10_Hb, SEQ ID NO: 22): 
QVQLVQSGAEVKKPGASVKVSCKASGYTFTDYYINWVRQAPGQGLEWMGWIFPGSESAYH
SEKFKARATLTVDTSISTAYMELSRLRSDDTAVYYCTRGDRSGPFAYWGQGTLVTVSS
VH/1-2 variant 3 (Hu129C10_Hc, SEQ ID NO: 24): 
QVQLVQSGAEVKKPGASVKVSCKASGYTFTDYYINWVRQAPGQGLEWMGWIFPGSESAYH
SQKFQGRVTMTVDTSISTAYMELSRLRSDDTAVYYCTRGDRSGPFAYWGQGTLVTVSS
VH/1-2 variant 4 (Hu129C10_Hd, SEQ ID NO: 26):

QVQLVQSGAEVKKPGASVKVSCKASGYTFTDYYINWVRQAPGQGLEWMGWIFPGSESAYH
SQKFQGRATLTVDTSISTAYMELSRLRSDDTAVYYCTRGDRSGPFAYWGQGTLVTVSS

Light chain (LC) variants 1 and 2 were obtained by direct grafting the three CDRs to the germline sequence (SEQ ID NO: 38), and in addition the back mutations of F36L for LC variant 1 (SEQ ID NO: 28) and F36L, T69A for LC variant 2 (SEQ ID NO: 30), respectively.
Germline sequences for 129C10 LC
VK/2-30 (129C10-LC-germline, SEQ ID NO: 38) 
DVVMTQSPLSLPVTLGQPASISCRSSQSLVYSDGNTYLNWFQQRPGQSPRRLIYKVSNRDSGV
PDRFSGSGSGTDFTLKISRVEAEDVGVYYCMQGTHWP
VK/2-30 variant 1 (Hu129C10_La, SEQ ID NO: 28) 
DVVMTQSPLSLPVTLGQPASISCKSSQSLLYSNGKTYLNWLQQRPGQSPRRLIYLVSKLDSGV
PDRFSGSGSGTDFTLKISRVEAEDVGVYYCVQVTHFPFTFGQGTKLEIK
VK/2-30 variant 2 (Hu129C10_Lb, SEQ ID NO: 30)

DVVMTQSPLSLPVTLGQPASISCKSSQSLLYSNGKTYLNWLQQRPGQSPRRLIYLVSKLDSGV
PDRFSGSGSGADFTLKISRVEAEDVGVYYCVQVTHFPFTFGQGTKLEIK

The cDNAs of the variable regions of the above heavy chains and light chains were synthesized and then fused with the sequences of the constant region of human IgG4 and human kappa. The resulting antibody gene sequences were cloned into an expression vector. Large-scale DNA was prepared by using Plasmid Maxiprep System from Qiagen, and cell transfection was carried out using the ExpiFectamine^{™} CHO Reagent from Invitrogen according to the manufacturer's protocol. Supernatant was harvested when cell viability was more than 60% and filtered through 0.22 µm filtration capsule to remove cell debris. The filtered supernatant was subsequently loaded onto a pre-equilibrated Protein-A affinity column. Protein A resin was washed with equilibration buffer (PBS), and 25 mM citrate (pH3.5) was then used to elute antibody. The purified antibody solution was adjusted to pH 6.0-7.0 by using 1M Tris-base (pH 9.0). The endotoxin was controlled below 1 EU/mg. Finally, the purified antibody was characterized by SDS-PAGE.

Benchmark antibody OMS721-analog and 129C10-hu-YTE (Hu129C10_HaLa-hIgG4 with amino acid substitutions M252Y/S254T/T256E [YTE], see Tables 2-3) were also constructed, and expression and purification procedures were same as above.

### Example 6: Blocking activity of the chimeric and humanized MASP-2 antibodies

The purified antibodies were serially diluted from 100 µg/ml to get a gradient concentration. The complement C3 activation assay was used to evaluate MASP-2 antibody blocking activity as described in Example 3. 100 µl per well of 10 µg/ml mannan was coated onto ELISA plate at 4°C for overnight. Antibodies were incubated with 1% human serum (Quidel, A113) for 45 min on ice. After washing and blocking of the plate, the antibody-serum mixture was added and incubated at 37°C for 90 min. After washing, the deposited activated C3 was detected with an HRP-linked anti-C3c antibody (Quidel-A205). Figure 4 shows blocking activity of the chimeric and humanized antibodies for activation of the complement C3. As the humanized 129C10 variant, 129C10HaLa, showed best affinity and C3 blocking activity, 129C10HaLa was selected as the lead antibody for further *in vitro* and *in vivo* studies and it was named as 129C10-hu.

### EXAMPLE 7: Activity comparison of blocking complement C4 and MAC between the lead antibody 129C10-hu and the benchmark antibody OMS721-analog

### 1. Activity Comparison of Blocking Complement C4 and MAC Activation

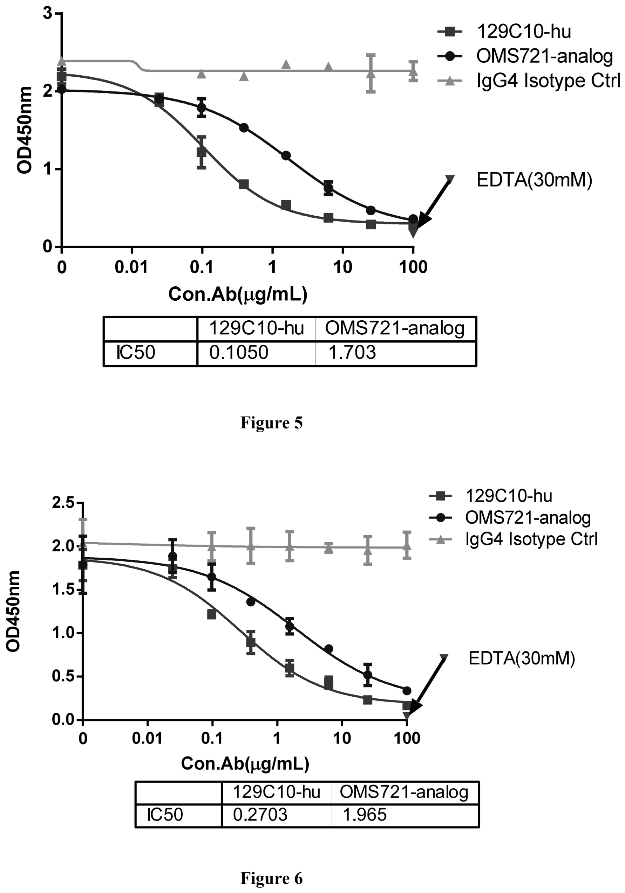

The lead MASP-2 antibody 129C10-hu was compared with OMS721-analog in their blocking activity to complement C4 and MAC activation in 2% human serum using the assay as described in Example 3 with minor changes. 100 µl per well of 10 µg/ml mannan was coated onto ELISA plate at 4°C for overnight. Antibodies were incubated with 2% human serum (Quidel, A113) for 45 min on ice. After washing and blocking of the plate, the antibody-serum mixture was added and incubated at 37°C for 90 min. After washing, the deposited activated C4 was detected with an HRP-linked antiC4c antibody (Quidel-A211). For MAC activation, it was detected with an HRP-linked anti-MAC (SC5b-9) antibody (Quidel-A239). As shown in Figure 5 and Figure 6, the results demonstrated that 129C10-hu was around 10-folds more potent than OMS721-analog in blocking activation of complement C4 (IC50: 0.11 µg/mL vs. 1.70 µg/mL) and MAC (IC50: 0.27 µg/mL vs. 1.97 µg/mL).

### 2. Activity Comparison in Different Concentrations of Serum

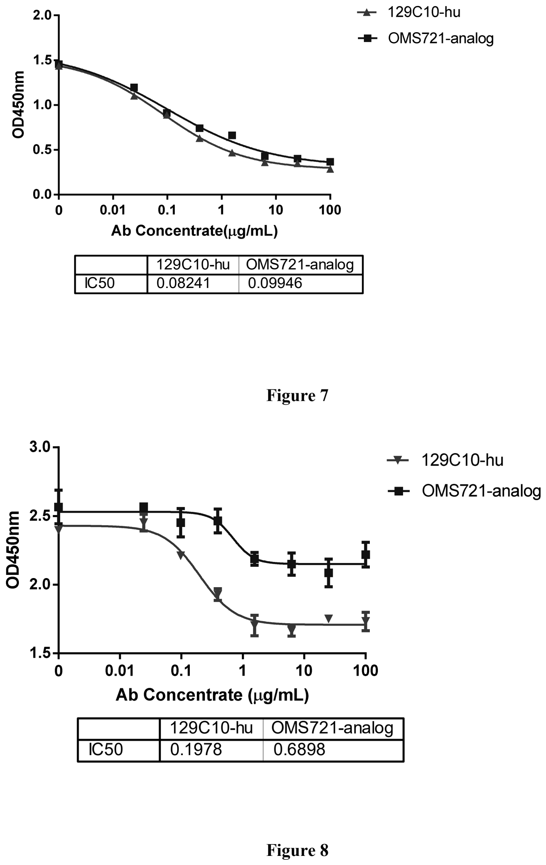

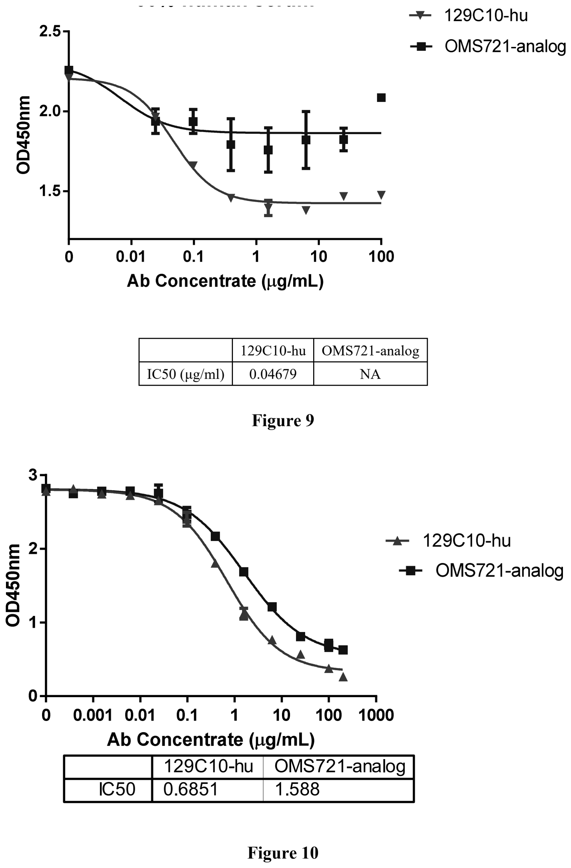

Different concentrations (1%, 10% and 50%) of human serum were used in complement C3 activation assay. For 1% and 10% human serum, the assay method was same as described in Example 3. For 50% human serum, mannan was coated at a concentration of 1 µg/ml, 100 µl per well at 4°C for overnight. After washing 3 times with PBS + 0.1% Tween20, the plates were blocked for 1hr with blocking buffer (10 mM Tris-HCl + 0.1% human serum albumin + 140 mM NaCl). Antibodies were serially diluted with assay buffer (0.1% human serum albumin + 20 mM Tris-HCl + 2 mM CaCl₂ + 140mM NaCl + 1 mM MgCl₂ + 0.05% Tween20) containing 50% human serum (Quidel, A113) and then incubated on ice for 45min. Blocking buffer was removed from mannan coated plates and the antibody-serum mixture were added. The plates were incubated at 37°C for 30 min. After washing for 3 times, the activated C3 was detected with an HRP-linked anti-C3c antibody (Quidel-A205). As shown in Figure 7, 129C10-hu and OMS721-analog had a similar inhibition efficacy to C3 activation in 1% serum (IC50: 0.08 µg/mL vs. 0.10 µg/mL). Interestingly 129C10-hu demonstrated 3-folds more potent than OMS721-analog at a high concentration of 10% serum (IC50: 0.20 µg/mL vs. 0.69 µg/mL) (see Figure 8). Even more significantly at a concentration of 50% serum, 129C10-hu remained its blocking activity to C3 activation at an IC50 of 0.05 µg/mL, while OMS721-analog lost its activity (see Figure 9). For blocking C4 activation, 129C10-hu also showed 2-folds more potent than OMS721-analog in 10% human serum (IC50: 0.69 µg/mL vs. 1.59 µg/mL) (see Figure 10).

### EXAMPLE 8: Binding affinity of MASP-2 antibodies to human MASP-2 by Bio-Layer Interferometry (ForteBio)

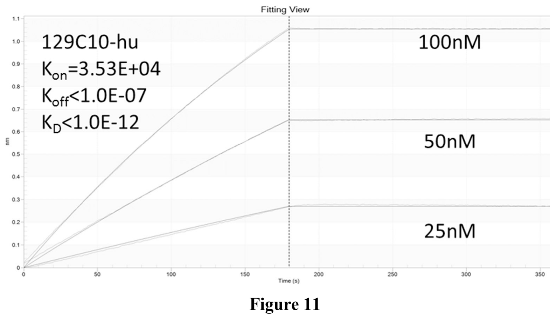

Antibodies to be tested were diluted with ForteBio kinetics buffer (PBS pH 7.4, 0.1% BSA + 0.002% Tween-20) to a concentration of 100 nM. Human MASP-2 protein was diluted with kinetics buffer to get a three-concentration gradient of 100 nM, 50 nM and 25 nM. 0 nM was used as a reference control. Antibodies was immobilized onto Protein A biosensor. The baseline was detected for 60 seconds, and then antibody-MASP-2 association was detected for 180 seconds to get the Kₒₙ factor data. Followed by dissociation in kinetic buffer for 180 seconds to get the K_{off} factor data. The regeneration of biosensors was in a buffer of 10 mM glycine, pH2.0. All the kinetics data were collected at 30°C. Data were acquired by using the ForteBio Octet RED96 and analyzed by using the Octet Data Analysis software. As shown in Table 3, all the tested MASP-2 hybridoma antibodies had a high binding affinity to human MASP-2 with KD values ranging from 10⁻¹⁰ M to 10⁻⁸ M. After humanization, the lead antibody 129C10-hu (129C10-HaLa) kept the highest binding affinity to hMASP-2 at a KD value of <10⁻¹² M (reach the detection limit of ForteBio) (Table 4), with a Kₒₙ value of 3.53E+4 and a K_{off} value of <1.0E-7 (see Figure 11).

**Table 3 Binding Affinity of MASP-2 Hybridoma Antibodies**

| Sample | Affinity | | |
|---|---|---|---|
| | K_{D} (M) | kₒₙ(1/Ms) | k_{dis}(1/s) |
| 129C10H2C1 | 7.46E-10 | 6.40E+04 | 4.78E-05 |
| 160D10 | 2.78E-08 | 3.61E+04 | 1.00E-03 |
| 77C1H1E1 | 1.18E-09 | 6.96E+04 | 8.20E-05 |
| 128D5G3H2 | 1.05E-09 | 8.39E+04 | 8.81E-05 |
| 125D5G1G2 | 1.64E-09 | 5.94E+04 | 9.73E-05 |
| 160F3E1G5 | 1.41E-09 | 7.09E+04 | 1.00E-04 |
| 80C12G2F10 | 4.49E-09 | 9.25E+04 | 4.15E-04 |
| 115H10B3E4 | 7.20E-10 | 7.29E+04 | 5.24E-05 |

**Table 4 Binding Affinity of MASP-2 Humanized or Chimeric Antibodies**

| Sample | K_{D} (M) | kₒₙ(1/Ms) | k_{dis}(1/s) |
|---|---|---|---|
| 129C10HaLa | <1.0E-12 | 3.53E+04 | <1.0E-07 |
| 129C10HbLa | <1.0E-12 | 3.29E+04 | <1.0E-07 |
| 129C10HbLb | <1.0E-12 | 3.29E+04 | <1.0E-07 |
| 129C10HcLb | 1.36E-09 | 3.37E+04 | 4.56E-05 |
| 129C10HaLb | 1. 14E-08 | 2.55E+04 | 2.91E-04 |
| 129C10HdLb | <1.0E-12 | 3.60E+04 | <1.0E-07 |
| 129C10HdLa | 1.71E-08 | 2.49E+04 | 4.25E-04 |
| 129C10HcLa | <1.0E-12 | 3.52E+04 | <1.0E-07 |
| 129C10chiIgG4 | 8.02E-09 | 2.57E+04 | 2.06E-04 |
| 160D10chiIgG4 | 9.84E-10 | 3.40E+04 | 3.34E-05 |
| 125D5chilgG4 | 1.95E-08 | 2.56E+04 | 4.99E-04 |
| OMS721-analog | 7.40E-09 | 4.16E+04 | 3.08E-04 |

### EXAMPLE 9: Binding specificity of 129C10-hu by ELISA

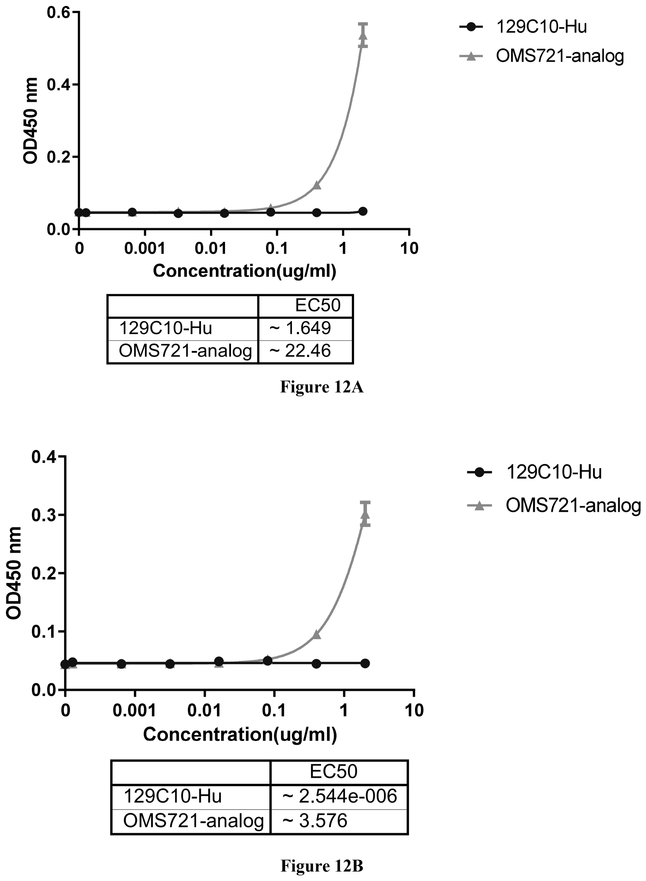

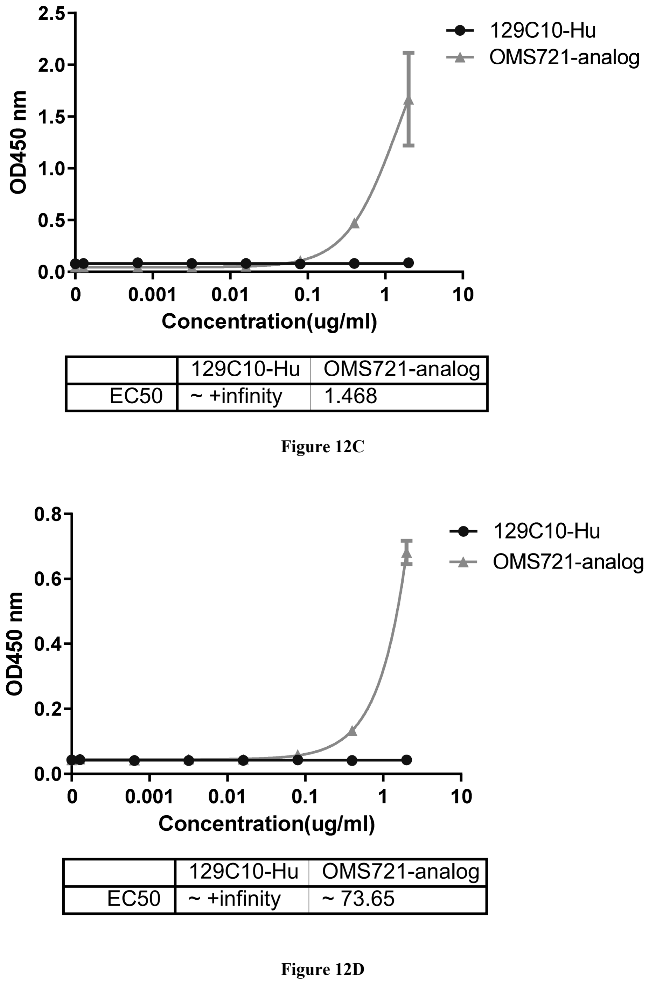

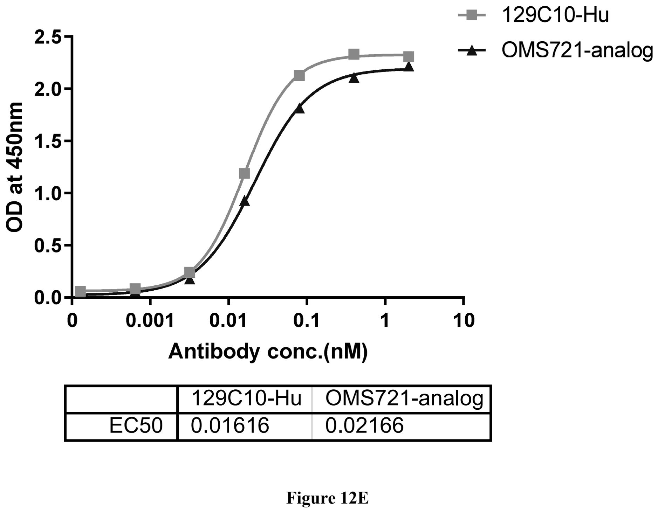

Human C1s /C1r, MASP-1/3 were purchased from R&D, Cusbio etc. Coat Recombinant Human Complement Component C1s /C1r, MASP-1 or MASP-3 (1 mg/ml) at 4°C for overnight; wash three times with wash buffer; add blocking buffer (200 µL/well) at 4°C for 1h; wash three times; add serially diluted Ab at RT for 1h; wash three times; add mouse anti-human IgG4 HRP (1:20000) at RT for 1h; wash three times; detected with tetramethylbenzidine (TMB) for 40min at OD450nm. The EC50 of binding of 129C10-Hu and OMS721-analog to C1s, C1r, MASP1, MASP2 or MASP3 were shown in Figure 12A-12E, respectively. The results showed that 129C10-Hu only bound to human MASP2, but not to human C1s, C1r, MASP1 or MASP3.

### EXAMPLE 10: Cross-reactivity of 129C10-hu by ELISA

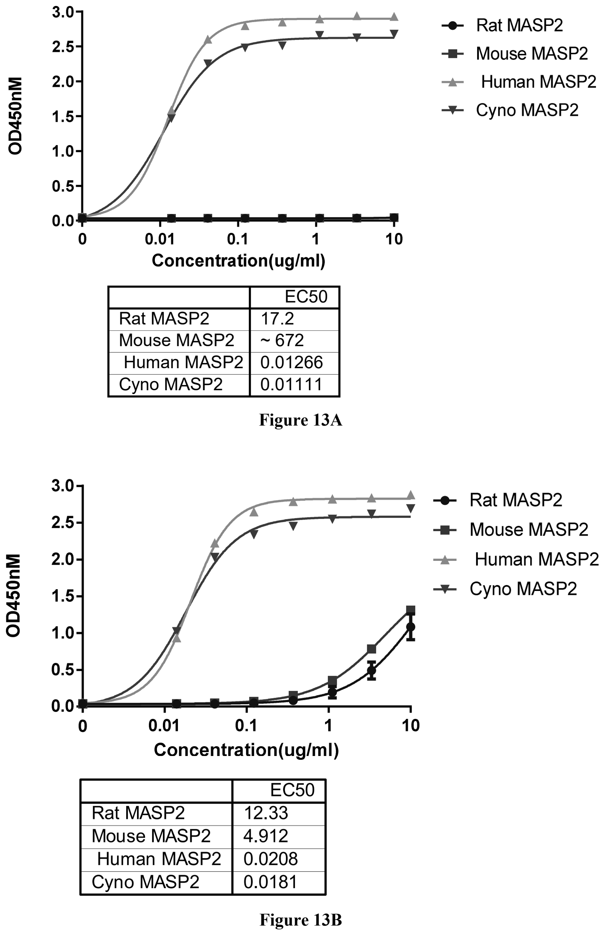

Rat/mouse MASP-2 were ordered from Cusbio company. Human/cyno MASP-2 were generated in house. ELISA assay was performed with following procedure. Coat MASP2 (1 µg/ml) of different species at 4°C for overnight; wash three times with wash buffer; add blocking buffer (200 µL/well) at RT for 2h; wash three times; add serially diluted 129C10-hu or OMS721-analog as a control at RT for 1h; wash three times; add mouse anti-human IgG4 Fc HRP (1:20000) at RT for 1h; wash three times; detected with TMB for 2 min at OD450 nm. The EC50 of binding of 129C10-Hu and OMS721-analog to MASP-2 of different species were shown in Figure 13A and Figure 13B, respectively.

### EXAMPLE 11: Cross-reactivity of 129C10-hu to cynomolgus MASP-2 by using C4 activation assay in cynomolgus serum

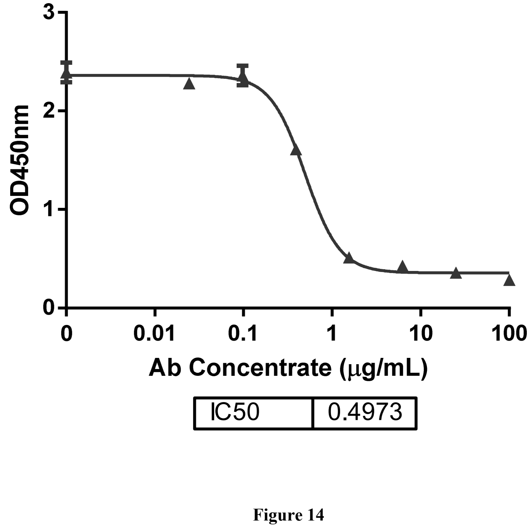

The assay method was same as described in Example 3 except for using cynomolgus serum. 100 µl per well 10 µg/ml mannan was coated onto ELISA plate at 4°C for overnight. Antibodies were incubated with 1% cynomolgus serum for 45 min on ice. After washing and blocking the plate, antibody-serum mixture was added and then incubated at 37°C for 90min. After washing, the deposited activated C4 was detected with an HRP-linked anti-C4c antibody (Quidel-A211). As the result shown in Figure 14, 129C10 blocked cynomolgus C4 activation with an IC₅₀ of 0.4973 µg/ml, suggesting 129C10 could bind with cynomolgus MASP-2 to block its activity.

### EXAMPLE 12: The selectivity of 129C10 in blocking activation of the MB-Lectin complement pathway

There are 3 pathways to initiate complement activation: classical pathway, MB-Lectin (MBL) pathway and alternative pathway. These pathways depend on different molecules for their initiation, while they converge to generate the same set of effector molecules like membrane attack complex (MAC). All three pathways are important parts of innate immunity and play different roles in defending different infections (Noris M, et. Al. 2013. JM.). Next, selectivity of blocking the MBL pathway by 129C10-hu was tested.

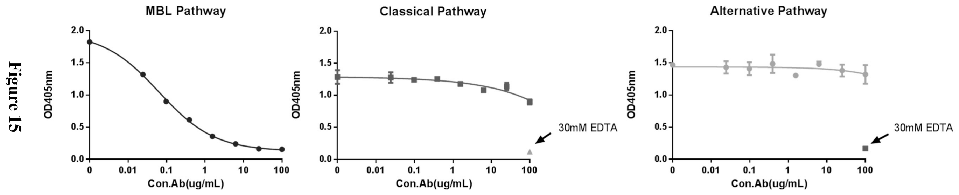

The Wieslab complement system screen kit (IBL America, Cat# COMPL 300 RUO) was used to determine the selectivity of the lead antibody 129C10-hu. The plate was pre-coated with mannan as an initiator for the MBL pathway, IgM as an initiator for classical pathway, and LPS as an initiator for alternative pathway. 129C10-hu was serially diluted with assay buffer containing human serum (Quidel, A113) and then incubated for 45 min on ice. Antibody-serum mixture was added to the plates and incubated at 37°C for 60min. After washing, the deposited MAC was detected with an AP-linked anti-C5b-9 antibody. EDTA was used as a positive control of blocking complement activation. As the results shown in Figure 15a-15C, 129C10-hu only blocked complement activation initialized from the MBL pathway, but not the other two complement pathways, suggesting that 129C10-hu antibody selectively blocked the MBL pathway complement activation.

### EXAMPLE 13: This modification of 129C10-hu to extend half-life by introducing YTE mutation at Fe

Dall'Acqua WF et. al reported that introduction of triple mutation M252Y/S254T/T256E (YTE) into Fc portion of IgG could enhance its binding affinity to FcRn and pro-long its half-life *in vivo* (Dall'Acqua WF, et.al. 2006. JBC). Motavizumab-YTE, the first YTE mutation IgG in human was demonstrated well tolerated and exhibited an extended half-life in a phase I clinical trial (Robbie, G. J., et. al. 2013. AAC).

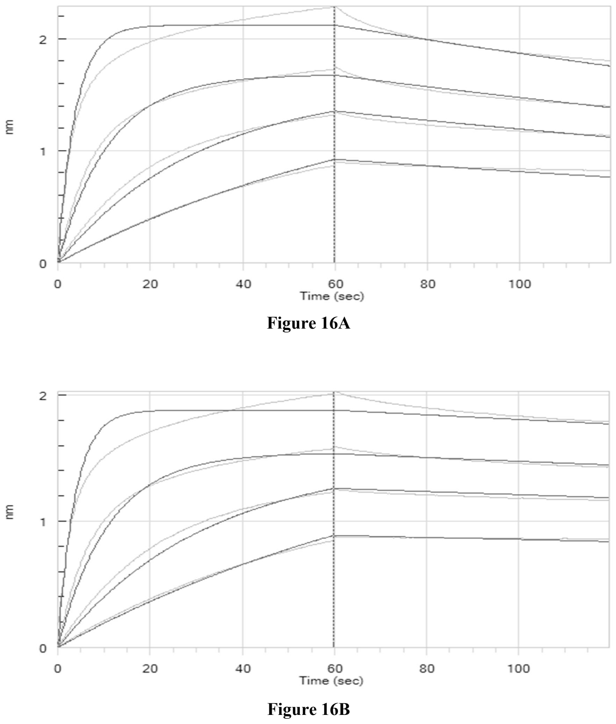

We introduced this M252Y/S254T/T256E (YTE) mutation into 129C10-hu to generate 129C10-hu-YTE. Its binding affinity to FcRn was evaluated with Bio-Layer Interferometry (ForteBio). 129C10-hu or 129C10-hu-YTE was diluted with ForteBio kinetics buffer (PBS pH 7.4, 0.1% BSA + 0.002% Tween-20) to series concentrations of 500 nM, 167 nM, 56 nM, 19 nM and 0 nM. Human FcRn (FCGRT&B2M) protein with His tag was diluted with kinetics buffer to a concentration gradient of 100 nM. FcRn protein was immobilized onto Ni-NTA biosensors. The association and dissociation kinetics were recorded and analyzed. As the results shown in Figure 16A and Figure 16B and Table 5, the YTE mutation increased 129C10-hu binding affinity to human FcRn to 3 folds.

**Table 5 129C10-hu and 129C10-hu-YTE binding affinity to human FcRn**

| **Sample ID** | **KD (M)** | **kon(1/Ms)** | **kdis(1/s)** |
|---|---|---|---|
| 129C 10-hu | 6.10E-09 | 5.20E+05 | 3.17E-03 |
| 129C 10-hu-YTE | 1.88E-09 | 5.36E+05 | 1.01E-03 |

### EXAMPLE 14: Phamacokinetics (PK)/Phamacodynamics (PD) study of MASP-2 antibody 129C10-hu and 129C10-hu-YTE in cynomolgus

2 cynomolgus each group was administrated intravenously with 10 mg/kg 129C10-hu, 129C10-hu-YTE or OMS721-analog. Serum samples were collected at 0, 0.5, 2, 8, 24, 48, 72, 96, 168, 336, 504, 672, 840 hours post infusion. Antibody concentrations in serum and the potency of lectin pathway activation were tested.

Serum concentrations were determined by developed ELISA methods with detection range from 0.625-40 ng/mL. Microplate wells were pre-coated with a human IgG specific anti-IgG antibody [R10z8e6]. After blocking, standard (STD), quality control (QC) samples, matrix blank samples and the test samples were added. After washing, the biotin mouse anti-human IgG4 was added to the microplate wells and followed by Streptavidin labeled with HRP. TMB was added to the microplate wells. The conversion of OD values for QC and test samples into concentration was performed by comparison to a concurrently analyzed standard curve regressed according to a four-parameter logistic model.

For lectin pathway activation potency test, 10 µg/ml mannan was coated onto ELISA plate. Cynomolgus serum samples were diluted to 2% with C4 activation buffer. After washing and blocking of the plate, the diluted serum was added and incubated at 37°C for 90min. After washing, the deposited activated C4 was detected with an HRP-linked anti-C4c antibody (Quidel-A211).

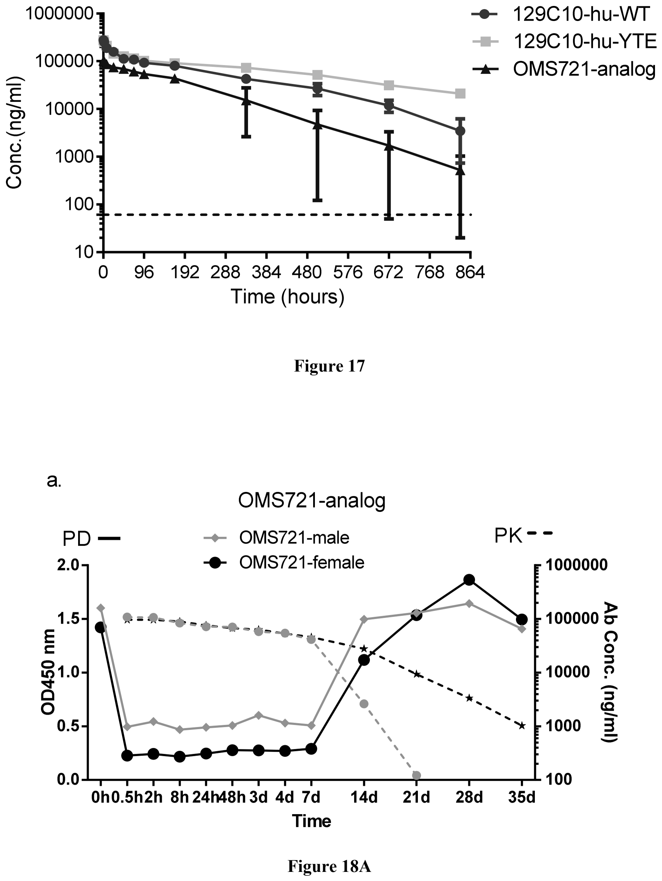

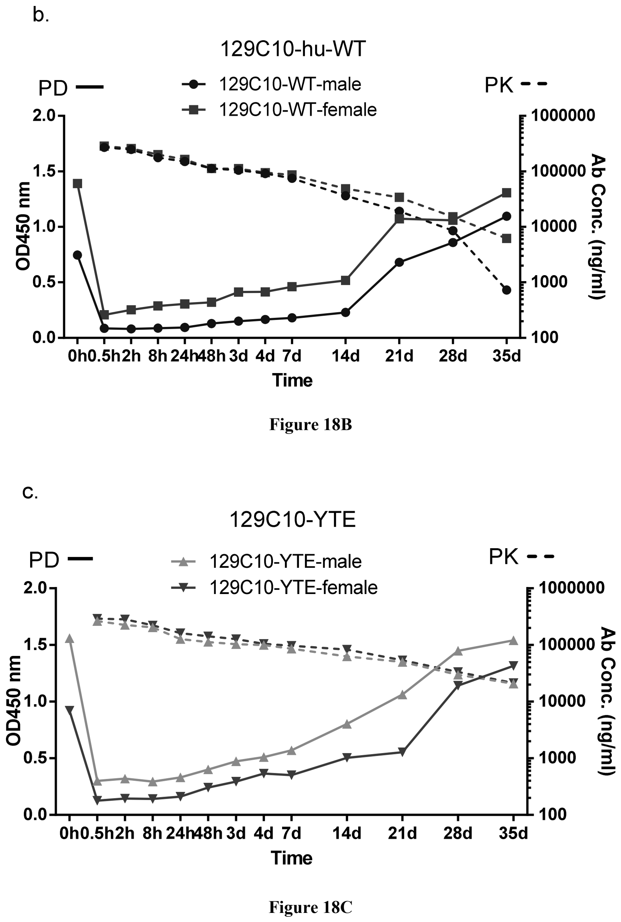

As the PK results shown in Figure 17 and Table 6, half-life of 129C10-hu in cynomolgus is 164.77 hours longer than OMS721-analog (130.152 hours). YTE mutation increased the half-life of 129C10-hu to 274.404 hours. As the PD results shown in Figure 18, the lectin pathway activation potency was inhibited to a basal level after 0.5h of antibody administration. The inhibition effect lasted for 2 weeks in OMS721-analog group, 3 weeks in 129C10-hu group and approximately 4 weeks in 129C10-hu-YTE group.

**Table 6 Data summary of PK test in cynomolgus**

| **PK Parameter** | **Unit** | **129C10-hu 10mpk** | **129C10-hu-YTE 10mpk** | **OMS721-analog 10mpk** |
|---|---|---|---|---|
| t1/2 | h | 164.777 | 274.404 | 130.152 |
| Cmax | ng/ml | 277231.420 | 278092.525 | 102730.925 |
| AUC 0-t | ng/ml*h | 39897468.881 | 56133410.517 | 17892207.152 |
| Cl_obs | ml/day/kg | 5.894 | 3.725 | 13.270 |

In another separate study, male and female cynomolgus monkeys were assigned to five groups of 2 males and 2 females in each group, and dosed with 0 (vehicle control), 15, 20 and 295.8 mg/kg 129C10-hu via subcutaneous injection or 15 mg/kg 129C10-hu via intravenous injection at a volume of 3 mL/kg for 4 weeks (up to 5 doses). The vehicle control article used the drug Formulation Buffer.

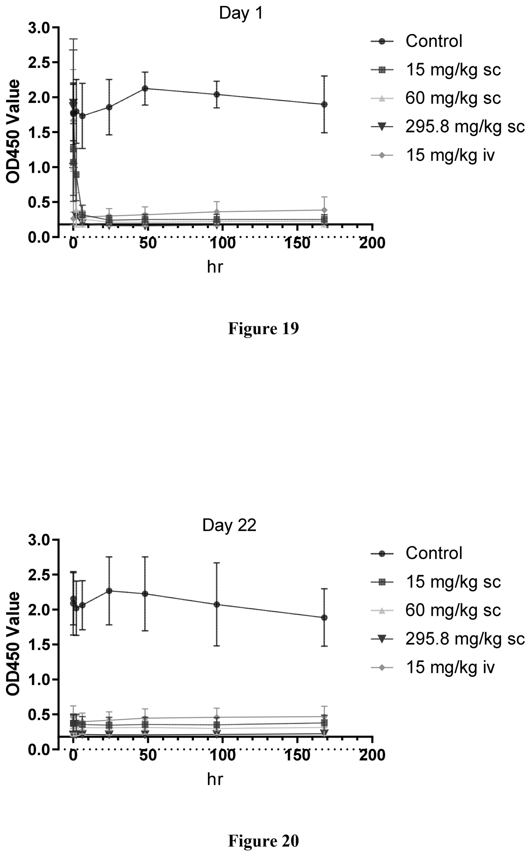

Blood samples were collected predose and at approximately 0.083, 2, 6, 24, 48, 96 and 168 hours post the first (see Figure 19) and fourth doses (see Figure 20), respectively. Complement 4c (C4c) in serum was analyzed with Enzyme-linked immune sorbent assay (ELISA). Briefly, Mannan (Sigma) was diluted to 10 µg/ml with coating buffer. 100 µl of mannan working solution was added to each well of 96 well plates. Incubated the plates overnight at 4°C. Washed the plates three times and then added 200 µl of blocking buffer and incubated for 1h at room temperature. Dilute monkey serum to 2% with C4 activation buffer. Added 100 µl of diluted serum into 96 well plate and incubated at 37°C for 70 min. Washed the plates three times with washing buffer. Added 100 µl of HRP linked anti-C4c antibody (diluted 1:5000 with block buffer) and incubated for 1h at room temperature. Washed the plates three times with washing buffer. Added 100 µl TMB substrate solution into each well of 96 well plates, incubated at room temperature for 5-10 minutes. Added 50 µl stop solution into each well of plates. Read the plates at 450 nm. The optical density (OD) values at 450 nm (OD450) were expressed as individual values and the mean value at each time point of every animal was calculated.

Dose-dependent reduction of serum C4c was observed at ≥ 15 mg/kg following the first and fourth doses, and the reduction appeared from 2 hr post dose in animals dosed via S.C. and from 0.083 hr post dose in animals dosed via I.V., the effect persisted in the whole dosing period with maximum effect at 24-96 hr post first dosing across the 3 dose levels by S.C. or I.V., maximum mean reduction up to 88.4% in males and 92.8% in females were noted at 295.8 mg/kg by S.C. compared with baseline value. In conclusion, 129C10-hu exerted remarkably dose-dependent reduction effect on serum C4c in monkeys following S.C. or I.V. administration once weekly at 15, 60 or 295.8 mg/kg for 5 doses, suggesting serum C4c could be a potential pharmacodynamic marker.

### Example 15: Materials and methods for the antibody formulation research

The protein, namely antibody molecule selected in the formulation study of the present invention was investigated with 129C10-hu-YTE as an example.

The instruments and equipments used in the present invention are shown in Table 7 below, the consumables are shown in Table 8 below, and the reagents are shown in Table 9 below.

**Table 7 Instruments and Equipments**

| **Instrument** | **Vendor** | **Model** |
|---|---|---|
| Water purification system | Millipore | Milli-Q Advantage A10 |
| Analytical balances | Mettler Toledo | ML204T/ 02 |
| Magnetic stirrer | WIGGENS | MIX 6 |
| osmometer | Advanced | OsmoPRO |
| pH meter | Mettler Toledo | S220 |
| UV-Vis spectrophotometer | Thermo Scientific | Nano Drop 2000 |
| Clean bench | AIRTECH | VS-1300L-U |
| -80°C Refrigerator | Haier | DW-86L490J |
| 40°C Stability Chamber | Memmert | HPP400 |
| 25°C Stability Chamber | Memmert | ICH110L |
| 2 to 8°C Refrigerator | Haier | HYC-1378 |
| Clarity detector | Tianda Tianfa | YB-2 |
| Microflow- imaging system | Protein Simple | 368-WPR2 |
| Microplate Reader | PerkinElmer | 2105 Model |
| Dynamic light scattering instrument | WYATT | NanoPro |
| Differential Scanning Calorimetry | TA | Nano DSC |
| Centrifuge | Eppendorf | 5804R |

**Table 8 Consumables**

| **Consumables** | **Vendor** | **Model** |
|---|---|---|
| 0.22 µm PVDF membrane | Millipore | SLGVR33RS |
| 13 mm rubber stopper | West Pharma Packaging | 7002-0664 |
| 13 mm aluminum cap | West Pharma Packaging | 54133001 |
| 20 mm rubber stopper | West Pharma Packaging | 1970-0022 |
| 20 mm aluminum cap | West Pharma Packaging | 5420-3603 |
| 2R Injection vial | Schott Glass Technologies | 1542306 |
| 6R Injection vial | Schott Glass Technologies | 1546216 |
| 5 mL PC vial | Fisher | 3500-05 |

**Table 9 Reagents**

| **Reagents** | **Vendor** | **Grade** | **Catalog number** |
|---|---|---|---|
| Acetic acid | J.T Baker | Multi-Compendial, GMP Manufactured Product, B.P., E.P., J.P., U.S.P | 9526-03 |
| Trihydratesodium acetate | Merck | EMPROVE^{®}bio, PhEur, BP, JP, USP | 1.37012.100 0 |
| L- histidine | Merck | EMPROVE^{®}exp, PhEur, USP, JP | 1.04352.100 0 |
| L-histidine hydrochloride monohydrate | Merck | EMPROVE^{®}exp, PhEur, BP, JP | 1.04354.050 0 |
| Citrate monohydrate | Merck | EMPROVE^{®}exp, Ph Eur, BP, JP, USP. | 1.00242.500 0 |
| Sodium citrate | Merck | EMPROVE^{®}exp, Ph Eur, BP, JP, USP. | 1.06446.100 0 |
| 6N hydrochloric acid | J.T Baker | GMP manufactured Product | 0327-02 |
| Polysorbate 80 | NOF | USP, NF, EP, JP | HX2 |
| Polysorbate 20 | Croda | Super RefinedTM USP, NF, PhEur, JP | SR40606 |
| Sucrose | Pfanstiehl | high purity, low endotoxin, NF,EP,JP,ChP | 36583A |
| Sodium chloride | Merck | EMPROVE^{®}exp, Ph Eur, BP, JP, USP. low in endotoxins suitable for use as excipient | 1.16224 |
| Trehalose | Pfanstiehl | high purity, low endotoxin, NF,EP,JP,ChP | T-104-4 |
| Sorbitol | Merck | EMPROVE^{®}exp, Ph Eur, BP, JP, NF | 1.11597.902 8 |
| Mannitol | Merck | EMPROVE^{®} APIPh Eur, BP, JP, USP | 1.05303.500 0 |
| L-proline | Merck | EMPROVE^{®}exp, Ph Eur, USP | 1.07430.100 0 |
| L-arginine hydrochloride | Merck | EMPROVE^{®}exp, Ph Eur, BP, JP | 1.01544.100 0 |

The process of the present invention involves the following steps.
1. General buffer preparation method: All buffer solutions were prepared using particular acidic and basic ion pairs. The excipients that provide the acidic and basic ion pairs were accurately weighed, about 60% Milli-Q water of the specified buffer volume was added, mixed evenly, and the pH of the solution was measured. If the pH value deviates from the target value, the corresponding ion pairs was used to adjust the pH. The solution was diluted with Milli-Q water to the target weight or volume. Finally, the conductivity, osmolarity and pH of the solution were measured for verification.
2. Sample preparation method: The DS was buffer-exchanged into the formulation with the formulae of interest by dialysis. Alternatively, a high-concentration of excipient and surfactant solution was directly added into a high-concentration of DS, and then DS was diluted into the target concentration. According to the consumables used, there are two kinds of dialysis methods as follows: (1) If the formula of the target formulation is different from the buffer of the DS solution, the DS was buffer-exchanged into the target formulation by dialysis (a surfactant cannot be introduced via dialysis). The sample was placed into a SnakeSkin^{®} dialysis bag, sealed, and placed into a buffer solution having a volume approximately 100 to 200 times the sample volume. Dialysis was carried out for three times with stirring at 200 rpm, with duration times of 4 hours, 4 hours, and overnight, respectively. Then, an appropriate amount of surfactant and other excipient stock solutions were moved into the sample solution, and the sample solution was diluted to the target concentration. (2) Dialysis was also performed using the Slide-A-Lyzer cassette. A 3-12 mL sample was loaded into the cassette using a syringe with needle, and then immersed into the target buffer with a volume of approximately 100 to 200 times the sample volume. The cassette was dialyzed for 3 times at room temperature with stirring at 300 rpm/min, with duration times of 4 hours, 4 hours, and overnight, respectively.
3. Appearance: The appearance was examined against black background and white background by a YB-2 clarity detector. The clarity, the color and the visible particle were reported.
4. pH: The pH of a sample was measured by a SevenCompact pH meter equipped with an Inlab^{®} Micro-Pro-ISM electrode. The pH meter is calibrated prior to use.
5. Protein concentration: Protein concentration was determined by measuring the absorbance at 280 nm using a Nano Drop 2000 spectrophotometer. The extinction coefficient (E1%) used was 1.422 L/g/cm throughout the study. Each sample was measured twice at a loading volume of 2.0 µL. The mean concentration is reported.
6. Dynamic Light Scattering (DLS) instrument: Protein size distribution was determined by DLS using the following measurement conditions: acquisition time: 5 s, acquisition per measurement: 20 times, temperature: 25°C.
6.1. Determination of *k_{D}* value by DLS: The sample was diluted with the corresponding buffer to a concentration of 1, 2, 4, 8, 12, 16, 20 mg/mL, and the *k_{D}* value was analyzes by DLS, the acquisition time: 5 s, acquisition per measurement: 20 times, temperature: 25°C.
6.2. Determination of viscosity by DLS: The tested sample was mixed with standard particles with a diameter of 100 nm. The final concentration of the standard particles is 1%. The apparent particle size of the standard particles was measured by DLS. The acquisition time: 5 s, acquisition per measurement: 20 times, temperature: 25°C. The collected data was analyzed using DYNAMICS software, and the viscosity of the system was calculated.
6.3. Determination of Tₒₙₛₑₜ value by DLS: The transition temperature at which configuration change or aggregation occur to protein during heating process so that the diameter changes was measured as Tₒₙₛₑₜ. The sample was diluted to 4 mg/mL with the corresponding buffer, 100 µL was taken and added to a 96-well plate, centrifuged at 3000 rpm for 5 minutes, and 10 µL of silicone oil was added to cover the sample, and centrifuged at 3000 rpm for 5 minutes. Linear heating at 1°C/min in the range of 25-80°C was performed on the instrument, and a single sample was measured for 8 times, with each acquisition time of 3 seconds. The particle size and temperature data were collected, and plotted, and the Tₒₙₛₑₜ of the sample was calculated using a line-line intersection function provided by the official software.

7. Size Exclusion Chromatography (SEC): Protein aggregation was determined by SEC using a Waters H Class ultra-high performance liquid chromatography and a Waters BEH Protein SEC column (150 × 4.6 mm, 1.7µm). The mobile phase is 50 mM phosphate buffer, 300 mM NaCl, pH 6.8 ± 0.1. The flow rate is 0.4 mL/min. Samples were diluted to 2 mg/mL, the detection volume is 10 µL, and the detection wavelength is 280 nm.
8. Cation exchange chromatography (CEX): Charge heterogeneity of protein was determined by CEX-HPLC. The Liquid Chromatography system is Agilent 1260 Infinity II and the chromatographic column is Thermo Propac WCX-10 BioLC column (4×250 mm, 10 µm). After mixing evenly, the sample was centrifuged, and the supernatant was transferred. Then, the sample was diluted to 2.0 mg/mL with mobile phase A. The chromatographic conditions are shown in Table 10 below.

**Table 10 Operating conditions of cation chromatography**

| Items | Parameters | | |
|---|---|---|---|
| Wavelength | 280 nm | | |
| Column temperature | 30 ± 1 °C | | |
| Flow rate | 0.6 mL/min | | |
| Loading volume | 20 µL | | |
| Mobile phase A | 0.5×CX-1 pH gradient soluiton A pH 5.5±0.2 | | |
| Mobile phase B | 1×CX-1 pH gradient soluiton B, 20 mM NaCl pH 10.1±0.2 | | |
| Gradient | Time (min) | A (%) | B (%) |
| | 0.00 | 90 | 10 |
| | 40.00 | 50 | 50 |
| | 40.10 | 0 | 100 |
| | 45.00 | 0 | 100 |
| | 45.10 | 90 | 10 |
| | 55.00 | 90 | 10 |

9. Non-reduced Capillary electrophoresis (NR-CE-SDS): Protein fragmentation was determined by NR CE-SDS. The reference standard or test samples were diluted to 4 mg/mL with phosphate-citrate buffer, 25 µL of which was then mixed with 75 µL SDS sample buffer and 5 µL NEM (100 mM N-ethylmaleimide) by vortex for denaturation. After centrifugation, the denatured sample was incubated at 70±2°C for 10±2 min, cooled at room temperature, and then centrifuged again. Separation of sample was performed on PA800 plus using a SDS separation gel kit and an uncoated capillary. The high-speed separation mode was used, and the effective separation length of the capillary was 10 cm.
10. Protein solution turbidity test (OD405): The turbidity of sample was tested using a Envision multifunctional microplate reader. The buffer solution was used as a blank control, and the difference between the sample and the blank control is the OD value of the sample at 405 nm.
11. Microflow- imaging system (MFI): The number of sub-visible particles in the formulation was measured by MFI 5200 purchased from Protein Simple. 1 mL sample was taken for injection using a pipette without dilution. The number of particles per mL in the diameter range of ≥ 2 µm, ≥ 10 µm, and ≥ 25 µm was reported.
12. Whole column imaged capillary isoelectric focusing electrophoresis (icIEF): The sample was mixed with an appropriate mixture containing pl markers and amphoteric electrolytes, and then injected into the FC-coated capillary in the iCE3 system through an automatic injector. After a separation step under a high-voltage, the different charge isomers of the protein migrate to their corresponding isoelectric point positions. Then, the positions determined after separation were captured by a UV detector (UV absorption spectrum was collected by a CCD camera with whole column detection at UV280 nm). Using an iCE system software, the isoelectric point of the sample peak was corrected by the focusing position of the sample peak and the corresponding pl marker. The subsequent data analysis was performed in the Empower software. The chromatographic conditions are shown in Table 11 below.

**Table 11 Chromatographic operating conditions**

| Items | Parameters |
|---|---|
| Temperature of atuomatic injector | 15°C |
| Focusing voltage and time | First focousing stage: 1500 volts, 1 minute |
| Focusing voltage and time | Second focousing stage: 3000 volts, 8 minutes |
| Injection time | Injection time may vary according to TTM test results |

13. Binding activity: Anti-MASP-2 antibody C4 activation inhibition assay was used in this method. In the experiment, the coating material is mannosan, the detection antibody is Complement C4c Antibody (HRP), and the substrate of the enzymatic reaction is TMB. The mannosan coating was adsorbed on the ELISA solid phase carrier adsorption plate. After washing and blocking, a mixture of test sample and human serum complements was added for binding. The higher the sample concentration, the stronger the effect of inhibiting the cleavage of C4 by MASP-2 in the serum would be, and the lower the C4b content, and the less C4c would be obtained by shearing. After washing followed by incubation, the detection antibody was added, and then incubated. The unbound detection antibody was removed by washing. The substrate was added for color development, finally, the reaction stopping solution was added. The absorbance value was read on the microplate reader at the detection wavelength of 450nm/650nm. Four-parameter fitting was performed using the log of the anti-MASP-2 antibody protein concentration as the horizontal axis and the OD₄₅₀ₙₘ₋₆₅₀ₙₘ as the vertical axis to obtain the IC₅₀ value of the sample and the standard. The final results were reported as relative activity, using the calculation formula of (IC₅₀ of the standard)/(IC₅₀ of the sample).

### Example 16: screening experiment for auxiliary materials

### 1. Experiment Design

The formulae of the sample, the placement conditions of the stability experiment and the detection method are as shown in Table 12. The UFDF sample was buffer-exchanged into the target formulae by dialysis. Then, the sample was diluted to 30 mg/mL using the corresponding auxiliary material stock solution. Finally, the sample was obtained by filteration using 0.22 µm PVDF membrane.

**Table 12 Experiment design in screening experiment for auxiliary material**

| Formulae | Protei n Conc. | Auxiliary material | Buffer system | Conditions of the stability experiment and test items |
|---|---|---|---|---|
| F1(Arg) | 30 mg/m L | 150mM arginine (Arg) | 20 mM Acetate, pH 5.5, 0.05% PS 80 | T0: appearance, Protein Conc., pH; OD405, DLS; SEC, NR CE-SDS, CEX; MFI |
| F2 (NaCl) | | 150mM Sodium chloride (NaCl) | | |
| | | | | 40°C (3,7,14 days): Protein Conc., OD405, pH; DLS; SEC, NR CE-SDS, CEX |
| F3 (Sor) | | 5% sorbitol (Sor) | | |
| F4 (Suc) | | 9% sucrose (Suc) | | Freezing and thawing (1,3,5 time(s)): |
| F5 (Tre) | | 9% trehalose (Tre) | | Protein Conc., OD405, pH, DLS; MFI |
| | | | | Stirring (100 rpm 2,6 hours): appearance, Protein Conc., pH; OD405, DLS; CEX |
| | | | | Shaking (1,3,5 day(s)): appearance, Protein Conc., OD405, CEX, NR CE-SDS |

### 2. Experiment

### 2.1. Results of experiment under high temperature of 40°C

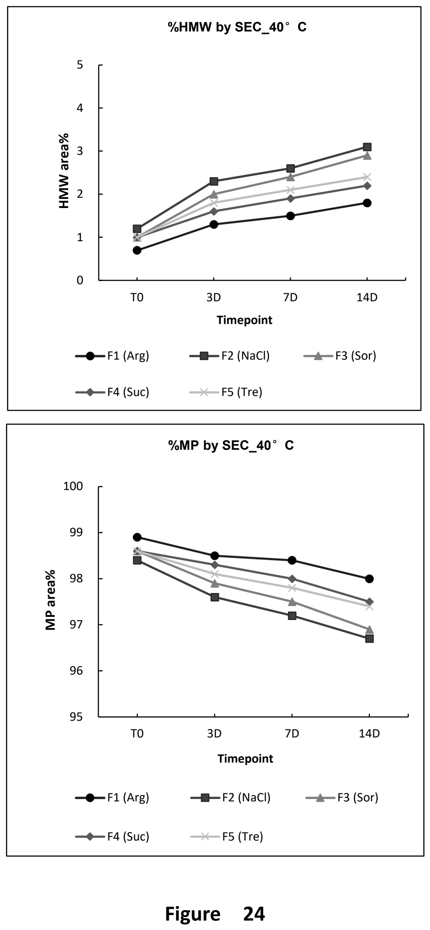

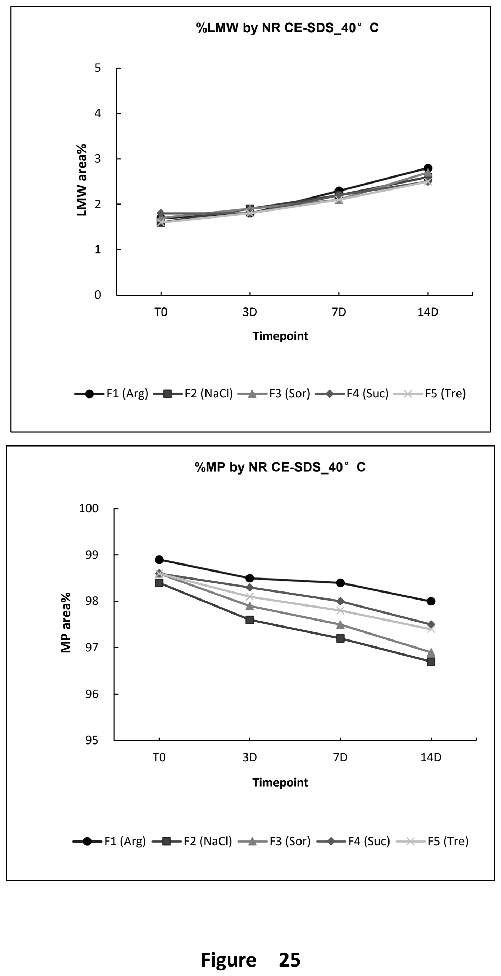

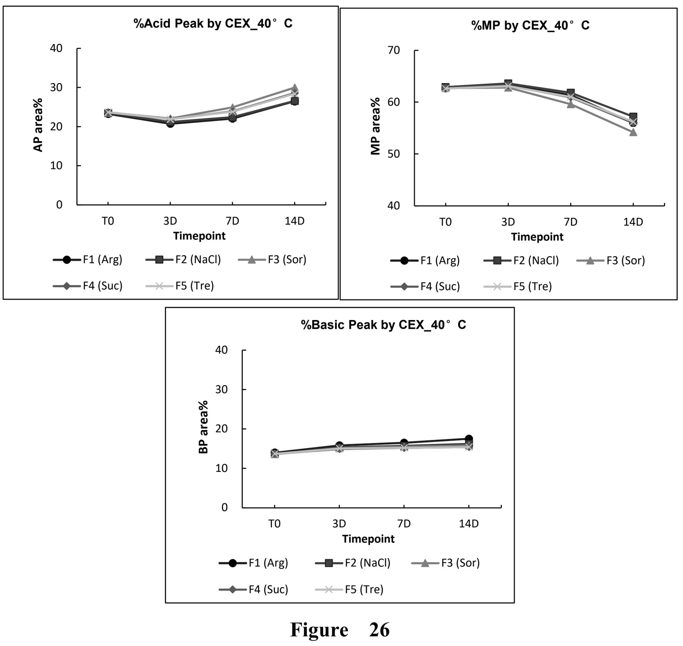

After being placed at 40°C for 14 days, no significant difference was observed among 5 formulae in terms of diameter, concentration, pH; regarding the purity, a decrease in purity was observed in all formulae. In which, F2 (NaCl) and F3 (Sor) have the maximum of SEC main peak, and CEX main peak of F3 (Sor) exhibited the largest decrease. The sepecific degradation trends are as shown in Figure 24, Figure 25, Figure 26.

### 2.2. Results of experiment under shaking

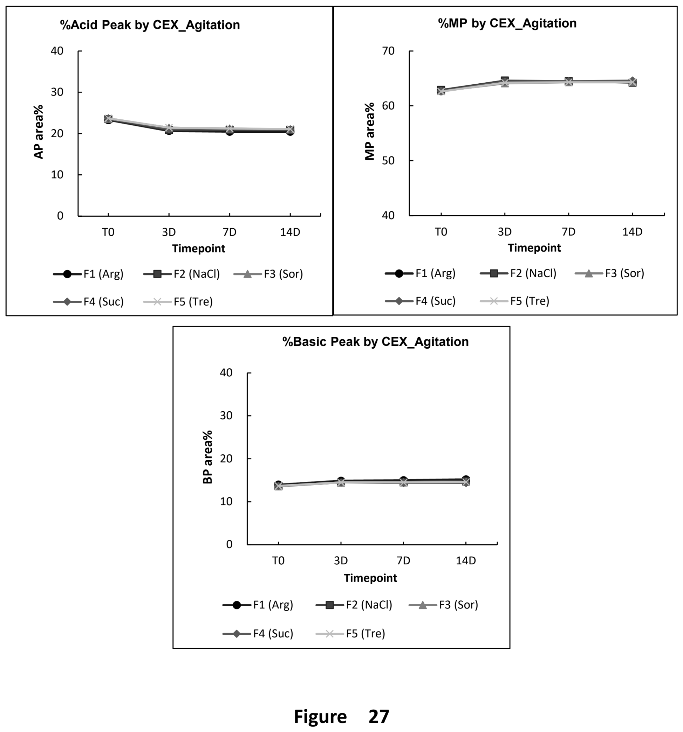

CEX results under shaking of each formula in the auxiliary material screening are as shown in Figure 27. After shaking for 1 day, 3 days, 5 days, no significant changes in appearance, concentration, OD405 and purity were observed in 5 formulae.

### 2.3. Results of experiment under stirring

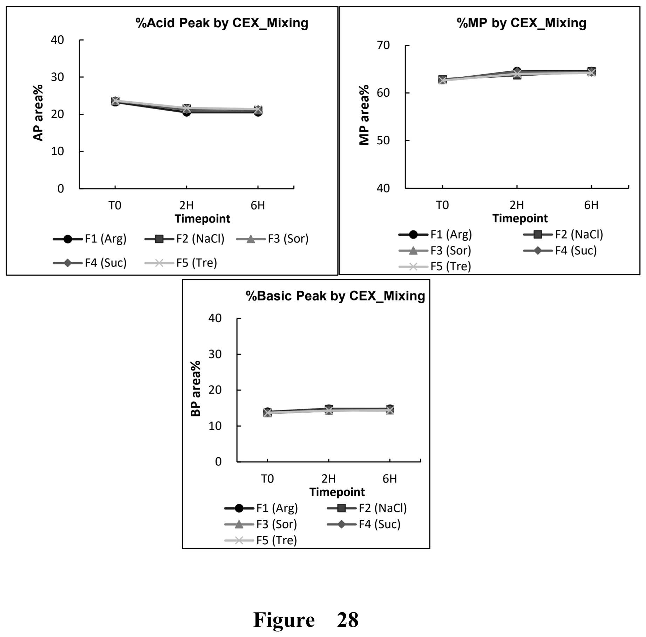

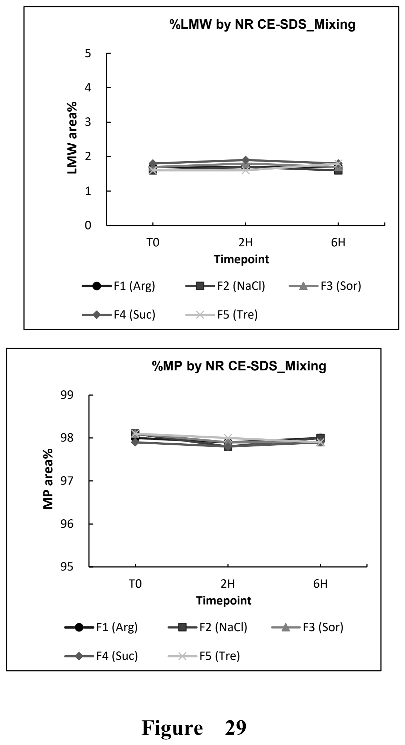

The appearance of all 5 formulae changed from opalescent to turbid, F4 (Suc) remained opalescent after stirring for 2 hours, and the turbidity was observed in other formulae after stirring for 2 hours. Except for F1 (Arg), an increase in OD405 was observed in all of the other formulae. No significant changes in concentration and pH were observed in any formula. No significant change in SEC purity was observed in any of the 5 formulae after stirring for 2 hours and 6 hours. CEX, NR CE-SDS results under stirring of each formula in the auxiliary material screening are as shown in Figure 28, Figure 29.

### 2.4. Freezing and thawing experiment results

After freezing and thawing for 5 cycles, no significant changes in sample concentration, diameter, OD405 and pH were observed in any of the 5 formulae. Regarding the insoluble particles, a significant increase in the concentration of insoluble particles was observed in F2 (NaCl). MFI results under freezing and thawing of each formula in the auxiliary material screening are as shown in Table 13.

**Table 13 MFI results_freezing and thawing**

| **Formula** | **2-10 µm** | | | | **10-25 µm** | | | | **≥25 µm** | | | |
|---|---|---|---|---|---|---|---|---|---|---|---|---|
| | T0 | 1 cycl e | 3 cycl es | 5 cycl es | T0 | 1 cycle | 3 cycle s | 5 cycle s | T0 | 1 cycle | 3 cyc les | 5 cycles |
| F1(Arg) | 4.5 | 11.3 | 15.9 | 19.3 | 46 | 4433 | 3157 | 3092 | 8 | 11 | 8 | 8 |
| F2 (NaCl) | 293. 1 | 218. 9 | 364. 5 | 440. 1 | 351 6 | 6158 8 | 3718 7 | 3230 3 | 0 | 344 | 42 | 15 |
| F3 (Sor) | 3.4 | 0.5 | 0.9 | 1.5 | 250 | 4 | 23 | 665 | 12 7 | 0 | 4 | 31 |
| F4 (Suc) | 2.8 | 0.5 | 2.0 | 187. 3 | 8 | 0 | 69 | 3719 6 | 0 | 0 | 0 | 4252 |
| F5 (Tre) | 1.1 | 0.2 | 0.5 | 1.7 | 27 | 0 | 8 | 76 | 4 | 0 | 0 | 8 |

### Conclusion

With respect to F2 (NaCl) and F3 (Sor), a greater degree of decreases in SEC main peak and CEX main peak at 40°C were observed, compared to other formulae, suggesting the two were significantly inferior to other formulae. Thus, the two were excluded. In summary, there was no significant difference among arginine, sucrose and trehalose, and all of them satisfied the test requirements. Taking into comprehensive consideration the extensiveness of the application of candidate auxiliary materials in biological formulations and the cost, sucrose F4 (Suc) was therefore selected for the subsequent experiments.

### Example 17: screening experiment for surfactant

### 1. Experiment Design

The formulae of the sample, the placement conditions of the stability experiment and the detection method are as shown in Table 14. The UFDF sample was diluted to 50 mg/mL using the corresponding auxiliary material stock solution. Finally, the sample was obtained by filteration using 0.22 µm PVDF membrane. 1 mL sample was taken to a 2R injection vial for the 40°C-experiment, 2 mL sample was taken into a 6R injection vial for experiment under stirring, 2 mL sample was taken into a 2R injection vial for experiment under shaking, respectively.

**Table 14 Experiment design in screening experiment for surfactant**

| Formulae of the formulation s | Protein Conc. | Surfactant | Buffer system | Conditions of the stability experiment and test items |
|---|---|---|---|---|
| F6 (T2) | 30 mg/mL | 0.025% polysorbate 80 (PS80) | 20 mM Acetate pH 5.5, 9% sucrose | T0: appearance, Protein Conc., pH; OD405, DLS; SEC, NR CE-SDS, CEX 40°C(3,7,14 days): appearance, Protein Conc., pH, OD405 DLS; SEC, NR CE-SDS, CEX |
| F7 (T5) | | 0.05% polysorbate 80 (PS80) | | |
| F8 (T10) | | 0.1% polysorbate 80 (PS80) | | |
| F9 (W2) | | 0.025% polysorbate 20 (PS20) | | Stirring (100 rpm for 2, 6 hours): appearance, Protein Conc., pH; OD405, DLS; NR CE-SDS, CEX |
| F10 (W5) | | 0.05% polysorbate 20 (PS20) | | Shaking (200rpm 1,3,5 day(s): appearance, Protein Conc., OD405, CEX, NR CE-SDS |
| F11 (W10) | | 0.1% polysorbate 20 (PS20) | | |

### 2. Experiment

### 2.1. Results of experiment under a high temperature of 40°C

### (1) Appearance, diameter, Conc., pH and OD405

As shown in Table 15, no changes in appearance, diameter, concentration, pH and OD405 were observed in any of the 6 formulae after treatment at 40°C.

**Table 15 Appearance, diameter, concentration and OD405 in screening experiment for surfactant _ results of experiment under high temperature**

| **Formula e** | **Appearance** | | **Concentration (mg/ml)** | | **OD405** | | **PH** | | **Diameter (nm)** | |
|---|---|---|---|---|---|---|---|---|---|---|
| | T0 | 14 days | T0 | 14 days | T0 | 14 days | T0 | 14 days | T0 | 14 days |
| F6 (T2) | SOC | SOC | 31.5 | 31.6 | 0.07 | 0.07 | 5.7 | 5.6 | 7.7 | 8.0 |
| F7(T5) | SOC | SOC | 31.4 | 31.6 | 0.07 | 0.07 | 5.7 | 5.6 | 7.8 | 8.1 |
| F8 (T10) | SOC | SOC | 31.6 | 31.8 | 0.07 | 0.07 | 5.7 | 5.6 | 7.7 | 8.1 |
| F9 (W2) | SOC | SOC | 31.5 | 31.8 | 0.07 | 0.08 | 5.7 | 5.6 | 7.8 | 8.8 |
| F10 (W5) | SOC | SOC | 31.5 | 31.6 | 0.07 | 0.08 | 5.7 | 5.6 | 7.7 | 8.0 |
| F11(W10 ) | SOC | SOC | 31.7 | 31.6 | 0.07 | 0.08 | 5.7 | 5.6 | 7.7 | 8.2 |

| | | | | | | | | | | |
|---|---|---|---|---|---|---|---|---|---|---|
| SOC= slightly opalescent | | | | | | | | | | |

### (2) SEC

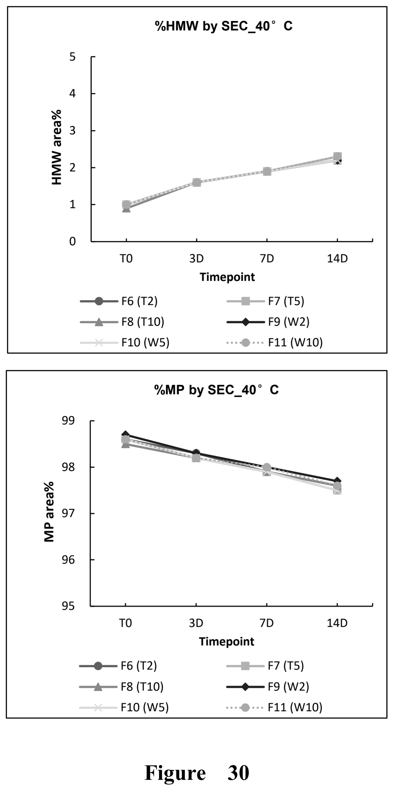

As shown in Table 16 and Figure 30, a slight decrease in SEC main peak was observed in all samples after treatment at 40°C, and there was no significant difference among the 6 formulae.

**Table 16 SEC results in screening experiment for surfactant_experiment under high temperature**

| **Formulae** | **HMW%** | | | | **MP%** | | | | **LMW%** | | | |
|---|---|---|---|---|---|---|---|---|---|---|---|---|
| | T0 | 3 days | 7 days | 14 days | T0 | 3 days | 7 days | 14 days | T0 | 3 days | 7 days | 14 days |
| F6 (T2) | 1.0 | 1.6 | 1.9 | 2.2 | 98.6 | 98.3 | 97.9 | 97.6 | 0.4 | 0.2 | 0.2 | 0.2 |
| F7(T5) | 1.0 | 1.6 | 1.9 | 2.3 | 98.6 | 98.2 | 97.9 | 97.5 | 0.5 | 0.2 | 0.1 | 0.2 |
| F8 (T10) | 0.9 | 1.6 | 1.9 | 2.2 | 98.5 | 98.2 | 97.9 | 97.6 | 0.6 | 0.1 | 0.1 | 0.2 |
| F9 (W2) | 1.0 | 1.6 | 1.9 | 2.2 | 98.7 | 98.3 | 98.0 | 97.7 | 0.4 | 0.1 | 0.1 | 0.2 |
| F10 (W5) | 1.0 | 1.6 | 1.9 | 2.2 | 98.6 | 98.2 | 97.9 | 97.5 | 0.4 | 0.2 | 0.2 | 0.2 |
| F11(W10) | 1.0 | 1.6 | 1.9 | 2.3 | 98.6 | 98.2 | 98.0 | 97.6 | 0.4 | 0.1 | 0.1 | 0.2 |

### (3) NR CE-SDS

As shown in Table 17, a descrease in NR CE-SDS MP% was observed in all formulae after treatment at 40°C, but the degrees of decrease were not significant, and were all within an acceptable range.

**Table 17 NR CE-SDS results in screening experiment for surfactant _experiment under high temperature**

| **formulae** | **LMW%** | | | | **MP%** | | | |
|---|---|---|---|---|---|---|---|---|
| | T0 | 3D | 7D | 14D | T0 | 3D | 7D | 14D |
| F6 (T2) | 1.7 | 1.8 | 2.1 | 2.5 | 98.0 | 97.4 | 97.1 | 96.4 |
| F7(T5) | 1.5 | 1.8 | 2.0 | 2.5 | 98.3 | 97.6 | 97.2 | 96.3 |
| F8 (T10) | 1.7 | 1.9 | 2.0 | 2.4 | 98.1 | 97.3 | 97.2 | 96.5 |
| F9 (W2) | 1.6 | 1.9 | 2.0 | 2.5 | 98.1 | 97.3 | 97.2 | 96.3 |
| F10 (W5) | 1.7 | 1.9 | 2.0 | 2.4 | 98.0 | 97.4 | 97.2 | 96.3 |
| F11(W10) | 1.6 | 1.9 | 2.1 | 2.4 | 98.0 | 97.4 | 97.1 | 96.5 |

### (4) CEX

As shown in Table 18, a medium decrease in main peak MP% was observed in all formulae after treatment at 40°C, which is within an acceptable range, and there was no significant difference in CEX of each formula.

**Table 18 CEX results_experiment under high temperature**

| **Formul ae** | **Acidic peak %** | | | | **MP%** | | | | **Basic peak %** | | | |
|---|---|---|---|---|---|---|---|---|---|---|---|---|
| | T0 | 3D | 7D | 14 D | T0 | 3D | 7D | 14 D | T0 | 3D | 7D | 14 D |
| F6 (T2) | 23.7 | 21. 7 | 24. 0 | 28. 5 | 62.7 | 63. 4 | 60. 7 | 56. 2 | 13. 6 | 14. 9 | 15. 3 | 15. 3 |
| F7(T5) | 23.6 | 22. 2 | 24. 0 | 29. 1 | 62.8 | 63. 0 | 60. 7 | 55. 5 | 13. 6 | 14. 9 | 15. 2 | 15. 4 |
| F8 (T10) | 23.9 | 21. 9 | 24. 1 | 29. 0 | 62.5 | 63. 1 | 60. 7 | 55. 7 | 13. 6 | 15. 0 | 15. 2 | 15. 3 |
| F9 (W2) | 23.7 | 21. 9 | 24. 1 | 28. 7 | 62.7 | 63. 2 | 60. 8 | 56. 0 | 13. 7 | 14. 9 | 15. 1 | 15. 3 |
| F10 (W5) | 23.7 | 22. 0 | 24. 2 | 29. 1 | 62.7 | 63. 1 | 60. 5 | 55. 7 | 13. 6 | 14. 9 | 15. 3 | 15. 2 |
| F11(W1 0) | 23.7 | 24. 8 | 24. 1 | 28. 6 | 62.7 | 60. 6 | 60. 8 | 56. 0 | 13. 6 | 14. 6 | 15. 2 | 15. 4 |

### 2.2. Results of experiment under stirring

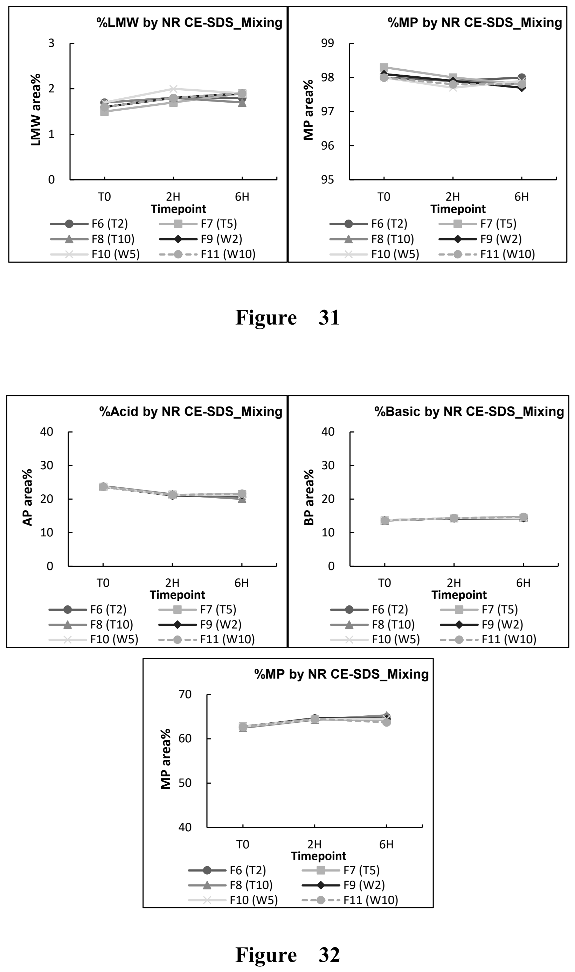

The stability results of each formula under stirring at 100 rpm in surfactant screening are as shown in Table 19, Figure 31, Figure 32. After stirring at 100 rpm for 6 hours, the appearance of F6(T2), F7 (T5), F9 (W2) and F10 (W5) changed from opalescent to turbid. In all formulae, an increase in OD405 was observed, no significant changes in concentration or pH were observed. After stirring for 2 hours, 6 hours, no significant change in purity was observed in any of the 6 formulae.

**Table 19 Appearance, concentration, OD405, PH, diameter results_experiment under stirring**

| **Formula e** | **Appearance** | | | **Concentration (mg/ml)** | | | **OD405** | | | **PH** | | | **Diameter (nm)** | | |
|---|---|---|---|---|---|---|---|---|---|---|---|---|---|---|---|
| | T0 | 2H | 6H | T0 | 2H | 6H | T0 | 2H | 6H | T 0 | 2 H | 6 H | T 0 | 2 H | 6 H |
| F6 (T2) | SOC | SOC | Turbi d | 31.5 | 31.6 | 31.7 | 0.07 | 0.0 9 | 0.0 9 | 5. 7 | N T | 5. 6 | 7. 7 | 8. 2 | 8. 1 |
| F7(T5) | SOC | SOC | Turbi d | 31.4 | 31.6 | 31.6 | 0.07 | 0.0 9 | 0.1 5 | 5. 7 | N T | 5. 6 | 7. 8 | 7. 8 | 6. 0 |
| F8 (T10) | SOC | SOC | SOC | 31.6 | 31.6 | 31.7 | 0.07 | 0.1 1 | 0.0 8 | 5. 7 | N T | 5. 6 | 7. 7 | 8. 9 | 7. 7 |
| F9 (W2) | SOC | SOC | Turbi d | 31.5 | 31.6 | 31.5 | 0.07 | 0.0 8 | 0.1 2 | 5. 7 | N T | 5. 6 | 7. 8 | 7. 9 | 8. 0 |
| F10 (W5) | SOC | SOC | Turbi d | 31.5 | 31.6 | 31.9 | 0.07 | 0.0 8 | 0.1 3 | 5. 7 | N T | 5. 6 | 7. 7 | 8. 7 | 8. 1 |
| F11(W1 0) | SOC | SOC | SOC | 31.7 | 31.7 | 31.5 | 0.07 | 0.0 8 | 0.0 9 | 5. 7 | N T | 5. 6 | 7. 7 | 7. 9 | 8. 1 |

| | | | | | | | | | | | | | | | |
|---|---|---|---|---|---|---|---|---|---|---|---|---|---|---|---|
| SOC= slightly opalescent, Turbid= turbid | | | | | | | | | | | | | | | |

### 2.3. Results of Experiment under shaking

### (1) Appearance, concentration, OD405

As shown in Table 20, no significant changes in appearance, concentration and OD405 were observed in each formula after shaking at 200 rpm for 5 days.

**Table 20 Appearance, concentration, OD405 results_experiment under shaking**

| **Formula e** | **Appearance** | | | | **Concentration (mg/ml)** | | | | **OD405** | | | |
|---|---|---|---|---|---|---|---|---|---|---|---|---|
| | T0 | 1D | 3D | 5D | T0 | 1D | 3D | 5D | T0 | 1D | 3D | 5D |
| F6 (T2) | SOC | SOC | SOC | SOC | 31.5 | 31.6 | 31.5 | 31.7 | 0.0 7 | 0.08 | 0.07 | 0.07 |
| F7(T5) | SOC | SOC | SOC | SOC | 31.4 | 31.7 | 31.5 | 31.6 | 0.0 7 | 0.08 | 0.07 | 0.07 |
| F8 (T10) | SOC | SOC | SOC | SOC | 31.6 | 31.7 | 31.6 | 31.7 | 0.0 7 | 0.08 | 0.07 | 0.07 |
| F9 (W2) | SOC | SOC | SOC | SOC | 31.5 | 31.8 | 31.7 | 31.6 | 0.0 7 | 0.07 | 0.07 | 0.08 |
| F10 (W5) | SOC | SOC | SOC | SOC | 31.5 | 31.6 | 31.5 | 31.6 | 0.0 7 | 0.08 | 0.08 | 0.07 |
| F11(W1 0) | SOC | SOC | SOC | SOC | 31.7 | 31.5 | 31.6 | 31.5 | 0.0 7 | 0.08 | 0.07 | 0.07 |

| | | | | | | | | | | | | |
|---|---|---|---|---|---|---|---|---|---|---|---|---|
| SOC=slightly opalescent | | | | | | | | | | | | |

### (2) NR CE-SDS

As shown in Table 21, there was no significant difference in change of NR CE-SDS main peak between all formulae after shaking.

**Table 21 NR CE-SDS results_experiment under shaking**

| **formulae** | **LMW%** | | | | **MP%** | | | |
|---|---|---|---|---|---|---|---|---|
| | T0 | 1D | 3D | 5D | T0 | 1D | 3D | 5D |
| F6 (T2) | 1.7 | 2.0 | 1.9 | 1.9 | 98.0 | 97.7 | 97.7 | 97.6 |
| F7(T5) | 1.5 | 1.9 | 1.8 | 1.8 | 98.3 | 97.8 | 97.9 | 97.7 |
| F8 (T10) | 1.7 | 2.1 | 1.8 | 2.1 | 98.1 | 97.6 | 97.8 | 97.4 |
| F9 (W2) | 1.6 | 2.0 | 1.8 | 2.0 | 98.1 | 97.7 | 97.9 | 97.5 |
| F10 (W5) | 1.7 | 2.1 | 1.8 | 2.1 | 98.0 | 97.6 | 97.8 | 97.5 |
| F11(W10) | 1.6 | 1.9 | 1.9 | 2.0 | 98.0 | 97.8 | 97.7 | 97.5 |

### (3) CEX

As shown in Table 22, no significant change in CEX main peak was observed in any formula after shaking.

**Table 22 CEX results_experiment under shaking**

| **formula e** | **Acidic peak %** | | | | **MP%** | | | | **Basic peak %** | | | |
|---|---|---|---|---|---|---|---|---|---|---|---|---|
| | T0 | 3D | 7D | 14 D | T0 | 3D | 7D | 14 D | T0 | 3D | 7D | 14D |
| F6 (T2) | 23.7 | 21. 0 | 21. 1 | 21. 3 | 62.7 | 64. 6 | 64. 5 | 64. 2 | 13. 6 | 14. 4 | 14. 4 | 14.5 |
| F7(T5) | 23.6 | 21. 0 | 21. 1 | 21. 0 | 62.8 | 64. 7 | 64. 5 | 64. 5 | 13. 6 | 14. 3 | 14. 4 | 14.4 |
| F8 (T10) | 23.9 | 21. 2 | 21. 2 | 21. 0 | 62.5 | 64. 4 | 64. 3 | 64. 2 | 13. 6 | 14. 4 | 14. 5 | 14.8 |
| F9 (W2) | 23.7 | 20. 7 | 20. 9 | 21. 0 | 62.7 | 64. 9 | 64. 8 | 64. 5 | 13. 7 | 14. 4 | 14. 4 | 14.5 |
| F10 (W5) | 23.7 | 21. 1 | 21. 2 | 21. 0 | 62.7 | 64. 5 | 64. 1 | 64. 5 | 13. 6 | 14. 4 | 14. 7 | 14.5 |
| F11(W1 0) | 23.7 | 21. 5 | 20. 7 | 21. 3 | 62.7 | 64. 1 | 64. 9 | 64. 1 | 13. 6 | 14. 5 | 14. 4 | 14.6 |

### 3. Conclusion

There was no significant change in appearance, OD405 and DLS, etc, in any formula undergoing the 40°C condition. A slight decrease in SEC, a medium decrease in NR CE-SDS and a medium decrease in CEX main peak were observed, and there was no significant difference among the formulae.

Under stirring, no significant change in concentration was observed in any formula, but the appearance of F6 (T2), F7 (T5), F8 (W2) and F10(W5) changed from opalescent to turbid, an increase in OD405 was observed in F7 (T5), F9 (W2) and F10 (W5), increases in DLS diameter and PDI were observed in all formulae, no significant change in purity was observed.

Under shaking, no significant changes in results such as appearance and DLS were observed in any formula, no significant change in purity was observed in all formulae, there was no difference between formulae.

In surfactant screening, there was no significant difference between polysorbate 80 and polysorbate 20, all formulae exhibited resistance against shaking stress, and all of them satisfied the test requirements. Taking cost into comprehensive consideration, polysorbate 80 was selected as surfactant for subsequent experiments.

### Example 18: Screening experiment for pH values

### 1. Experiment Design

The formulae of the sample and the detection method are as shown in Table 23. The UFDF sample was buffer-exchanged into the target formulae by dialysis, Then, the sample was diluted to 30 mg/mL using the corresponding auxiliary material stock solution. Finally, the sample was obtained by filteration using 0.22 µm PVDF membrane. After sample preparation, appearance, concentration, osmotic pressure, viscosity, k_{D}, Tₒₙₛₑₜ of sample were assayed.

**Table 23 Experiment design of screening experiment for pH**

| **Formul ae** | **Protein Conc.** | **Buffer system** | | **Surfactant** | **Conditions of the stability experiment and test items** |
|---|---|---|---|---|---|
| F12 | 30 mg/mL | 20mM acetate (Ace) | 4.5 | | |
| F13 | | | 5.0 | | |
| F14 | | | 5.5 | 0.05% polysorbate 80 | T0: Protein Conc., appearance, OD405, pH, DLS, SEC, CEX, NR CE-SDS |
| F15 | | 20mM of histidine (His) | 5.0 | | |
| F16 | | | 5.5 | | Freezing and thawing (-70°C to RT, 3/5 cycles): Protein Conc., pH, OD405, DLS, MFI |
| F17 | | | 6.0 | | |
| F18 | | 20mM Citrate (Cit) | 5.5 | | |
| | | | | | Shaking (200 rpm, 1/3/5day(s)): |
| F19 | | | 6.0 | | Protein Conc., appearance, pH, OD405, CEX, NR CE-SDS, High temperature 40°C (3/7/14 days): Protein Conc., OD405, pH, DLS, SEC, CEX, NR CE-SDS |
| F20 | | | 6.5 | | |

### 2. Experiment

### 2.1. Freezing and thawing experiment

### (1) concentration, pH, diameter and OD405 results

As shown in Table 24, after freezing and thawing for 5 cycles, an increasing trend in OD405 was observed in F18 (Cit5.5), F19 (Cit6.0) and F20 (Cit6.5), no significant change in OD405 was observed in any other formula. No significant changes in sample concentration and pH were observed in any of the 9 formulae. F18 (Cit5.5), F19 (Cit6.0) and F20 (Cit6.5) samples had a larger diameter, and no significant change in sample diameter was observed after freezing and thawing.

**Table 24 Appearance, concentration, pH and OD405 results_freezing and thawing**

| **Formula e** | **Concentration (mg/mL)** | | **OD405** | | **pH** | | **Diameter (nm)** | |
|---|---|---|---|---|---|---|---|---|
| | T0 | 5 cycles | T0 | 5 cycles | T0 | 5 cycles | T0 | 5 cycles |
| F12 (Ace4.5) | 29.5 | 29.7 | 0.062 | 0.065 | 4.7 | 4.8 | 2.8 | 2.8 |
| F13 (Ace5.0) | 30.7 | 31.8 | 0.067 | 0.071 | 5.2 | 5.2 | 4.5 | 4.6 |
| F14 (Ace5.5) | 30.5 | 31.6 | 0.073 | 0.077 | 5.7 | 5.6 | 6.2 | 4.6 |
| F15 (His5.0) | 30.1 | 30.5 | 0.071 | 0.076 | 5.1 | 5.1 | 5.8 | 5.8 |
| F16 (His5.5) | 30.5 | 31.1 | 0.076 | 0.078 | 5.6 | 5.6 | 6.8 | 7.6 |
| F17 (His6.0) | 30.2 | 31.0 | 0.072 | 0.077 | 6.1 | 6.1 | 6.7 | 7.5 |
| F18 (Cit5.5) | 30.4 | 31.1 | 0.063 | 0.090 | 5.6 | 5.5 | 12.0 | 11.3 |
| F19 (Cit6.0) | 30.7 | 30.5 | 0.079 | 0.086 | 6.0 | 5.9 | 10.0 | 9.9 |
| F20 (Cit6.5) | 30.2 | 30.6 | 0.075 | 0.080 | 6.5 | 6.3 | 8.9 | 9.0 |

### (2) MFI results

As shown in Table 25, at T0, the numbers of the insoluble particles ≥25 µm in all formulae were less than 10 /mL; the numbers of the insoluble particles ≥10 µm in all formulae were less than 100 /mL. After freezing and thawing, the insoluble particles ≥2 µm and ≥ 10 µm was observed significantly increased in F19 (Cit6.0), and no significant difference was observed in any other formula.

**Table 25 MFI results_freezing and thawing**

| **Formula e** | **2-10 µm** | | | | **10-25 µm** | | | | **≥25 µm** | | | |
|---|---|---|---|---|---|---|---|---|---|---|---|---|
| | T0 | 1 cycl e | 3 cycl es | 5 cycl es | T0 | 1 cycle | 3 cycles | 5 cycles | T0 | 1 cycl e | 3 cycles | 5 cycle s |
| F12 (Ace4.5) | 726 | 1211 | 344 | 1429 | 19 | 15 | 4 | 176 | 4 | 4 | 0 | 11 |
| F13 (Ace5.0) | 194 9 | 589 | 925 | 1429 | 57 | 19 | 19 | 76 | 0 | 0 | 11 | 8 |
| F14 (Ace5.5) | 220 9 | 967 | 1143 | 3020 | 42 | 15 | 65 | 456 | 0 | 0 | 4 | 139 |
| F15 (His5.0) | 982 | 405 | 940 | 2713 | 4 | 27 | 38 | 283 | 0 | 0 | 4 | 34 |
| F16 (His5.5) | 715 | 822 | 6699 | 772 | 0 | 8 | 917 | 69 | 0 | 4 | 23 | 4 |
| F17 (His6.0) | 507 1 | 428 | 459 | 482 | 57 | 8 | 11 | 15 | 0 | 0 | 8 | 0 |
| F18 (Cit5.5) | 486 5 | 699 | 688 | 1204 | 19 | 73 | 15 | 80 | 0 | 4 | 8 | 0 |
| F19 (Cit6.0) | 389 4 | 1295 1 | 1368 1 | 1256 9 | 54 | 1597 | 959 | 1536 | 4 | 8 | 19 | 19 |
| F20 (Cit6.5) | 145 2 | 5385 | 2281 | 1093 7 | 50 | 46 | 8 | 696 | 0 | 8 | 0 | 19 |

### 2.2. Experiment under shaking

### (1) Appearance, concentration, pH and OD405 results

As shown in Table 26, at T0, all samples were opalescent, no visible foreign matter was observed in all samples, all of them satisfied the test requirements in terms of protein concentration and pH. After shaking at 200 rpm for 5 days, the appearance of F18 (Cit5.5) became turbid, an increasing trend in OD405 was observed in F18 (Cit5.5), F19 (Cit6.0) and F20 (Cit6.5), no significant change in appearance and OD405was observed in any other formula. No significant changes in sample concentration and pH were observed in any of the 9 formulae.

**Table 26 Appearance, concentration, pH and OD405 results_shaking**

| **Formulae** | **Appearance** | | **Concentration (mg/mL)** | | **OD405** | | **pH** | |
|---|---|---|---|---|---|---|---|---|
| | T0 | 5 days | T0 | 5 days | T0 | 5 days | T0 | 5 days |
| F12 (Ace4.5) | SOC | SOC | 29.5 | 29.3 | 0.062 | 0.062 | 4.7 | 4.6 |
| F13 (Ace5.0) | SOC | SOC | 30.7 | 30.7 | 0.067 | 0.067 | 5.2 | 5.0 |
| F14 (Ace5.5) | SOC | SOC | 30.5 | 30.5 | 0.073 | 0.073 | 5.7 | 5.5 |
| F15 (His5.0) | SOC | SOC | 30.1 | 30.2 | 0.071 | 0.071 | 5.1 | 5.0 |
| F16 (His5.5) | SOC | SOC | 30.5 | 30.4 | 0.076 | 0.075 | 5.6 | 5.5 |
| F17 (His6.0) | SOC | SOC | 30.2 | 30.3 | 0.072 | 0.075 | 6.1 | 6.1 |
| F18 (Cit5.5) | SOC | Turbid | 30.4 | 30.3 | 0.063 | 0.074 | 5.6 | 5.4 |
| F19 (Cit6.0) | SOC | SOC | 30.7 | 30.7 | 0.079 | 0.086 | 6.0 | 5.9 |
| F20 (Cit6.5) | SOC | SOC | 30.2 | 30.2 | 0.075 | 0.080 | 6.5 | 6.4 |

| | | | | | | | | |
|---|---|---|---|---|---|---|---|---|
| SOC=slightly opalescent Turbid=turbid | | | | | | | | |

### (2) NR CE-SDS and CEX results

As shown in Table 27 and Table 28, no changes in NR CE-SDS main peak and CEX main peak were observed in any of all formulae.

**Table 27 NR CE-SDS results_shaking**

| **Formulae** | **LMW%** | | | | **MP%** | | | |
|---|---|---|---|---|---|---|---|---|
| | T0 | 1 day | 3 days | 5 days | T0 | 1 day | 3 days | 5 days |
| F12 (Ace4.5) | 1.6 | 1.8 | 1.6 | 1.8 | 98.1 | 98.0 | 98.1 | 97.8 |
| F13 (Ace5.0) | 1.6 | 1.7 | 1.6 | 1.8 | 98.1 | 97.9 | 98.0 | 97.7 |
| F14 (Ace5.5) | 1.6 | 1.9 | 1.6 | 1.8 | 98.1 | 97.7 | 97.8 | 97.7 |
| F15 (His5.0) | 1.5 | 1.8 | 1.6 | 1.8 | 98.2 | 98.0 | 98.0 | 97.8 |
| F16 (His5.5) | 1.5 | 1.9 | 1.6 | 1.8 | 98.2 | 97.7 | 98.0 | 97.8 |
| F17 (His6.0) | 1.6 | 1.8 | 1.5 | 1.9 | 98.0 | 97.9 | 98.1 | 97.6 |
| F18 (Cit5.5) | 2.2 | 1.9 | 1.7 | 1.9 | 97.6 | 97.7 | 98.0 | 97.8 |
| F19 (Cit6.0) | 1.5 | 1.9 | 1.6 | 1.9 | 98.1 | 97.7 | 97.8 | 97.5 |
| F20 (Cit6.5) | 1.6 | 2.0 | 1.7 | 1.8 | 98.1 | 97.5 | 97.7 | 97.5 |

**Table 28 CEX results_shaking**

| **Formulae** | **Acidic peak** | | | | **Main peak** | | | | **Basic peak** | | | |
|---|---|---|---|---|---|---|---|---|---|---|---|---|
| | T0 | 1D | 3D | 5D | T0 | 1D | 3D | 5D | T0 | 1D | 3D | 5D |
| F12 (Ace4.5) | 23. 8 | 21. 1 | 21. 4 | 21. 6 | 62. 4 | 64. 2 | 63. 4 | 63. 5 | 13. 9 | 14. 8 | 15. 2 | 14.9 |
| F13 (Ace5.0) | 23. 7 | 21. 0 | 21. 2 | 21. 2 | 62. 4 | 64. 4 | 64. 1 | 64. 1 | 13. 9 | 14. 6 | 14. 7 | 14.7 |
| F14 (Ace5.5) | 23. 3 | 20. 8 | 20. 9 | 20. 7 | 63. 0 | 64. 6 | 64. 6 | 64. 6 | 13. 7 | 14. 6 | 14. 5 | 14.7 |
| F15 (His5.0) | 21. 5 | 20. 4 | 20. 7 | 20. 7 | 64. 7 | 65. 1 | 64. 7 | 64. 5 | 13. 8 | 14. 5 | 14. 7 | 14.8 |
| F16 (His5.5) | 21. 9 | 21. 1 | 20. 8 | 20. 8 | 64. 4 | 64. 5 | 64. 6 | 64. 5 | 13. 7 | 14. 4 | 14. 5 | 14.7 |
| F17 (His6.0) | 22. 1 | 21. 1 | 20. 7 | 21. 1 | 64. 2 | 64. 4 | 65. 0 | 64. 4 | 13. 7 | 14. 5 | 14. 3 | 14.5 |
| F18 (Cit5.5) | 23. 4 | 20. 7 | 20. 6 | 21. 0 | 62. 9 | 64. 7 | 64. 7 | 64. 4 | 13. 7 | 14. 6 | 14. 6 | 14.6 |
| F19 (Cit6.0) | 23. 3 | 20. 9 | 21. 1 | 21. 2 | 63. 1 | 64. 6 | 64. 3 | 64. 2 | 13. 6 | 14. 5 | 14. 6 | 14.6 |
| F20 (Cit6.5) | 23. 5 | 21. 0 | 20. 8 | 21. 3 | 62. 9 | 64. 6 | 64. 7 | 64. 2 | 13. 6 | 14. 4 | 14. 5 | 14.5 |

### 2.3. Experiment under a high temperature of 40°C

### (1) Concentration, pH, diameter and OD405 results

As shown in Table 29, after being placed at 40°C for 14 days, no significant differences in concentration, pH and diameter were observed in any of the 9 formulae. However, an increase in OD405 to certain extent was observed in F18 (Cit5.5).

**Table 29 Concentration, pH and OD405 results_high temperature**

| **Formulae** | **Concentration (mg/mL)** | | **OD405** | | **pH** | | **Diameter (nm)** | |
|---|---|---|---|---|---|---|---|---|
| | T0 | 14 days | T0 | 14 days | T0 | 14 days | T0 | 14 days |
| F12 (Ace4.5) | 29.5 | 29.5 | 0.062 | 0.064 | 4.7 | 4.7 | 2.8 | 2.8 |
| F13 (Ace5.0) | 30.7 | 30.8 | 0.067 | 0.070 | 5.2 | 5.1 | 4.5 | 4.6 |
| F14 (Ace5.5) | 30.5 | 30.4 | 0.073 | 0.076 | 5.7 | 5.5 | 6.2 | 7.2 |
| F15 (His5.0) | 30.1 | 30.3 | 0.071 | 0.073 | 5.1 | 5.0 | 5.8 | 6.2 |
| F16 (His5.5) | 30.5 | 30.5 | 0.076 | 0.072 | 5.6 | 5.5 | 6.8 | 7.3 |
| F17 (His6.0) | 30.2 | 30.3 | 0.072 | 0.080 | 6.1 | 6.0 | 6.7 | 7.5 |
| F18 (Cit5.5) | 30.4 | 30.6 | 0.063 | 0.097 | 5.6 | 5.5 | 12.0 | 8.0 |
| F19 (Cit6.0) | 30.7 | 30.5 | 0.079 | 0.090 | 6.0 | 5.9 | 10.0 | 9.6 |
| F20 (Cit6.5) | 30.2 | 30.2 | 0.075 | 0.084 | 6.5 | 6.4 | 8.9 | 9.2 |

### (2) SEC results

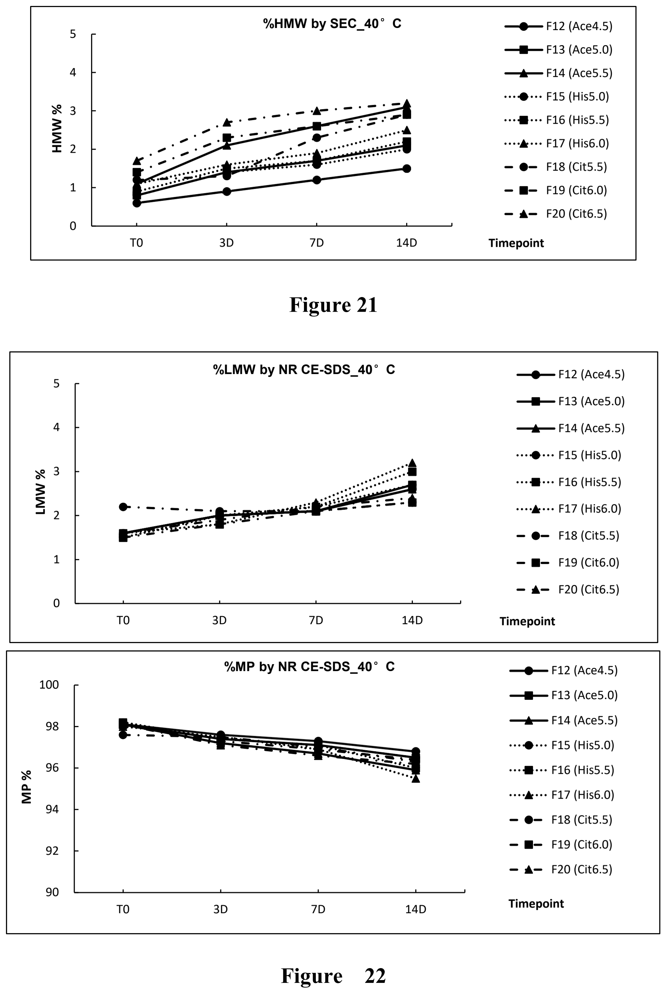

As shown in Table 30 and Figure 21, the SEC HMW% content of protein in histidine formulae was generally lower, and the SEC HMW% content of protein in acetate formulae and citrate formulae increased with increasing pH, and the SEC HMW% of F14 (Ace5.5) increased to a greater extent.

**Table 30 SEC results_high temperature**

| **Formulae** | **HMW%** | | | | **MP%** | | | | **LMW%** | | | |
|---|---|---|---|---|---|---|---|---|---|---|---|---|
| | T0 | 3 day s | 7 day s | 14 day s | T0 | 3 day s | 7 day s | 14 day s | T0 | 3 day s | 7 day s | 14 day s |
| F12 (Ace4.5) | 0.6 | 0.9 | 1.2 | 1.5 | 99. 0 | 98. 9 | 98. 7 | 98. 3 | 0.4 | 0.1 | 0.2 | 0.2 |
| F13 (Ace5.0) | 0.8 | 1.4 | 1.7 | 2.1 | 98. 8 | 98. 5 | 98. 1 | 97. 7 | 0.4 | 0.1 | 0.2 | 0.2 |
| F14 (Ace5.5) | 1.1 | 2.1 | 2.6 | 3.1 | 98. 5 | 97. 7 | 97. 3 | 96. 6 | 0.4 | 0.2 | 0.1 | 0.2 |
| F15 (His5.0) | 0.8 | 1.4 | 1.6 | 2.0 | 98. 8 | 98. 5 | 98. 2 | 97. 8 | 0.4 | 0.2 | 0.2 | 0.2 |
| F16 (His5.5) | 0.9 | 1.5 | 1.7 | 2.2 | 98. 7 | 98. 4 | 98. 1 | 97. 6 | 0.4 | 0.2 | 0.2 | 0.2 |
| F17 (His6.0) | 1.1 | 1.6 | 1.9 | 2.5 | 98. 5 | 98. 2 | 98. 0 | 97. 3 | 0.4 | 0.2 | 0.2 | 0.2 |
| F18 (Cit5.5) | 1.2 | 1.3 | 2.3 | 2.9 | 98. 4 | 98. 5 | 97. 6 | 96. 9 | 0.4 | 0.2 | 0.1 | 0.2 |
| F19 (Cit6.0) | 1.4 | 2.3 | 2.6 | 2.9 | 98. 2 | 97. 5 | 97. 3 | 96. 9 | 0.4 | 0.2 | 0.2 | 0.2 |
| F20 (Cit6.5) | 1.7 | 2.7 | 3.0 | 3.2 | 97. 9 | 97. 0 | 96. 9 | 96. 6 | 0.4 | 0.3 | 0.2 | 0.2 |

### (3) NR CE-SDS results

As shown in Table 31 and Figure 22, at high temperature of 40°C, the LMW% in all formulae increased over time, but the results of all formulae were within the acceptable range.

**Table 31 NR CE-SDS results_high temperature**

| **Formulae** | **LMW%** | | | | **MP%** | | | |
|---|---|---|---|---|---|---|---|---|
| | T0 | 3 days | 7 days | 14 days | T0 | 3 days | 7 days | 14 days |
| F12 (Ace4.5) | 1.6 | 2.0 | 2.1 | 2.6 | 98.1 | 97.6 | 97.3 | 96.8 |
| F13 (Ace5.0) | 1.6 | 2.0 | 2.1 | 2.7 | 98.1 | 97.4 | 97.1 | 96.5 |
| F14 (Ace5.5) | 1.6 | 2.0 | 2.1 | 2.6 | 98.1 | 97.2 | 96.7 | 95.9 |
| F15 (His5.0) | 1.5 | 2.0 | 2.2 | 2.7 | 98.2 | 97.4 | 97.1 | 96.5 |
| F16 (His5.5) | 1.5 | 2.0 | 2.2 | 3.0 | 98.2 | 97.4 | 97.1 | 96.0 |
| F17 (His6.0) | 1.6 | 1.8 | 2.3 | 3.2 | 98.0 | 97.5 | 96.9 | 95.5 |
| F18 (Cit5.5) | 2.2 | 2.1 | 2.1 | 2.3 | 97.6 | 97.5 | 97.0 | 96.3 |
| F19 (Cit6.0) | 1.5 | 1.8 | 2.1 | 2.3 | 98.1 | 97.3 | 96.9 | 96.4 |
| F20 (Cit6.5) | 1.6 | 1.9 | 2.2 | 2.4 | 98.1 | 97.1 | 96.6 | 96.2 |

### (4) CEX results

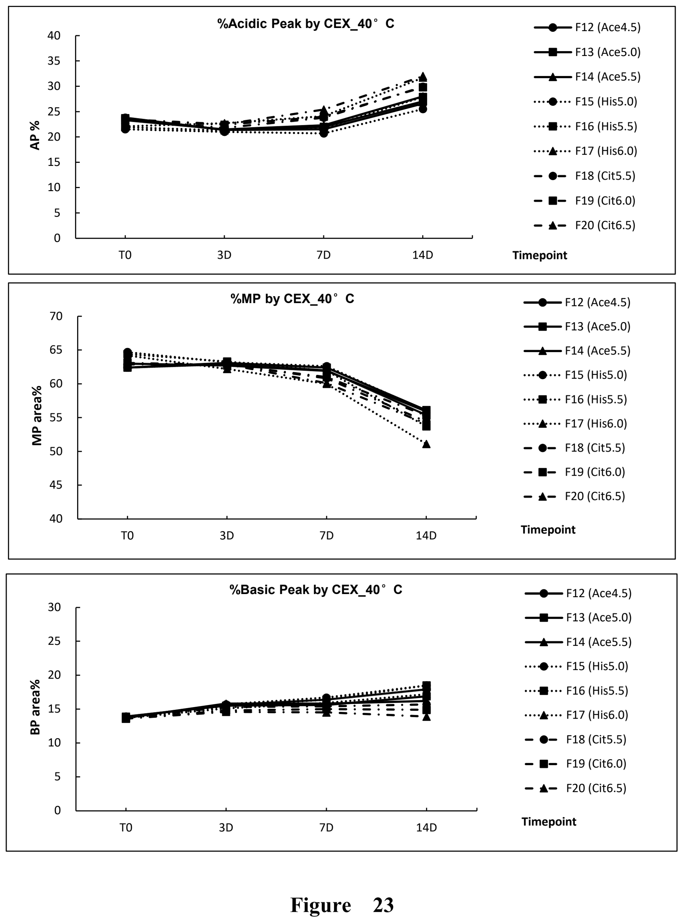

As shown in Table 32 and Figure 23, results of all formulae were within the acceptable range, wherein the main peaks of F17 (His6.0) exhibit the largest change, and there was no significant difference for other formulae.

**Table 32 CEX results_high temperature**

| **formulae** | **Acidic peak %** | | | | **MP%** | | | | **Basic peak %** | | | |
|---|---|---|---|---|---|---|---|---|---|---|---|---|
| | T0 | 3 days | 7 days | 14 days | T0 | 3 days | 7 days | 14 days | T0 | 3 days | 7 days | 14 days |
| F12 (Ace4.5) | 23.8 | 21.4 | 21.5 | 26.6 | 62.4 | 62.9 | 62.0 | 55.4 | 13.9 | 15.6 | 16.4 | 17.9 |
| F13 (Ace5.0) | 23.7 | 21.4 | 22.0 | 27.0 | 62.4 | 63.1 | 62.4 | 56.1 | 13.9 | 15.5 | 15.6 | 16.9 |
| F14 (Ace5.5) | 23.3 | 21.5 | 22.3 | 28.0 | 63.0 | 62.7 | 61.9 | 55.9 | 13.7 | 15.8 | 15.8 | 16.2 |
| F15 (His5.0) | 21.5 | 21.0 | 20.7 | 25.5 | 64.7 | 63.2 | 62.6 | 56.0 | 13.8 | 15.7 | 16.7 | 18.5 |
| F16 (His5.5) | 21.9 | 21.3 | 21.7 | 27.9 | 64.4 | 63.3 | 61.9 | 53.7 | 13.7 | 15.4 | 16.4 | 18.5 |
| F17 (His6.0) | 22.1 | 22.7 | 24.1 | 31.7 | 64.2 | 62.2 | 60.0 | 51.1 | 13.7 | 15.1 | 15.9 | 17.2 |
| F18 (Cit5.5) | 23.4 | 21.8 | 23.8 | 29.9 | 62.9 | 62.9 | 60.8 | 54.4 | 13.7 | 15.3 | 15.4 | 15.7 |
| F19 (Cit6.0) | 23.3 | 22.4 | 24.0 | 29.8 | 63.1 | 62.8 | 61.0 | *55.3* | 13.6 | 14.8 | 15.0 | 14.9 |
| F20 (Cit6.5) | 23.5 | 22.5 | 25.4 | 32.0 | 62.9 | 62.9 | 60.1 | 54.1 | 13.6 | 14.6 | 14.5 | 13.9 |

### 3. Conclusion

In screening experiment for pH, at T0, the diameters of F18 (Cit5.5), F19 (Cit6.0) and F20 (Cit6.5) were bigger, suggesting that their looser structures; in experiment under shaking, the appearance of F18 (Cit5.5) changed from opalescent to turbid, which was inferior to other formulae; in freezing and thawing experiments, significantly changes in the number of insoluble particles were observed in F19 (Cit6.0) and F20 (Cit6.5); under the conditions of 40°C, an increase in OD405 was observed in F18 (Cit5.5), a significantly increase in SEC polymers was observed in F14 (Ace5.5), a significantly decrease in CEX main peak and a significantly increase in NR CE-SDS oligomers were observed in F16 (His5.5) and F17 (His6.0).

In general, the acetic acid and histidine systems had superior performances on stability. Generally, the acetic acid system at pH 4.5 and pH 5.0, and the histidine system at pH 5.0 and pH 5.5 lead to good protein stability. Considering that the acetate buffer system at pH 5.0 and the histidine system at pH 5.5 have good capacities in terms of buffering efficacy and robustness, these two systems ehxibit a good operating flexibility during pH adjustment in the process, and a good risk control for extreme cases that deviate from the target value. In addition, with respect to the formulations for subcutaneous injection, the histidine has lower irritation, thus could be better tolerated by patients. Therefore, F13 (Ace5.0) acetate buffer at pH 5.0 and F16 (His5.5) histidine buffer at pH 5.5 are preferred buffer systems.

### Example 19: Confirmation experiment for low concentration formulae

### 1. Experiment Design

The formulae of the sample and the detection method are as shown in Table 33. The samples were buffer-exchanged into the target formulae by dialysis, Then, the corresponding auxiliary material stock solution was used for diluting to 20 mg/mL. Finally, the sample was obtained by filteration using 0.22 µm PVDF membrane. After sample preparation, the appearance, concentration, osmotic pressure, viscosity, kD, Tₒₙₛₑₜ were assessed.

**Table 33 Experiment design of formulae**

| **Formula e** | **Protein Conc.** | **Formulae information** | **Conditions of the stability experiment** | **Test items** |
|---|---|---|---|---|
| F21 | 20 mg/mL | 10 mM Histidine, 8.6% sucrose, 0.05%, polysorbate 80, pH 5.3 | 5°C (1/3 months) | Visual inspection, pH, Protein Conc., osmotic pressure, viscosity, OD405, diameter, MFI, SEC, NR-CE, R-CE, CEX, biological activity, PS80 |
| | | | 25°C (2/4/6 weeks, 3 months) | |
| | | | 40°C (2/4/6 weeks) | |
| | | | -20°C (1/3 months) | |
| | | | -30°C (1/3 months) freezing and thawing (-70°C to room temperature, 3/5 cycles) | |
| | | | shaking (200 rpm, 1/3/5day(s) | |
| | | | high temperature40°C (3/7/14 day(s) | |

### 2. Experiment results

### 2.1 Appearance, concentration, pH, diameter and OD405 results

As shown in Table 34, after stirring, freezing and thawing, exposuring to light, shaking, as well as incubating at 5°C, 25°C, 40°C, -20°C, -30°C, no significant changs in the basic properties (appearance, concentration, pH, biological activity) were observed in formula F21. With respect to diameter and PD% results, except for the condition at 40°C, no significant difference was observed under all conditions of the stability experiment; the significant change in results is normal since 40°C is a more severe stress condition for protein formulations, and the judgment should be made based on the specific results of SEC, NR-CE, and CEX.

**Table 34 Appearance, concentration, pH and OD405 results**

| **Conditions of the stability experiment** | | **Appearan ce** | **Concentrati on (mg/mL)** | **OD40 5** | **p H** | **diameter(n m)** | **PD%** | **Biologic al activity** |
|---|---|---|---|---|---|---|---|---|
| T0 | | SOC | 20.7 | 0.017 | 5. 3 | 5.9 | 23.1 | 111 |
| Stirring | 2H | 20.6 | 20.6 | 0.020 | 5. 3 | 6.4 | 51.9 | NT |
| Freezing and thawing for 5 cycles | 20 C | SOC | 20.7 | 0.018 | N T | 6.1 | 22.9 | |
| | - 30 C | SOC | 20.6 | 0.019 | | 6.1 | 22.5 | |
| | - 70 C | SOC | 20.6 | 0.018 | | 6.0 | 23.0 | |
| Exposurin g to light | 7D | SOC | 20.6 | 0.018 | | 5.7 | 15.2 | 102 |
| Shaking | 10 D | SOC | 20.7 | 0.018 | | 5.8 | 21.8 | NT |
| 25°C | 2W | SOC | 20.5 | 0.020 | | 6.1 | 19.5 | |
| | 4W | SOC | 20.8 | 0.019 | 5. 3 | 6.3 | 27.9 | |
| | 6W | SOC | 20.7 | 0.019 | N T | 6.2 | 40.1 | |
| | 3M | SOC | 20.6 | 0.018 | 5. 3 | 6.7 | 47.2 | |
| 40°C | 2W | SOC | 20.4 | 0.023 | N T | 7.1 | 56.0 | |
| | 4W | SOC | 20.8 | 0.023 | 5. 3 | 11.2 | Multimod al | 96 |
| | 4W I | SOC | 20.7 | 0.022 | 5. 3 | 12.7 | Multimod al | 93 |
| | 6W | SOC | 20.7 | 0.032 | N T | 20.7 | Multimod al | 104 |
| 5°C | 1M | SOC | 20.6 | 0.018 | 5. 3 | 5.7 | 11 | NT |
| | 3M | SOC | 20.5 | 0.020 | 5. 3 | 6.1 | 20.2 | |
| -20°C | 1M | SOC | 20.6 | 0.019 | 5. 3 | 5.6 | 8 | |
| | 3M | SOC | 20.5 | 0.017 | 5. 3 | 5.7 | 9.0 | |
| -30°C | 1M | SOC | 20.6 | 0.017 | 5. 3 | 5.6 | 8.7 | |
| | 3M | SOC | 20.5 | 0.016 | 5. 3 | 5.6 | 8.6 | |

| | | | | | | | | |
|---|---|---|---|---|---|---|---|---|
| SOC=slightly opalescent, Multimodal=multiple peaks, NT=not tested | | | | | | | | |

### 2.2 SEC, CEX, NR CE-SDS results

As shown in Table 35, after stirring, freezing and thawing, exposuring to light, shaking as well as incubating at 5°C, 25°C, -20°C, -30°C, no significant changes in SEC, CEX, NR CE were observed in formula F21. Decreases in a certain extent in main peaks of SEC, CEX and NR CE were observed under 40°C, which does not affect biological activity; no significant difference in each test item was observed between the inverted samples and the upright samples which are placed at 40°C for 4 weeks, indicating that the packaging material has good compatibility with the protein formulation.

**Table 15 SEC, CEX, NR CE-SDS results**

| **Conditions of the stability experiment** | | **SEC** | | | **CEX** | | | **NR CE** | |
|---|---|---|---|---|---|---|---|---|---|
| | | HMW% | MP% | LMW% | Acidic peak % | Main peak % | Basic peak % | MP% | LMW% |
| T0 | | 0.6 | 99.4 | ND | 24.0 | 73.1 | 2.9 | 98.0 | 1.7 |
| Stirring | 2H | 0.6 | 99.4 | ND | 23.7 | 73.5 | 2.8 | 98.7 | 1.1 |
| Freezing and thawing for 5 cycles | 20C | 0.8 | 99.2 | ND | 22.3 | 74.3 | 3.4 | 98.6 | 1.4 |
| | 30C | 0.8 | 99.2 | ND | 22.5 | 73.9 | 3.6 | 98.9 | 1.1 |
| | - 70C | 0.8 | 99.2 | ND | 22.0 | 74.8 | 3.2 | 98.7 | 1.3 |
| Exposuring to light | 7D | 0.9 | 99.1 | ND | 24.2 | 72.3 | 3.6 | 98.6 | 1.3 |
| Shaking | 10D | 0.7 | 99.3 | ND | 24.0 | 71.7 | 4.3 | 98.8 | 1.2 |
| 25°C | 2W | 0.8 | 99.2 | ND | 22.2 | 74.0 | 3.7 | 98.8 | 1.0 |
| | 4W | 0.7 | 99.3 | ND | 24.1 | 72.4 | 3.6 | 98.2 | 1.6 |
| | 6W | 0.7 | 99.3 | ND | 26.5 | 69.7 | 3.9 | 98.4 | 1.6 |
| | 3M | 0.8 | 99.1 | 0.1 | 31.0 | 64.6 | 4.3 | 98.4 | 1.6 |
| | 6M | 1.0 | 98.8 | 0.2 | 41.0 | 54.5 | 4.5 | 97.1 | 2.5 |
| 40°C | 2W | 0.9 | 98.8 | 0.3 | 34.5 | 60.3 | 5.2 | 97.0 | 2.6 |
| | 4W | 1.1 | 98.2 | 0.7 | 49.5 | 45.7 | 4.8 | 93.0 | 6.3 |
| | 4WI | 1.0 | 98.3 | 0.7 | 49.1 | 46.3 | 4.6 | 93.4 | 6.1 |
| | 6W | 1.7 | 95.2 | 1.0 | 54.5 | 39.0 | 6.5 | 91.3 | 8.6 |
| 5°C | 1M | 0.7 | 99.3 | ND | 21.4 | 75.4 | 3.2 | 98.4 | 1.4 |
| | 3M | 0.7 | 99.3 | ND | 24.4 | 71.3 | 4.2 | 98.9 | 1.1 |
| | 6M | 0.7 | 99.3 | ND | 23.3 | 72.6 | 4.1 | 97.7 | 1.4 |
| -20°C | 1M | 0.7 | 99.3 | ND | 22.3 | 74.1 | 3.6 | 98.3 | 1.5 |
| | 3M | 0.7 | 99.3 | ND | 23.7 | 72.3 | 4.0 | 98.9 | 1.1 |
| | 6M | 0.7 | 99.3 | ND | 22.5 | 73.1 | 4.4 | 98.4 | 1.3 |
| -30°C | 1M | 0.7 | 99.3 | ND | 22.1 | 74.7 | 3.2 | 98.1 | 1.7 |
| | 3M | 0.7 | 99.3 | ND | 22.6 | 73.4 | 4.0 | 99.1 | 0.9 |
| | 6M | 0.7 | 99.3 | ND | 22.7 | 73.2 | 4.1 | 98.4 | 1.3 |

| | | | | | | | | | |
|---|---|---|---|---|---|---|---|---|---|
| ND represents "not detected" | | | | | | | | | |

### 3. Conclusion

The results of confirmation experiment of low concentration formulae suggest that, the final formula F21 remains stable after being placed at 5°C, 25°C, -20°C, -30°C for a long time, and well tolerates freezing and thawing and shaking, remains unchanged under exposure to light and stirring, but will change to a certain extent under 40°C, so that such sample should be prevented from being placed at high temperatures for too long. The surfactant screening, excipient screening, pH screening, and final formula confirmation study were carried out for the development of low concentration formulation, and a final formula comprising 20 mg/mL fo protein, 10 mM of histidine salt, 8.6% (w/v) of sucrose, 0.05% (w/v) of polysorbate 80, and having a pH of 5.3 was selected for the low concentration formulation.

### Example 20: High concentration experiment

### 1. Experiment Design

The formulae of the sample, the placement conditions of the stability experiment and the detection method are as shown in Table 36. The UFDF sample was concentrated by ultrafiltration, and then diluted to 150 mg/mL using the corresponding auxiliary material stock solution. Finally, the sample was obtained by filteration using 0.22 µm PVDF membrane. The prepared samples were taken for *k_{D},* Tₒₙₛₑₜ and viscosity measurements respectively.

**Table 36 Experiment design for high concentration formulation**

| **Formulae** | **Buffer system** | **pH** | **Auxiliary material** | **Test items** |
|---|---|---|---|---|
| F22(A45) | 10 mM acetate (Ace) | 4.5 | NA | *k_{D}* |
| F23(A50) | | 5.0 | | |
| F24(A55) | | 5.5 | | |
| F25(H50) | 10mM histidine (His) | 5.0 | | |
| F26(H55) | | 5.5 | | |
| | | | | Tₒₙₛₑₜ |
| F27(H60) | | 6.0 | | |
| | | | | osmotic pressure |
| F28(SUC) | 10mM acetate (Ace) | 5.0 | 5.8% sucrose (Suc) | |
| F29 (PRO) | | | 230 mM proline (Pro) | |
| F30(ARG) | | | 125 mM arginine (Arg) | |

### 2. Experiment results

**Table 37 kD, Tonset and osmotic pressure results**

| **Formulae** | ***k_{D}* (mL/g)** | **Tₒₙₛₑₜ,(°C)** | **Osmotic pressure Osmo. (mOsm/kg)** |
|---|---|---|---|
| F22 (A45) | 107.0 | 70.5 | NT (not tested) |
| F23(A50) | 40.6 | 68.0 | |
| F24 (A55) | 13.2 | 68.1 | |
| F25(H50) | 21.1 | 61.7 | |
| F26(H55) | 9.3 | 63.4 | |
| F27(H60) | 6.5 | 64.9 | |
| F28(SUC) | 22.2 | 67.2 | 262 |
| F29 (PRO) | 22.5 | 63.7 | 316 |
| F30(ARG) | -9.8 | 58.3 | 276 |

### (1) k_{D} results

The kD values of acetate buffers at pH 4.5, 5.0, and 5.5 and histidine buffers at pH 5.0, 5.5, and 6.0 are all positive. In 10 mM acetate buffer at pH 5.0, the kD values of the formulations with 5.8% sucrose or 230 mM proline as the auxiliary material are positive, while the kD value is negative when the auxiliary material is 125 mM arginine. A positive kD indicates that the interaction between protein molecules in the system is a strong net repulsive force, which is beneficial to the long-term colloidal stability of protein. When the kD is negative, it indicates that the interaction between protein molecules is a net attractive force, which is unfavorable to the long-term colloidal stability of protein.

### (2) Tₒₙₛₑₜ results

The thermal stability of proteins could be predicted based on Tₒₙₛₑₜ. Except for the F29 (ARG) arginine formulae, the Tₒₙₛₑₜ values of all samples are greater than 60°C, indicating that the proteins have good thermal stability in these formulae.

### (3) Osmotic pressure

According to the osmotic pressure results, the concentrations of 125 mM arginine and 5.8% sucrose are lower than the target values and need to be further adjusted in subsequent experiments.

### 3. Conclusion

In the general property study of different buffers and auxiliary materials, the k_{D} of 10mM Acetate (pH 4.5, pH 5.0, pH 5.5) and 10mM Histidine (pH5.0, pH5.5 and pH6.0) are positive, and the Tₒₙₛₑₜ values are all greater than 60°C, and all of them satisfied the test requirements. With respect to three auxiliary materials, the k_{D} and Tₒₙₛₑₜ of arginine are inferior to that of sucrose and proline. For injections for subcutaneous administration, the injection using histidine as buffer system has lower irritation, and the pH of the injection should not be too low. Therefore, on the whole, 10mM Acetate at pH 5.0, 10mM Histidine at pH 5.0, and 10mM Histidine at pH 5.5 are preferred, and are used for subsequent studies in combination with sucrose or proline.

### Example 21: Experiment process comparison experiment for 6 formulae

### 1. Experiment Design

The formulae of the sample, the placement conditions of the stability experiment and the detection method are as shown in Table 38. UFDF sample from Fed-batch was concentrated to suitable concentration by ultrafiltration, then the corresponding auxiliary material stock solution was used for diluting to 150 mg/mL. Finally, the sample was obtained by filteration using 0.22 µm PVDF membrane. 1 mL sample was taken into a 2R injection vial for experiment under exposuring to light at 25°C, 40°C, 2 mL sample was taken into a 6R injection vial for experiment under stirring, 2 mL sample to was taken into a 2R injection vial for experiment under shaking, respectively.

**Table 38 Experiment Design for optimal 6 formulae**

| **Formulae** | **Concentratio n** | **Production technique of the drug substance** | **Formulae information** | **Conditions of the stability experiment** |
|---|---|---|---|---|
| F31 (Ace50Suc) | 150 mg/mL | Perfusion | 10 mM Acetate pH5.0, 190 mM sucrose, 0.1% polysorbate 80 | T0 |
| | | | | Exposure to light (3D) |
| F32 (His50Suc) | | | 10 mM Histidine pH5.0, 190 mM sucrose, 0.1% polysorbate 80 | |
| | | | | Shaking (10D) |
| | | | | 25°C (2/4W) |
| | | | | 40°C (2/4/6W) |
| F33 (His55Suc) | | | 10 mM Histidine pH5.5, 190 mM | |
| | | | sucrose, 0.1% polysorbate 80 | |
| F34 (Ace50Pro) | | | 10 mM Acetate pH5.0, 230 mM proline, 0.1% polysorbate 80 | |
| F35 (His50Pro) | | | 10 mM Histidine pH5.0, 230 mM proline, 0.1% polysorbate 80 | |
| F36 (His55Pro) | | | 10 mM Histidine pH5.5, 230 mM proline, 0.1% polysorbate 80 | |
| F37 (FedBatch) | | Fed-Batch | 10 mM Acetate pH5.0, 190 mM sucrose, 0.1% polysorbate 80 | T0, 40°C (2/4/6W) |
| Test items | T0: appearance, OD405, pH, concentration, osmotic pressure, SEC, NR CE-SDS, CEX, activity, viscosity | | | |
| | exposure to light: appearance, OD405, concentration, DLS, SEC, NR CE-SDS, CEX | | | |
| | Shaking: appearance, OD405, concentration, DLS, SEC, NR CE-SDS, CEX, MFI | | | |
| | 25°C: appearance, OD405, pH, concentration, DLS, SEC, NR CE-SDS, CEX | | | |
| | 40°C: appearance, OD405, pH, concentration, DLS, SEC, NR CE-SDS, CEX, activity | | | |

### 2. Experiment

### 2.1. Experiment under high temperature of 40°C

### (1) Appearance, diameter, concentration, OD405 and pH

As shown in Table 39, the osmotic pressure in all formulae was within the target scope (250 to 350 mOsm/kg H₂O), comparing with the formulae comprising proline as the auxiliary material (F34, F35, F36), the formulae comprising sucrose as the auxiliary material (F31, F32, F33, F37) have a higher viscosity, the formulae comprising histidine buffer has a higher viscosity than the formulae comprising acetate buffer. The pH of F34 (Ace50Pro), F35 (His50Pro) and F36 (Fed-Batch) are higher than the target value, and and needed to be adjusted in subsequent experiments. After the treatment at 40°C for 4 weeks, no changes in appearance, diameter, concentration and pH were observed in 7 formulae. However, a slight increase in OD405 was observed in F31 (Ace50Suc), F32 (His50Suc), F33 (His55Suc), F34 (Ace50Pro) and F36 (His55Pro).

**Table 39 Appearance, diameter, concentration, OD405 and pH results_ experiment under high temperature of 40°C**

| **Formu la** | **Osmoti c pressur e (mOsm /kg H2O)** | **Visc osity cP** | **Appearance** | | **Concentrati on** | | **OD405** | | **PH** | | **Diameter** | |
|---|---|---|---|---|---|---|---|---|---|---|---|---|
| | | | T0 | 4W | T0 | 4W | T0 | 4W | T0 | 2W | T0 | 4W |
| F31 (Ace50 Suc) | 262 | 18.7 | SYC | SYC | 146. 2 | 147. 1 | 0.22 | 0.27 | 5.1 | 5.2 | 2.9 | 3.3 |
| F32 (His50 Suc) | 306 | 29.2 | SYC | SYC | 147. 4 | 148. 6 | 0.22 | 0.26 | 5.1 | 5.1 | 3.5 | 5.1 |
| F33 (His55 Suc) | 311 | 28.2 | SYC | SYC | 145. 5 | 148. 5 | 0.23 | 0.27 | 5.4 | 5.4 | 3.6 | 6.0 |
| F34 (Ace50 Pro) | 300 | 10.9 | SYC | SYC | 148. 4 | 148. 3 | 0.23 | 0.27 | 5.3 | 5.3 | 2.4 | 3.0 |
| F35 (His50 Pro) | 322 | 14.1 | SYC | SYC | 148. 4 | 148. 4 | 0.23 | 0.24 | 5.2 | 5.2 | 2.7 | 4.2 |
| F36 (His55 Pro) | 307 | 14.6 | SYC | SYC | 148. 9 | 147. 1 | 0.22 | 0.27 | 5.5 | 5.4 | 3.4 | 4.7 |
| F37 (FedBa tch) | 300 | 23.3 | CC | CC | 150. 3 | 150. 5 | 0.03 | 0.03 | 5.3 | 5.2 | 5.3 | 2.6 |

| | | | | | | | | | | | | |
|---|---|---|---|---|---|---|---|---|---|---|---|---|
| SYC: yellowish and clear, free of visible foreign matter CC: colorless and clear, free of visible foreign matter | | | | | | | | | | | | |

### (2) SEC

As shown in Table 40 and Figure 33, after the treatment at 40°C for 4 or 6 weeks, an increase in SEC HMW% and a decrease in MP% were observed in all formulae, a greater decrease in main peak was observed in F31 (Ace50Suc) and F32 (His50Suc), a smaller decrease in main peak was observed in F33 (His55Suc) and F36 (His55Pro). When comparing F31 (Ace50Suc) and F37 (Fed-Batch), which have the same formulae, it was found that the drug substance from the fed-batch bioprocess exhibited a smaller decrease in the SEC main peak at 40 °C than the drug substance from the perfusion bioprocess.

**Table 40 SEC results_experiment under a high temperature**

| **Formulae** | **HMW%** | | | | **MP%** | | | | **LMW%** | | | |
|---|---|---|---|---|---|---|---|---|---|---|---|---|
| | T0 | 2W | 4W | 6W | T0 | 2W | 4W | 6W | T0 | 2W | 4W | 6W |
| F31 (Ace50Suc) | 2.9 | 6.9 | 8.8 | 10.1 | 95.1 | 92.3 | 90.0 | 89.3 | 1.9 | 0.8 | 1.2 | 0.7 |
| F32 (His50Suc) | 2.8 | 6.4 | 8.5 | NT | 95.3 | 93.7 | 90.2 | NT | 1.9 | ND | 1.2 | NT |
| F33 (His55Suc) | 3.1 | 6.6 | 8.0 | NT | 95.0 | 93.5 | 90.9 | NT | 1.9 | ND | 1.1 | NT |
| F34 (Ace50Pro) | 2.8 | 6.4 | 8.2 | NT | 95.2 | 93.3 | 90.7 | NT | 2.0 | 0.2 | 1.2 | NT |
| F35 (His50Pro) | 2.8 | 6.2 | 8.3 | NT | 95.1 | 92.9 | 90.4 | NT | 2.1 | 0.9 | 1.3 | NT |
| F36 (His55Pro) | 2.9 | 6.3 | 7.7 | NT | 95.4 | 92.7 | 91.2 | NT | 1.6 | 1.1 | 1.1 | NT |
| F37 (FedBatch) | 0.2 | 1.2 | 1.9 | 2.6 | 99.7 | 98.7 | 98.0 | 96.9 | 0.1 | 0.1 | 0.2 | 0.5 |

### (3) NR CE-SDS

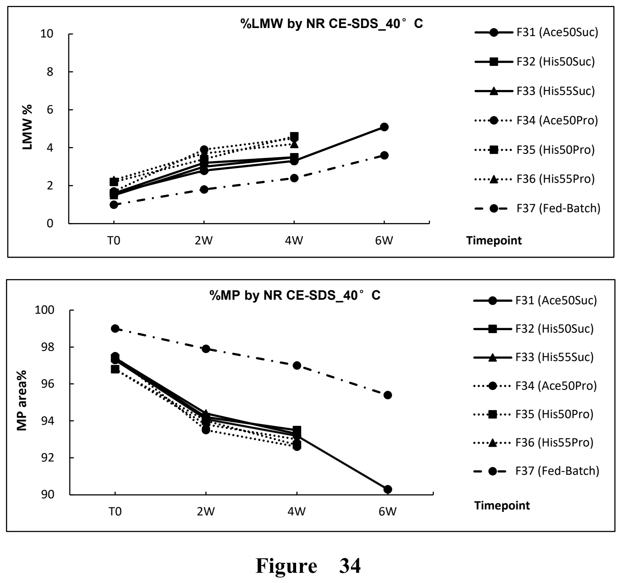

As shown in Table 38 and Figure 34, after the treatment at 40°C for 4 or 6 weeks, an increase in NR CE-SDS LMW% to a certain extent and a decrease in MP% were observed in all formulae, wherein F34 (Ace50Pro) exihibited the largest change. Comparing F31 (Ace50Suc) and F37 (Fed-Batch) which have the same formula, the extent of decrease in NR CE-SDS main peak at 40°C of the DS from Fed-batch is less than that from Perfusion.

**Table 41 NR CE-SDS results_experiment under high temperature**

| **formulae** | **LMW%** | | | | **MP%** | | | |
|---|---|---|---|---|---|---|---|---|
| | T0 | 2W | 4W | 6W | T0 | 2W | 4W | 6W |
| F31 (Ace50Suc) | 1.6 | 2.8 | 3.3 | 5.1 | 97.3 | 94.1 | 93.2 | 90.3 |
| F32 (His50Suc) | 1.6 | 3.2 | 3.5 | NT | 97.4 | 94.2 | 93.5 | NT |
| F33 (His55Suc) | 1.5 | 3.0 | 3.5 | NT | 97.4 | 94.4 | 93.3 | NT |
| F34 (Ace50Pro) | 1.7 | 3.9 | 4.5 | NT | 97.5 | 93.5 | 92.6 | NT |
| F35 (His50Pro) | 2.2 | 3.4 | 4.6 | NT | 96.8 | 94.0 | 92.7 | NT |
| F36 (His55Pro) | 2.3 | 3.7 | 4.2 | NT | 96.8 | 93.8 | 93.0 | NT |
| F37 (FedBatch) | 1.0 | 1.8 | 2.4 | 3.6 | 99.0 | 97.9 | 97.0 | 95.4 |

### (4) CEX

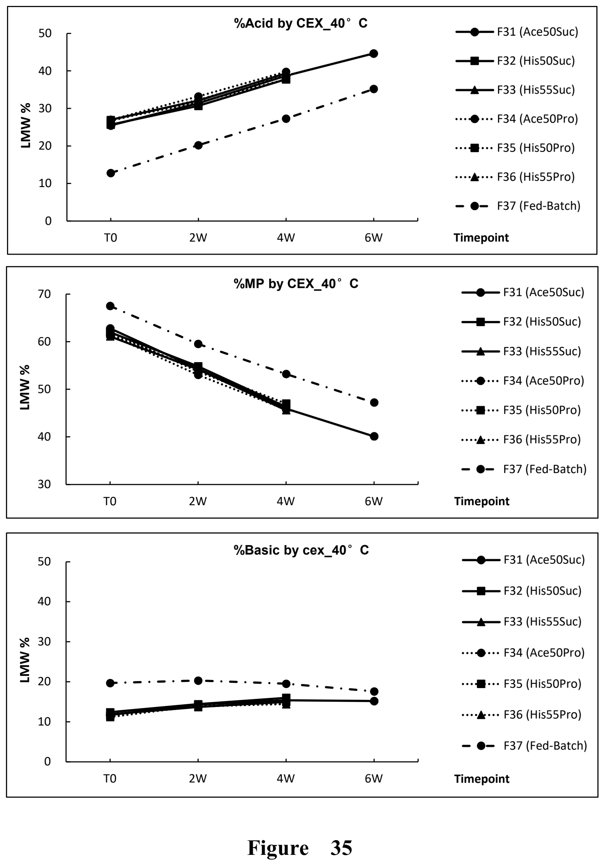

As shown in Table 42 and Figure 35, after the treatment at 40°C for 4 weeks, an increase in acidic peak and a decrease in MP% were observed in all formulae. The CEX main peak of F31 (Ace50Suc) decreases by a slightly bigger extent than that of F37(Fed-Batch) which has a same formula.

**Table 42 CEX results_experiment under high temperature**

| **Formulae** | **Acidic peak area%** | | | | **MP%** | | | | **Basic peak area%** | | | |
|---|---|---|---|---|---|---|---|---|---|---|---|---|
| | T0 | 2W | 4W | 6W | T0 | 2W | 4W | 6W | T0 | 2W | 4W | 6W |
| F31 (Ace50Su c) | 25. 5 | 31. 4 | 38. 7 | 44. 7 | 62. 8 | 54. 3 | 45. 9 | 40. 1 | 11. 7 | 14. 3 | 15. 4 | 15. 2 |
| F32 (His50Su c) | 25. 7 | 30. 7 | 37. 8 | NT | 62. 0 | 54. 8 | 46. 3 | NT | 12. 4 | 14. 4 | 16. 0 | NT |
| F33 (His55Su c) | 27. 0 | 32. 1 | 39. 4 | NT | 61. 1 | 54. 2 | 45. 6 | NT | 11. 9 | 13. 7 | 15. 0 | NT |
| F34 (Ace50Pr o) | 27. 0 | 33. 2 | 39. 8 | NT | 61. 4 | 53. 0 | 45. 7 | NT | 11. 6 | 13. 8 | 14. 5 | NT |
| F35 (His50Pro ) | 26. 9 | 31. 6 | 37. 8 | NT | 61. 6 | 54. 1 | 47. 0 | NT | 11. 5 | 14. 3 | 15. 2 | NT |
| F36 (His55Pro ) | 26. 7 | 32. 3 | 39. 1 | NT | 62. 1 | 53. 7 | 46. 5 | NT | 11. 2 | 14. 0 | 14. 4 | NT |
| F37 (FedBatch ) | 12. 8 | 20. 2 | 27. 3 | 35. 2 | 67. 5 | 59. 5 | 53. 2 | 47. 2 | 19. 7 | 20. 3 | 19. 5 | 17. 6 |

### (5) Activity

As shown in Table 43, after being placed at 40°C for 4 weeks, no significant difference in activity was observed for any formula.

**Table 43 Activity results_experiment under high temperature**

| **Formulae** | **Celluar activity%** | |
|---|---|---|
| | T0 | 40°C4W |
| F31 (Ace50Suc) | NT | 92 |
| F32 (His50Suc) | NT | 92 |
| F33 (His55Suc) | NT | 104 |
| F34 (Ace50Pro) | NT | 96 |
| F35 (His50Pro) | NT | 93 |
| F36 (His55Pro) | NT | 92 |
| F37 (FedBatch) | 103 | 95 |

### 2.2. Experiment under 25°C

After the treatment at 25°C for 4 weeks, no changes in appearance, Conc., pH and OD405 were observed in any formula. A medium degree of decrease in purity (SEC, NR CE-SDS, CEX) was observed in all samples, wherein F31 (Ace50Suc) exhibited the largest change in SEC, F34 (Ace50Pro) exhibited the largest change in NR CE-SDS. Generally, the histidine buffer system is superior than the acetate buffer system. The specific degradation trends are as shown in Figure 36, Figure 37 and Figure 38.

### 2.3. Exposure to light

After exposuring to light for 3 days, no changes in appearance, DLS, Conc., pH and OD405 were observed in any formula. A descrease in purity was observed in each formula, the specific trends are as shown in Figure 39, Figure 40 and Figure 41. F35 (His50Pro) exhibited the minimum increase in SEC HMW% and NR CE-SDS LMW%. F33 (His55Suc) and F36 (His55Pro) exhibited the smallest change in CEX.

### 2.4. Shaking

After shaking at 200 rpm for 10 days, no changes in appearance, DLS, Conc., pH and OD405 were observed in any formula. Regarding the purity, no significant change in CEX was observed. After shaking at 200 rpm for 10 days, changes in SEC and NR CE-SDS were observed in any formula, wherein F32 (His50Suc) exhibited the minimum increase in LMW%, no significant difference was observed for other formulae, the sepecific trends are as shown in Figure 42, Figure 43 and Figure 44. After shaking at 200 rpm for 10 days, no significant difference in the number of insoluble particleswas observed in any formula.

### 3. Conclusion

After being placed at 25°C for 4 weeks, there was no significant changes in appearance, DLS, OD405, etc among the formulae. However, F31(Ace50Suc) exhibited a bigger change in SEC than other formulae, and F34 (Ace50Pro) exhibited a bigger change in NR CE-SDS than other formulae.

After being placed at 40°C for 4 weeks, no changes in appearance, Conc, pH, DLS were observed. However, a slight increase in OD405 was observed in F31 (Ace50Suc), F32 (His50Suc), F33 (His55Suc), F34 (Ace50Pro) and F36 (His55Pro), decreases in main peaks of SEC, CEX and NR CE-SDS to certain extent were observed in all formulae. F31 (Ace50Suc) and F32 (His50Suc) exhibited a change to a greater extent in SEC than other formulae, and F34 (Ace50Pro) exhibited change to a greater extent in NR CE-SDS than other formulae. When comparing F31 (Ace50Suc) and F37 (Fed-Batch), which have the same formulae, it was found that in F37, in which the drug substance is from the fed-batch bioprocess, the extents of all changes in purity at the condition of 40°C are smaller than those in F31, in which the drug substance is from the perfusion bioprocess.

After exposure to light for 3 days, no significant changes in concentration, appearance, pH and DLS, etc were observed in any formula. Decreases in main peaks of SEC, NR CE-SDS and CEX were observed in all formulae, wherein F35 (His50Pro) exhibited a decrease to a smaller extent in main peaks of SEC and NR CE-SDS than other formulae, F33 (His55Suc) and F36 (His55Pro) exhibited decreases to a smaller extent in CEX main peak than other formulae.

After shaking, there was no significant changes in concentration, appearance, pH, DLS, OD405, CEX main peaks, etc in any formula. Slight decreases in main peaks of SEC and NR CE-SDS were observed, which may be caused by the experiment being carried out under shaking and at 25°C. Among which, F35 (His50Pro) exhibited change to the smallest extent in SEC, F32 (His50Suc) exhibited change to the smallest extent in NR CE-SDS.

In this study, acetate buffer at pH 5.0 and histidine buffer at pH 5.0 and pH 5.5, in combination with sucrose, proline, and 0.05% polysorbate 80 were selected for carrying out the experiment. The stress conditions such as exposure to light, shaking, 25°C, 40°C were investigated. In addition, the stability differences between the drug substances produced by perfusion process and fed-batch process were identified. The results showed that under 40°C, 25°C, and exposuring to light, the change in purity of the histidine buffer is less than that of the acetate buffer, and the drug substance produced by the fed-batch bioprocess was more stable at 40°C than the drug substance produced by the perfusion process. Considering that histidine is more friendly to subcutaneous injection and sucrose is more widely used in biological formulations, 10mM of Histidine, and the pH of 5.3, a middle value between 5.0 and 5.5, in combination with sucrose were selected for the final formula confirmation experiment.

### Example 22: Screening experiment for surfactant concentration in high concentration protein formulation

### 1. Experiment Design

The formulae of the samples, the placement conditions of the stability experiment and the detection method are as shown in Table 44. The UFDF sample was diluted to 100 mg/mL using the corresponding auxiliary material stock solution. Finally, the sample was obtained by filteration using 0.22 µm PVDF membrane. 2 mL sample was taken into a 6R injection vial for experiment under stirring, 2 mL sample was taken into a 2R injection vial for experiment under shaking, respectively.

**Table 44 Experiment design of the second screening experiment for surfactant concentration**

| **Formulae** | **Concentratio n** | **Productio n technique of the drug substance** | **Formula information** | **Conditions of the stability experiment** |
|---|---|---|---|---|
| F38 (0.025%) | 100 mg/mL | Fed-batch | 10 mM Histidine pH5.3, 6.0% sucrose, 0.025% polysorbate 80 | T0 |
| F39 (0.05%) | | | 10 mM Histidine pH5.3, 6.0% sucrose, 0.05% polysorbate 80 | |
| | | | | Shaking (200 rpm, 5D) |
| | | | | Stirring (200 rpm, 2H) |
| F40 (0.1%) | | | 10 mM Histidine pH5.3, 6.0% sucrose, 0.1% polysorbate 80 | |
| Test items | T0: appearance, OD405, pH, concentration, osmotic pressure, SEC, MFI Stirring/shaking: appearance, OD405, SEC, MFI | | | |

### 2. Results

In this experiment, three concentrations of polysorbate 80, namely 0.025%, 0.05% and 0.1%, were investigated, and the samples were subjected to stress tests such as shaking and stirring. The results showed that there was no significant difference in appearance, insoluble particles, diameter, purity (SEC) and other results in samples with each polysorbate 80 concentration, all of which satisfied the test requirements. An intermediate concentration of 0.05% was selected for the final formula confirmation test.

**Table 45 Experiment results of the second screening for surfactant concentration**

| Test Items | | F38 (0.025%) | | | F39 (0.05%) | | | F40 (0.1%) | | |
|---|---|---|---|---|---|---|---|---|---|---|
| | | T0 | M2H | A5D | T0 | M2H | A5D | T0 | M2H | A5D |
| Osmotic pressure (mOsm/kg H₂O) | | 278 | NT | | | | | | | |
| pH | | 5.2 | NT | | 5.2 | NT | | 5.2 | NT | |
| Appearance | | SOC | HOC | SOC | SOC | HOC | SOC | SOC | HOC | SOC |
| Concentration | | 91.1 | NT | NT | 91.1 | NT | NT | 92.8 | NT | NT |
| OD405 | | 0.03 | 0.06 | 0.04 | 0.03 | 0.05 | 0.04 | 0.04 | 0.06 | 0.04 |
| MFI | ≥2 µm | 3202 | 8155 | 99 | 61 | 28963 | 42 | 61 | 9428 | 134 |
| | ≥ 10 µm | 15 | 0 | 0 | 0 | 214 | 0 | 0 | 61 | 0 |
| | ≥ 25 µm | 0 | 0 | 0 | 0 | 23 | 0 | 0 | 11 | 0 |
| DLS | Single peak diameter (nm) | 4.0 | 4.1 | 4.4 | 4.0 | 4.4 | 4.2 | 4.1 | 4.3 | 4.1 |
| SEC | High molecular weight | 1.0 | 1.0 | 1.2 | 1.0 | 1.1 | 1.3 | 1.1 | 1.1 | 1.3 |
| | Main peak % | 92.5 | 92.5 | 92.2 | 92.4 | 92.6 | 92.2 | 92.4 | 92.5 | 92.0 |
| | Low molecular weight% | 6.5 | 6.4 | 6.6 | 6.6 | 6.4 | 6.6 | 6.5 | 6.4 | 6.7 |

| | | | | | | | | | | |
|---|---|---|---|---|---|---|---|---|---|---|
| NT=not tested | | | | | | | | | | |

### Example 23: Formulation Confirmation Study

### 1. Experiment Design

The formulae of the sample, the placement conditions of the stability experiment and the detection method are as shown in Table 46. The UFDF sample was diluted to the target concentrations of the protein, sucrose and polysorbate 80, and then filtered using a 0.22 µm PVDF membrane. 2 mL sample was taken into a 2R injection vial for the stability experiment under -40°C, -20°C, 5°C, 25°C, 40°C, freezing and thawing at -20°C/-40°C, shaking, or exposure to light. 2 mL sample was taken into a 6R injection vial for the experiment under stirring. 2 mL sample was taken into a 5 mL PC vial for experiment under freezing and thawing at -80°C.

**Table 46 Experiment results of formulae for formulation**

| **Formulae for formulation** | **Concentra tion** | **Formulae information** | **Conditions of the stability experiment** | **Test items** |
|---|---|---|---|---|
| F41 | 100 mg/mL | 10 mM Histidine, 7.0% sucrose, 0.05%, polysorbate 80, pH 5.3 | T0 | X, Z, U |
| | | | Freezing and thawing (-80/- 40/-20°C, 5 cycles) | X, Z |
| | | | Stirring (200 rpm, 2 H) | X |
| | | | Shaking (10D) | X, Z |
| | | | Exposure to light (7 D, 78.5% ICH intensity) | X, Y |
| | | | Stressed (40°C, 2, 4, 6 W, 4 W Invert) | X, Y (4W), T |
| | | | Accelerated (25°C, 2, 4, 6 W) | X, T |
| | | | Long-term (5/-20/-40°C, 1, 3, 6 M) | X |
| X: Appearance, Protein Conc., pH, DLS, OD405, SEC, NR-CE-SDS, CEX; | | | | |
| Y: activity; | | | | |
| Z: MFI; | | | | |
| U: osmotic concentration, viscosity | | | | |
| T: PS80. | | | | |

### 2. Results

In the confirmation experiment for formulae, the selected formulae were investigated under various conditions such as freezing and thawing at different temperature points, stirring, shaking, exposure to light, stressd degradation (40°C), accelerated (25°C), long-term storage(5/-20/-40°C). The results showed that no significant change in purity of the sample was observed under the freezing and thawing, stirring, shaking and long-term conditions; a decrease in purity was observed under the condition of 25°C and exposure to light; a significant decrease in purity of the sample was observed after placemant at 40°C for 6 weeks, which however does not affect the binding activity, and PS80 concentration did not decrease in the formula.

The results of the confirmation experiment for the final formula are shown in Table 47, 48, 49 and Table 50.

In the experiment investigating the long-term stability at 5°C, -20°C and -40°C, no significant changes in appearance, DLS, SEC, CEX and NR-CE-SDS, etc were observed in samples.

When being placed at 25°C, no significant change was observed in any formula within 6 weeks, a slight decrease in CEX main peak was observed after 3 months and 6 months. A decrease in polysorbate 80 content was not observed.

When being placed at 40°C for 6 weeks, significant decreases in all main peaks of SEC, CEX and NR-CE-SDS were observed in the sample. However, a decrease in polysorbate 80 content was not observed.

After exposure to light for 7 days, no significant changes in appearance and DLS, etc were ovserved in the sample. A slight decrease in purity was observed, which does not affect the binding activity.

After freezing and thawing, no significant changes in appearance, DLS, SEC, CEX, NR-CE-SDS, etc were observed in the sample. Moreover, there was not any significant increase in the concentration of insoluble particles observed. Freezing conditions at different temperatures had no significant effect on the freezing and thawing stability of the samples.

After stirring at 200 rpm for 2 hours, the appearance of the sample changed from slightly opalescent to turbid, the insoluble particle was therefore not investigated. No significant change was observed in other results.

After shaking at 200 rpm under 25°C for two weeks, no significant changes in appearance, DLS, OD405, MFI, SEC, NR-CE-SDS and CEX main peaks were observed in the sample.

No significant difference in each test item was observed between the inverted samples and the upright samples which are placed at 40°C for 4 weeks, indicating that the packaging material has good compatibility with the protein formulation.

**Table 47 Results of confirmation experiment for the final formula under a high temperature 40°C**

| Sampling point | | SEC | | | | NR CE-SDS | | | icIEF | | | | |
|---|---|---|---|---|---|---|---|---|---|---|---|---|---|
| | | HMW % | MP % | LMW % | | MP % | | LMW % | Acidic peak % | Main peak % | | Basic peak % | |
| T0 | | 1 | 99 | ND | | 98.2 | | 1.5 | 24.9 | 68.1 | | 7 | |
| 4 0 °C | 2W | 1.5 | 98.2 | 0.2 | | 97.4 | | 2.3 | 31 | 61.6 | | 7.5 | |
| | 4W | 2.5 | 97 | 0.4 | | 94 | | 4.8 | 43 | 49.4 | | 7.5 | |
| | 4WI | 2.6 | 96.9 | 0.5 | | 94.1 | | 4.7 | 42.8 | 49.7 | | 7.5 | |
| | 6W | 3 | 96.3 | 0.7 | | 90.5 | | 7.6 | 52.4 | 40.9 | | 6.7 | |

| Sampling point | | Appea rance | Diamete r (nm) | | OD4 05 | | Protein Conc. (mg/mL) | | pH | Osmoti c pressur e (mOsm ol/kg) | Visc osity cP | Activit y % | PS80 % |
|---|---|---|---|---|---|---|---|---|---|---|---|---|---|
| T0 | | SOC | 4.5 | | 0.05 | | 104.3 | | 5.4 | 293 | 6.8 | 90 | NT |
| 4 0 °C | 2W | SOC | 4.4 | | 0.05 | | 105.2 | | NT | NT | NT | NT | 0.044 |
| | 4W | SOC | 4.6 | | 0.06 | | 105.6 | | 5.3 | NT | NT | NT | 0.043 |
| | 4WI | SOC | 4.4 | | 0.07 | | 105.7 | | 5.3 | NT | NT | NT | NT |
| | | | | 6W | SOC | 3.9 | 0.06 | 103.3 | 5.5 | NT | NT | NT | 0.040 |

**Table 48 Results of accelerated (25°C) stability in confirmation experiment for the final formula**

| Sampling point | | SEC | | | | NR CE-SDS | | | icIEF | | | | |
|---|---|---|---|---|---|---|---|---|---|---|---|---|---|
| | | HMW % | MP % | LMW % | | MP % | | LMW % | Acidic peak % | Main peak % | | Basic peak % | |
| T0 | | 1 | 99 | ND | | 98.2 | | 1.5 | 24.9 | 68.1 | | 7 | |
| 25 °C | 2 W | 1 | 99 | ND | | 98.6 | | 1.3 | 23.1 | 70 | | 6.9 | |
| | 4 W | 1.2 | 98.7 | 0.1 | | 98.1 | | 1.5 | 23.8 | 69.7 | | 6.5 | |
| | 6 W | 1.3 | 98.6 | 0.1 | | 98 | | 1.6 | 25.2 | 68.3 | | 6.5 | |
| | 3 M | 1.2 | 98.7 | 0.1 | | 98.7 | | 1.2 | 29 | 65 | | 6 | |
| | 6 M | 1.4 | 98.4 | 0.2 | | 98.7 | | 1.3 | 32.2 | 61.3 | | 6.5 | |

| Sampling point | | Appe aranc e | Diamete r (nm) | | OD40 5 | | Protein Conc. (mg/mL) | | pH | Osmoti c pressur e(mOs mol/kg ) | Visco sity cP | Activi ty% | PS80 % |
|---|---|---|---|---|---|---|---|---|---|---|---|---|---|
| T0 | | SOC | 4.5 | | 0.05 | | 104.3 | | 5.4 | 293 | 6.8 | 90 | NT |
| 25 °C | 2 W | SOC | 3.8 | | 0.04 | | 106.1 | | NT | NT | NT | NT | NT |
| | 4 W | SOC | 4 | | 0.04 | | 106.6 | | 5.4 | NT | NT | NT | 0.044 |
| | 6 W | SOC | 4.1 | | 0.04 | | 103 | | 5.3 | NT | NT | NT | 0.044 |
| | 3 M | SOC | 4.7 | | 0.04 | | 104.6 | | 5.3 | NT | NT | NT | 0.046 |
| | 6 M | SOC | 4.2 | | 0.04 | | 106.8 | | 5.4 | NT | NT | NT | 0.04 |

**Table 49 Results of long-term (5°C/-20°C/-40°C) stability in confirmation experimen for the final formula**

| Sampling point | | SEC | | | | NR CE-SDS | | | icIEF | | | | |
|---|---|---|---|---|---|---|---|---|---|---|---|---|---|
| | | HMW % | MP% | LMW % | | MP % | | LMW% | Acidic peak % | Main peak % | | Basic peak % | |
| T0 | | 1 | 99 | ND | | 98.2 | | 1.5 | 24.9 | 68.1 | | 7 | |
| 5°C | 1M | 1 | 99 | ND | | 98.3 | | 1.2 | 22.5 | 70.9 | | 6.6 | |
| | 3M | 1 | 99 | 0 | | 98.8 | | 1.1 | 25.8 | 68.1 | | 6.1 | |
| | 6M | 1.1 | 98.9 | ND | | 98.8 | | 1.2 | 25 | 68.2 | | 6.8 | |
| 20°C | 1M | 1 | 99.1 | ND | | 98.5 | | 1.3 | 22.7 | 70.3 | | 7 | |
| | 3M | 0.9 | 99.1 | ND | | 98.7 | | 1.2 | 25.6 | 68.1 | | 6.3 | |
| | 6M | 1 | 99 | ND | | 98.6 | | 1.4 | 24.7 | 68.4 | | 6.9 | |
| 40°C | 1M | 1 | 99 | ND | | 98.4 | | 1.3 | 22.8 | 70.3 | | 7 | |
| | 3M | 0.9 | 99.1 | ND | | 98.4 | | 1.4 | 25 | 68.5 | | 6.5 | |
| | 6M | 1 | 99 | ND | | 98.6 | | 1.4 | 24.1 | 69.2 | | 6.7 | |

| Sampling point | | Appea rance | Diameter (nm) | | OD40 5 | | Protein Conc. (mg/mL) | | pH | Osmoti c pressur e (mOsm ol/kg) | Visc osity cP | Acti vity % | PS8 0% |
|---|---|---|---|---|---|---|---|---|---|---|---|---|---|
| T0 | | SOC | 4.5 | | 0.05 | | 104.3 | | 5.4 | 293 | 6.8 | 90 | NT |
| 5°C | 1M | SOC | 4 | | 0.05 | | 105.9 | | 5.3 | NT | NT | NT | NT |
| | 3M | SOC | 4.4 | | 0.04 | | 104.4 | | 5.3 | NT | NT | NT | NT |
| | 6M | SOC | 4.2 | | 0.04 | | 106.6 | | 5.4 | NT | NT | NT | NT |
| - 20°C | 1M | SOC | 4.5 | | 0.05 | | 106.2 | | 5.3 | NT | NT | NT | 0.04 3 |
| | 3M | SOC | 4.5 | | 0.04 | | 104.8 | | 5.3 | NT | NT | NT | NT |
| | 6M | SOC | 4.1 | | 0.04 | | 105.1 | | 5.4 | NT | NT | NT | NT |
| - 40°C | 1M | SOC | 4 | | 0.04 | | 105.8 | | 5.3 | NT | NT | NT | 0.04 3 |
| | 3M | SOC | 4.4 | | 0.04 | | 104.3 | | 5.3 | NT | NT | NT | NT |
| | 6M | SOC | 3.9 | | 0.04 | | 105.2 | | 5.4 | NT | NT | NT | NT |

**Table 50 Results of confirmation experiment for the final formula under stirring, freezing and thawing, exposuring to light, shaking**

| Sampling point | | SEC | | | NR CE-SDS | | icIEF | | | MFI | | |
|---|---|---|---|---|---|---|---|---|---|---|---|---|
| | | HMW % | MP % | LMW % | MP % | LM W % | Acid ic peak % | Main peak % | Ba sic pe ak % | ≥ 2µ m | ≥ 10 µm | ≥ 25µm |
| T0 | | 1 | 99 | ND | 98. 2 | 1.5 | 24.9 | 68.1 | 7.0 | 61 | 0 | 0 |
| Stirring | 2H | 1 | 99 | ND | 98. 3 | 1.3 | 22.9 | 70.2 | 6.9 | NT | NT | NT |
| Freezing and thawing | -80°C | 1 | 98.9 | 0.1 | 98. 4 | 1.3 | 23.4 | 70.3 | 6.3 | 31 | 42 | 23 |
| | -40°C | 1.1 | 98.8 | 0.1 | 98. 3 | 1.4 | 22.5 | 70.8 | 6.7 | 260 | 4 | 0 |
| | -20°C | 1 | 98.8 | 0.1 | 98. 4 | 1.3 | 22.7 | 70.4 | 6.9 | 92 | 0 | 0 |
| Exposuri ng to light | 7D | 2.8 | 97.1 | 0.1 | 96. 7 | 2 | 28 | 65.3 | 6.7 | NT | NT | NT |
| Shaking | 10D | 1 | 99 | ND | 98. 1 | 1.6 | 22.7 | 70.6 | 6.8 | 149 | 0 | 0 |

| Sampling point | | Appea rance | Dia mete r (nm) | OD40 5 | Protein Conc. (mg/mL) | | pH | Osmotic pressure (mOsmol/k g) | | Vis cos ity cP | Ac tivi ty % | PS80 % |
|---|---|---|---|---|---|---|---|---|---|---|---|---|
| T0 | | SOC | 4.5 | 0.05 | 104.3 | | 5.4 | 293 | | 6.8 | 90 | NT |
| Stirring | 2H | Turbi d | 4.4 | 0.06 | 103.7 | | 5.4 | NT | | NT | NT | NT |
| Freezing and thawing | -80°C | SOC | 4.0 | 0.04 | 103.5 | | 5.4 | NT | | NT | NT | NT |
| | -40°C | SOC | 4.2 | 0.04 | 104 | | 5.4 | NT | | NT | NT | NT |
| | -20°C | SOC | 4.1 | 0.04 | 103.9 | | 5.4 | NT | | NT | NT | NT |
| Exposuri ng to light | 7D | SOC | 4.5 | 0.04 | 102.7 | | 5.3 | NT | | NT | 96 | NT |
| Shaking | 10D | SOC | 4 | 0.04 | 103.2 | | 5.3 | NT | | NT | NT | NT |
| SOC=slightly opalescent, Turbid=turbid, NT=not tested | | | | | | | | | | | | |

### 3. Conclusion

The high concentration final formula remains stable after being placed at -40°C, -20°C, 5°C and 25°C for a long time, and well tolerates freezing and thawing and shaking. Changes would occur under exposure to light and stirring conditions, and the extent of change is even greater under the condition of 40°C, so that such sample should be prevented from placement under lighting, high temperature for a long time and violent stirring.

The scope of the present invention is not limited by the specific embodiments described herein. In fact, in addition to those described herein, various modifications provided herein are obvious to those skilled in the art based on the foregoing description and the drawings. Such modifications are deemed to fall within the scope of the appended claims.

## Claims

1. A stable liquid pharmaceutical formulation comprising:
(a) a monoclonal antibody or an antigen-binding fragment thereof that specifically binds to human MASP-2;
(b) a buffer comprising histidine and/or acetate buffer system;
(c) a surfactant comprising polysorbates; and
(d) an auxiliary material comprising sucrose, trehalose and/or proline;
wherein the formulation has a pH of 4.7-6.1, preferably 5.0-6.0, more preferably 5.0-5.5, most preferably 5.3.

2. The pharmaceutical formulation accroding to claim 1, wherein the formulation has one or more of the following characteristics:
(i) a mean diameter of 2-10 µm;
(ii) an osmotic pressure of 250 to 350 mOsm/kg H₂O;
(iii) a viscosity of about 1.0 centipoise to 30 centipoise, for example about 1.0 centipoise to 10 centipoise;
(iv) after being placed at 40°C or 25°C for 6 weeks or under shaking for 14 days, the appearance remains or basically remains in slightly opalescent or a clear state, the mean diameter is unchanged or basically unchanged, and the monomer purity of the antobody in a dissloved state is greater than 95%, preferably 98%, more preferably 99%;
(v) after being placed at 4°C for 1 year to 3 years, the appearance remains or basically remains in slightly opalescent or a clear state, the mean diameter is unchanged or basically unchanged, the monomer purity of the antobody in a dissloved state is greater than 95%, preferably 98%, more preferably 99%;
(vi) after exposure to light for 3 days, the appearance remains or basically remains in slightly opalescent or a clear state, the mean diameter is unchanged or basically unchanged, the monomer purity of the antobody in a dissloved state is greater than 95%, preferably 98%, more preferably 99%;
(vii) after stirring for 6 hours, the appearance remains or basically remains in slightly opalescent or a clear state, the mean diameter is unchanged or basically unchanged, the monomer purity of the antobody in a dissloved state is greater than 95%, preferably 98%, more preferably 99%; and
(viii) after freezing and thawing for 5 cycles, the appearance remains or basically remains in slightly opalescent or a clear state, the mean diameter is unchanged or basically unchanged, the monomer purity of the antobody in a dissloved state is greater than 95%, preferably 98%, more preferably 99%.

3. The pharmaceutical formulation accroding to claim 1 or 2, wherein the concentration of the antibody is 10 mg/ml ± 1 mg/ml to 200 mg/ml ± 20 mg/mL.

4. The pharmaceutical formulation accroding to claim 3, wherein the concentration of the antibody is 20 mg/ml ± 2 mg/mL.

5. The pharmaceutical formulation accroding to claim 3, wherein the concentration of the antibody is 60 mg/ml ± 6 mg/ml.

6. The pharmaceutical formulation accroding to claim 3, wherein the concentration of the antibody is 100 mg/ml ± 10 mg/ml.

7. The pharmaceutical formulation accroding to claim 3, wherein the concentration of the antibody is 150 mg/ml ± 15 mg/ml.

8. The pharmaceutical formulation accroding to claim 3, wherein the concentration of the antibody is from about 20 mg/ml to about 100 mg/ml.

9. The pharmaceutical formulation accroding to any one of claims 1 to 8, wherein the buffer comprises histidine, and the concentration of histidine is 5 mM ± 1 mM to 20 mM ± 4 mM.

10. The pharmaceutical formulation accroding to claim 9, wherein the concentration of the buffer is 10 mM ± 2 mM.

11. The pharmaceutical formulation accroding to claim 10, wherein the buffer comprises L-histidine and L-histidine hydrochloride monohydrate.

12. The pharmaceutical formulation accroding to claim 11, wherein the buffer comprises about 0.175 mg/ml of L- histidine and about 1.86 mg/ml of L-histidine hydrochloride monohydrate.

13. The pharmaceutical formulation accroding to any one of claims 1 to 12, wherein the concentration of the polysorbates is 0.025% ± 0.01% to 0.1% ± 0.01% (w/v).

14. The pharmaceutical formulation accroding to claim 13, wherein the concentration of the polysorbates is 0.05% ± 0.01% (w/v).

15. The pharmaceutical formulation accroding to any one of claims 1 to 14, wherein the polysorbates is polysorbate 80 or polysorbate 20, preferably polysorbate 80.

16. The pharmaceutical formulation accroding to any one of claims 1 to 15, wherein the auxiliary material comprises sucrose.

17. The pharmaceutical formulation accroding to claim 16, wherein the concentration of sucrose is 5.5% ± 0.5% to 9% ± 0.5% (w/v).

18. The pharmaceutical formulation accroding to claim 17, wherein the concentration of sucrose is 5.8% ± 0.5% to 8.6% ± 0.5% (w/v).

19. The pharmaceutical formulation accroding to claim 18, wherein the concentration of sucrose is 5.8% ± 0.5% (w/v).

20. The pharmaceutical formulation accroding to claim 18, wherein the concentration of sucrose is 6.0% ± 0.5% (w/v).

21. The pharmaceutical formulation accroding to claim 18, wherein the concentration of sucrose is 6.5% ± 0.5% (w/v).

22. The pharmaceutical formulation accroding to claim 18, wherein the concentration of sucrose is 7.0% ± 0.5% (w/v).

23. The pharmaceutical formulation accroding to claim 18, wherein the concentration of sucrose is 8.6% ± 0.5% (w/v).

24. The pharmaceutical formulation accroding to claim 1 comprising:
(a) 20 mg/ml ± 2 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of polysorbate 80, and
(d) 6.5% ± 0.5% to 8.6% ± 0.5% (w/v) of sucrose or trehalose,
having a pH of 5.3 ± 0.5.

25. The pharmaceutical formulation accroding to claim 24 comprising:
(a) 20 mg/ml ± 2 mg/ml of an antibody,
(b) a buffer comprising 10 mM ± 2 mM of histidine,
(c) 0.05% ± 0.01% (w/v) of a polysorbate, and
(d) 8.6% ± 0.5% (w/v) of sucrose,
having a pH of 5.3 ± 0.1.

26. The pharmaceutical formulation accroding to claim 25 comprising:
(a) 20 mg/ml ± 2 mg/ml of an antibody,
(b) about 0.175 mg/ml of L-histidine,
(c) about 1.86 mg/ml of L-histidinemono hydrochloride monohydrate,
(d) 0.05% ± 0.01% (w/v) of a polysorbate, and
(e) 8.6% ± 0.5% (w/v) of sucrose,
having a pH of 5.3 ± 0.1.

27. The pharmaceutical formulation accroding to claim 1 comprising:
(a) 60 mg/ml ± 6 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% + 0.5% to 8.6% ± 0.5% (w/v) of sucrose or trehalose,
having a pH of 5.3 ± 0.5.

28. The pharmaceutical formulation accroding to claim 27 comprising:
(a) 60 mg/ml ± 6 mg/ml of an antibody,
(b) a buffer comprising 10 mM ± 2 mM of histidine buffer system,
(c) 0.05% ± 0.01% (w/v) of a polysorbate, and
(d) 8.6% ± 0.5% (w/v) of sucrose,
having a pH of 5.3 ± 0.1.

29. The pharmaceutical formulation accroding to claim 28 comprising:
(a) 60 mg/ml ± 6 mg/ml of an antibody,
(b) about 0.175 mg/ml of L-histidine,
(c) about 1.86 mg/ml of L-histidine monohydrochloride monohydrate,
(d) 0.05% ± 0.01% (w/v) of a polysorbate, and
(e) 8.6% ± 0.5% (w/v) of sucrose,
having a pH of 5.3 ± 0.1.

30. The pharmaceutical formulation accroding to claim 1 comprising:
(a) 100 mg/ml ± 10 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose,
having a pH of 5.3 ± 0.5.

31. The pharmaceutical formulation accroding to claim 30 comprising:
(a) 100 mg/ml ± 10 mg/ml of an antibody,
(b) a buffer comprising 10 mM ± 1 mM of histidine buffer system,
(c) 0.05% ± 0.01% (w/v) of a polysorbate, and
(d) 7.0% ± 0.5% (w/v) of sucrose,
having a pH of 5.3 ± 0.1.

32. The pharmaceutical formulation accroding to claim 31 comprising:
(a) 100 mg/ml ± 10 mg/ml of an antibody,
(b) about 0.175 mg/ml of L-histidine,
(c) about 1.86 mg/ml of L-histidine monohydrochloride monohydrate,
(d) 0.05% ± 0.01% (w/v) of a polysorbate, and
(e) 7.0% ± 0.5% (w/v) of sucrose,
having a pH of 5.3 ± 0.1.

33. The pharmaceutical formulation accroding to claim 1 comprising:
(a) 150 mg/ml ± 15 mg/ml of an antibody,
(b) a buffer comprising 5 mM ± 1 mM to 20 mM ± 4 mM of histidine or acetate,
(c) 0.025% ± 0.01% to 0.1% ± 0.01% (w/v) of a polysorbate, and
(d) 6.5% ± 0.5% to 8.6% ± 0.5% (w/v) of sucrose or trehalose,
having a pH of 5.3 ± 0.5.

34. The pharmaceutical formulation accroding to claim 33 comprising:
(a) 150 mg/ml ± 15mg/ml of an antibody,
(b) a buffer comprising 10 mM ± 1 mM of histidine buffer system,
(c) 0.05% ± 0.01% (w/v) of a polysorbate, and
(d) 7.0% + 0.5% (w/v) of sucrose,
having a pH of 5.3 ± 0.1.

35. The pharmaceutical formulation accroding to claim 34 comprising:
(a) 150 mg/ml ± 15 mg/ml of an antibody,
(b) about 0.175 mg/ml of L-histidine,
(c) about 1.86 mg/ml of L-histidine monohydrochloride monohydrate,
(d) 0.05% ± 0.01% (w/v) of a polysorbate, and
(e) 7.0% ± 0.5% (w/v) of sucrose,
having a pH of 5.3 ± 0.1.

36. The pharmaceutical formulation accroding to any one of claims 21 to 35, wherein the polysorbates is polysorbate 20 or polysorbate 80, preferably polysorbate 80.

37. The pharmaceutical formulation accroding to any one of claims 21 to 35, wherein the antibody or an antigen-binding fragment thereof comprise a heavy chain variable region (VH) and/or a light chain variable region (VL), and wherein the VH comprises HCDR1, HCDR2 and HCDR3, and wherein the amino acid sequence of HCDR1 is DYYIN (SEQ ID NO: 1), the amino acid sequence of HCDR2 is **WIFPGSX₁ SX₂ YX₃ X₄ X₅ X₆ FX₇ X₈** (SEQ ID NO: 2), and the amino acid sequence of HCDR3 is **GDRSGPFX₉ Y** (SEQ ID NO: 3);
and/or
wherein the VL comprises LCDR1, LCDR2 and LCDR3, and wherein the amino acid sequence of LCDR1 is **KSSQSLLYSNGKTYLN** (SEQ ID NO: 4), the amino acid sequence of LCDR2 is **LVSKLDS** (SEQ ID NO: 5), and the amino acid sequence of LCDR3 is **VQX₁₀ THFPFT** (SEQ ID NO: 6);
wherein Xₗ is E, D or G, X₂ is A or P, X₃ is H or Y, X₄ is S or N, X₅ is E or Q, X₆ is K or N, X₇ is K or Q, X₈ is A or G, X₉ is A or P, and X₁₀ is V or G.

38. The pharmaceutical formulation accroding to claim 37, wherein the amino acid sequence of HCDR1 is SEQ ID NO: 1, the amino acid sequence of HCDR2 is SEQ ID NO: 7, 8, 9 or 10, and the amino acid sequence of HCDR3 is SEQ ID NO: 11 or 12;
and/or
the amino acid sequence of LCDR1 is SEQ ID NO: 4, the amino acid sequence of LCDR2 is SEQ ID NO: 5, and the amino acid sequence of LCDR3 is SEQ ID NO: 13 or 14.

39. The pharmaceutical formulation accroding to any one of claims 1-36, wherein the antibody or an antigen-binding fragment thereof comprise a heavy chain variable region (VH) and/or a light chain variable region (VL), and wherein the VH comprises HCDR1, HCDR2 and HCDR3, and wherein:
the amino acid sequence of HCDR1 is SEQ ID NO: 1, the amino acid sequence of HCDR2 is SEQ ID NO: 7, and the amino acid sequence of HCDR3 is SEQ ID NO: 11; or
the amino acid sequence of HCDR1 is SEQ ID NO: 1, the amino acid sequence of HCDR2 is SEQ ID NO: 9, and the amino acid sequence of HCDR3 is SEQ ID NO: 12; or
the amino acid sequence of HCDR1 is SEQ ID NO: 1, the amino acid sequence of HCDR2 is SEQ ID NO: 10, and the amino acid sequence of HCDR3 is SEQ ID NO: 11; or
the amino acid sequence of HCDR1 is SEQ ID NO: 1, the amino acid sequence of HCDR2 is SEQ ID NO: 8, and the amino acid sequence of HCDR3 is SEQ ID NO: 11.

40. The pharmaceutical formulation accroding to claim 39, wherein the antibody or an antigen-binding fragment thereof comprise a light chain variable region (VL), wherein the VL comprises LCDR1, LCDR2 and LCDR3, and wherein:
the amino acid sequence of LCDR1 is SEQ ID NO: 4, the amino acid sequence of LCDR2 is SEQ ID NO: 5, and the amino acid sequence of LCDR3 is SEQ ID NO: 13; or
the amino acid sequence of LCDR1 is SEQ ID NO: 4, the amino acid sequence of LCDR2 is SEQ ID NO: 5, and the amino acid sequence of LCDR3 is SEQ ID NO: 14.

41. The pharmaceutical formulation accroding to any one of claims 37-40, wherein
the amino acid sequence of HCDR1 is SEQ ID NO: 1, the amino acid sequence of HCDR2 is SEQ ID NO: 7, the amino acid sequence of HCDR3 is SEQ ID NO: 11, the amino acid sequence of LCDR1 is SEQ ID NO: 4, the amino acid sequence of LCDR2 is SEQ ID NO: 5, and the amino acid sequence of LCDR3 is SEQ ID NO: 13; or
the amino acid sequence of HCDR1 is SEQ ID NO: 1, the amino acid sequence of HCDR2 is SEQ ID NO: 9, the amino acid sequence of HCDR3 is SEQ ID NO: 12, the amino acid sequence of LCDR1 is SEQ ID NO: 4, the amino acid sequence of LCDR2 is SEQ ID NO: 5, and the amino acid sequence of LCDR3 is SEQ ID NO: 13; or
the amino acid sequence of HCDR1 is SEQ ID NO: 1, the amino acid sequence of HCDR2 is SEQ ID NO: 10, the amino acid sequence of HCDR3 is SEQ ID NO: 11, the amino acid sequence of LCDR1 is SEQ ID NO: 4, the amino acid sequence of LCDR2 is SEQ ID NO: 5, and the amino acid sequence of LCDR3 is SEQ ID NO: 14; or
the amino acid sequence of HCDR1 is SEQ ID NO: 1, the amino acid sequence of HCDR2 is SEQ ID NO: 8, the amino acid sequence of HCDR3 is SEQ ID NO: 11, the amino acid sequence of LCDR1 is SEQ ID NO: 4, the amino acid sequence of LCDR2 is SEQ ID NO: 5, and the amino acid sequence of LCDR3 is SEQ ID NO: 13.

42. The pharmaceutical formulation accroding to any one of claims 37-41, wherein the heavy chain variable region comprises any one amino acid sequence selected from SEQ ID NOs: 15, 17, 18, 20, 22, 24 and 26, or a sequence having at least 80% sequence identity thereof.

43. The pharmaceutical formulation accroding to claim 42, wherein the light chain variable region comprises any one amino acid sequence selected from SEQ ID NOs: 16, 19, 28 and 30, or a sequence having at least 80% sequence identity thereof.

44. The pharmaceutical formulation accroding to any one of claims 37-41, wherein the antibody or an antigen-binding fragment thereof comprise:
a heavy chain variable region comprising the amino acid sequence of SEQ ID NO: 15, and a light chain variable region comprising the amino acid sequence of SEQ ID NO: 16;
a heavy chain variable region comprising the amino acid sequence of SEQ ID NO: 17, and a light chain variable region comprising the amino acid sequence of SEQ ID NO: 16;
a heavy chain variable region comprising the amino acid sequence of SEQ ID NO: 18, and a light chain variable region comprising the amino acid sequence of SEQ ID NO: 19;
a heavy chain variable region comprising the amino acid sequence of SEQ ID NO: 20, and a light chain variable region comprising the amino acid sequence of SEQ ID NO: 28;
a heavy chain variable region comprising the amino acid sequence of SEQ ID NO: 20, and a light chain variable region comprising the amino acid sequence of SEQ ID NO: 30;
a heavy chain variable region comprising the amino acid sequence of SEQ ID NO: 22, and a light chain variable region comprising the amino acid sequence of SEQ ID NO: 28;
a heavy chain variable region comprising the amino acid sequence of SEQ ID NO: 22, and a light chain variable region comprising the amino acid sequence of SEQ ID NO: 30;
a heavy chain variable region comprising the amino acid sequence of SEQ ID NO: 24, and a light chain variable region comprising the amino acid sequence of SEQ ID NO: 28;
a heavy chain variable region comprising the amino acid sequence of SEQ ID NO: 24, and a light chain variable region comprising the amino acid sequence of SEQ ID NO: 30;
a heavy chain variable region comprising the amino acid sequence of SEQ ID NO: 26, and a light chain variable region comprising the amino acid sequence of SEQ ID NO: 28; or
a heavy chain variable region comprising the amino acid sequence of SEQ ID NO: 26, and a light chain variable region comprising the amino acid sequence of SEQ ID NO: 30.

45. The pharmaceutical formulation accroding to any one of claims 37-44, wherein the antibody or an antigen-binding fragment thereof comprise an immunoglobulin constant region, optionally comprise a heavy chain constant region of IgG, and/or a light chain constant region.

46. The pharmaceutical formulation accroding to claim 45, wherein the constant region comprises a mouse constant region, a rabbit constant region, or a human constant region, optionally the constant region comprises a constant region of human IgG1, IgG2, IgG3, or IgG4.

47. The pharmaceutical formulation accroding to claim 45, wherein the heavy chain constant region comprises one or more amino acid substitutions relative to a wild-type human IgG constant region at amino acid residue 252, 254 or 256, optionally the amino acid substitution at amino acid residue 252 is a substitution with tyrosine, the amino acid substitution at amino acid residue 254 is a substitution with threonine, an amino acid substitution at amino acid residue 256 is a substitution with glutamic acid.

48. The pharmaceutical formulation accroding to claim 47, wherein the heavy chain constant region comprises a sequence having at least 80% sequence identity with the wildtype human IgG constant region, and wherein the amino acid residue 252 is substituted with tyrosine, the amino acid residue 254 is substituted with threonine, and the amino acid residue 256 is substituted with glutamic acid, as compared to the wildtype human IgG constant region.

49. A pharmaceutical composition, wherein the composition comprises the pharmaceutical formulation accroding to any one of claims 1 to 48, and the composition in contained in a container.

50. The pharmaceutical composition accroding to claim 49, wherein the container is a vial, for example an injection vial.

51. The pharmaceutical composition accroding to claim 49, wherein the container is a syringe.

52. The pharmaceutical composition accroding to claim 49, wherein the container is a pre-filled syringe.

53. The pharmaceutical composition accroding to claim 49, which is contained in an automatic syringe.

54. The pharmaceutical composition accroding to any one of claims 49 to 53, wherein the container comprises a headspace comprising a gas, wherein the gas comprises less than less than 5 vol% of oxygen, preferably comprises less than 1 vol% of oxygen, more preferably comprises no more than 0.1 vol% of oxygen.

55. A kit comprising:
(i) a container containing a composition which comprises the pharmaceutical formulation accroding to any one of claims 1 to 48, and an instruction for using the composition; or
(ii) a container containing the pharmaceutical composition accroding to any one of claims 49 to 54, and an instruction for using the composition.

56. A unit dosage form comprising the pharmaceutical formulation accroding to any one of claims 1 to 48, wherein the antibody is presented in amount of 0.1 mg to 500 mg.

57. The unit dosage form accroding to claim 56, wherein the antibody is presented in amount of 200 mg or 400 mg.

58. The unit dosage form accroding to claim 56 or 57, which is a glass vial, for example a injection vial.

59. The unit dosage form accroding to claim 56 or 57, which is a pre-filled syringe.

60. The unit dosage form accroding to claim 56 or 57, which is an automatic syringe.

61. The unit dosage form accroding to claim 56 or 57, wherein the glass vial comprises a headspace comprising a gas, wherein the gas comprises less than less than 5 vol% of oxygen, preferably comprises less than 1 vol% of oxygen, more preferably comprises no more than 0.1 vol% of oxygen.

62. A method of inhibiting MASP-2 dependent complement activation, comprising administering to the subject in need thereof a therapeutically effective amount of the pharmaceutical formulation of any of claims 1-48, or the pharmaceutical composition of any of claims 49-54, or the kit of claim 55, or the unit dosage form of any of claims 56-61.

63. A method of reducing level of serum C4 in a subject, comprising administering to the subject in need thereof a therapeutically effective amount of the pharmaceutical formulation of any of claims 1-48, or the pharmaceutical composition of any of claims 49-54, or the kit of claim 55, or the unit dosage form of any of claims 56-61.

64. A method of treating a disease or disorder in a subject, wherein said disease or disorder (1) would benefit from reduction of serum C4 level; (2) is associated with abnormal (e.g., elevated) serum C4 level; (3) would benefit from inhibition of MASP-2 dependent complement activation; and/or (4) is associated with MASP-2 dependent complement activation,
comprising administering to the subject in need thereof a therapeutically effective amount of the pharmaceutical formulation of any of claims 1-48, or the pharmaceutical composition of any of claims 49-54, or the kit of claim 55, or the unit dosage form of any of claims 56-61.

65. Use of the pharmaceutical formulation of any of claims 1-48, or the pharmaceutical composition of any of claims 49-54, or the kit of claim 55, or the unit dosage form of any of claims 56-61 in the manufacture of a medicament, wherein the medicament is used for one or more of the follows:
(1) for inhibiting MASP-2 dependent complement activation;
(2) for treating or preventing a disease or a disorder associated with MASP-2 dependent complement activation;
(3) for treating a condition in a subject that would benefit from inhibition of MASP-2 dependent complement activation;
(4) for reducing level of serum C4 level in a subject;
(5) for treating a condition in a subject that would benefit from reduction of serum C4 level in a subject;
(6) for treating or preventing a disease or a disorder associated with abnormal (e.g. elevated) serum C4 level.

66. The use accroding to claim 65, wherein said a disease or a disorder is an autoimmune disease, a vascular condition, an ischemia-reperfusion injury, atherosclerosis, an inflammation, a pulmonary condition, extracorporeal reperfusion procedure, a musculoskeletal condition, a renal condition, a skin condition, an organ or tissue transplant procedure, a nervous system disorder or injury, a blood disorder, urogenital condition, a nonobese diabetes or a complication associated with Type 1 or Type 2 diabetes, cancer, endocrine disorder, an ophthalmologic condition, or COVID-19 (pneumonia caused by SARS-Cov-2).

67. The use accroding to claim 66, wherein
the autoimmune disease comprises thrombotic microangiopathies (TMAs), atypical hemolytic uremic syndrome (aHUS), hematopoietic transplant-associated thrombotic microangiopathy (TA-TMA), lupus nephritis, systemic lupus erythematosus (SLE) and IgA nephropathy;
the vascular condition comprises a cardiovascular condition, a cerebrovascular condition, a peripheral (e.g., musculoskeletal) vascular condition, a renovascular condition, a mesenteric/enteric vascular condition, revascularization to transplants and/or replants, vasculitis, Henoch-Schonlein purpura nephritis, systemic lupus erythematosus-associated vasculitis, vasculitis associated with rheumatoid arthritis, immune complex vasculitis, Takayasu's disease, dilated cardiomyopathy, diabetic angiopathy, Kawasaki's disease (arteritis), venous gas embolus (VGE), and restenosis following stent placement, rotational atherectomy and percutaneous transluminal coronary angioplasty (PTCA);
the ischemia-reperfusion injury comprises an ischemia-reperfusion injury associated with aortic aneurysm repair, cardiopulmonary bypass, vascular reanastomosis in connection with organ transplants and/or extremity/digit replantation, stroke, myocardial infarction, and hemodynamic resuscitation following shock and/or surgical procedures;
the inflammation comprises inflammatory gastrointestinal disorder comprising pancreatitis, Crohn's disease, ulcerative colitis, irritable bowel syndrome and diverticulitis;
the pulmonary condition comprises acute respiratory distress syndrome, transfusion-related acute lung injury, ischemia/reperfusion acute lung injury, chronic obstructive pulmonary disease, asthma, Wegener's granulomatosis, antiglomerular basement membrane disease (Goodpasture's disease), meconium aspiration syndrome, bronchiolitis obliterans syndrome, idiopathic pulmonary fibrosis, acute lung injury secondary to burn, non- cardiogenic pulmonary edema, transfusion-related respiratory depression, emphysema, cystic fibrosis, SARS-CoV, MERS-CoV and SARS-CoV-2(Covid-19) related condition;
the extracorporeal reperfusion procedure comprises hemodialysis, plasmapheresis, leukopheresis, exfracorporeal membrane oxygenator (ECMO), heparin-induced extracorporeal membrane oxygenation LDL precipitation (HELP) and cardiopulmonary bypass (CPB);
the musculoskeletal condition comprises osteoarthritis, rheumatoid arthritis, juvenile rheumatoid arthritis, gout, neuropathic arthropathy, psoriatic arthritis, spondyloarthropathy, crystalline arthropathy and systemic lupus erythematosus (SLE);
the renal condition comprises mesangioproliferative glomerulonephritis, membranous glomerulonephritis, membranoproliferative glomerulonephritis (mesangiocapillary glomerulonephritis), acute postinfectious glomerulonephritis (poststreptococcal glomerulonephritis), cryoglobulinemic glomerulonephritis, lupus nephritis, Henoch- Schonlein purpura nephritis and IgA nephropathy;
the skin condition comprises psoriasis, autoimmune bullous dermatoses, eosinophilic spongiosis, bullous pemphigoid, Epidermolysis bullosa acquisita (EBA), herpes gestationis, thermal burn injury and chemical burn injury;
the organ or tissue transplant procedure comprises organ allotransplantation, organ xenotransplantation organ and tissue graft;
the nervous system disorder or injury comprises multiple sclerosis, myasthenia gravis, Huntington's disease, amyotrophic lateral sclerosis, Guillain Barre syndrome, reperfusion following stroke, degenerative discs, cerebral trauma, Parkinson's disease, Alzheimer's disease, Miller-Fisher syndrome, cerebral frauma and/or hemorrhage, demyellination and meningitis;
the blood disorder comprises sepsis, severe sepsis, septic shock, acute respiratory distress syndrome resulting from sepsis, systemic inflammatory response syndrome, hemorrhagic shock, hemolytic anemia, autoimmune thrombotic thrombocytopenic purpura and hemolytic uremic syndrome;
the urogenital condition comprises painful bladder disease, sensory bladder disease, chronic abacterial cystitis, interstitial cystitis, infertility, placental dysfunction and miscarriage and pre-eclampsia;
the endocrine disorder comprises Hashimoto's thyroiditis, stress, anxiety and hormonal disorders involving regulated release of prolactin, growth or other insulin-like growth factor and adrenocorticotropin from the pituitary;
the ophthalmologic condition comprises age-related macular degeneration.

68. The use accroding to claim 65, wherein said medicament is used for subcutaneous administration, parenteral administration, and/or intravenous administration.
